# EUROPEAN PATENT APPLICATION

(11) **EP 4 144 728 A1**
(43) Date of publication of application: **08.03.2023**
(21) Application number: 21796061.6
(22) Date of filing: 29.04.2021
(51) Int. Cl.: C07D 401/14, C07D 487/04, A61K 31/4745, A61P 35/00

(54) **HETEROCYCLE AND GLUTARIMIDE SKELETON-BASED COMPOUND AND APPLICATIONS THEREOF**

(30) Priority: 30.04.2020 CN 202010366530
(71) Applicant: Shanghaitech University, Shanghai 201210 (CN)
(72) Inventor: YANG, Xiaobao, Shanghai 201210 (CN); JIANG, Biao, Shanghai 201210 (CN); LIU, Linyi, Shanghai 201210 (CN); REN, Chaowei, Shanghai 201210 (CN); QIU, Xing, Shanghai 201210 (CN); LIU, Haixia, Shanghai 201210 (CN); SUN, Ning, Shanghai 201210 (CN); SUN, Renhong, Shanghai 201210 (CN)
(74) Representative: J A Kemp LLP
(86) International application number: PCT/CN2021/090964
(87) International publication number: WO 2021/219078

(57) **Abstract**

The present disclosure relates to compounds of Formula (I) or salts, enantiomers, stereoisomers, solvates, or polymorphs thereof, and uses thereof. The present disclosure also relates to pharmaceutical compositions comprising, as an active ingredient, the compound of Formula (I) or a salt, enantiomer, stereoisomer, solvate, or polymorph thereof, and use thereof. A series of compounds designed and synthesized by the present disclosure can effectively prevent and/or treat diseases or disorders associated with TNF-α.

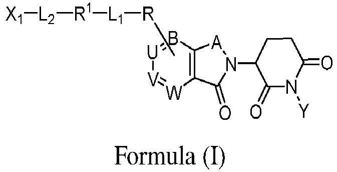

## Description

### Technical Field

The present disclosure relates to compounds of Formula (I) or a salt, an enantiomer, a stereoisomer, a solvate, or a polymorph thereof and applications thereof, especially their application in the prevention and/or treatment of a disease or disorder associated with TNF-α.

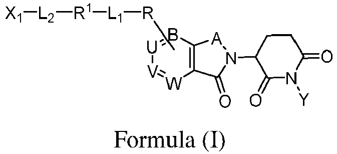

### Background

Lenalidomide is a derivative with lower toxicity but higher activity, and was approved by U.S. Food and Drug Administration (FDA) in 2006 in combination with Dexamethasone for the treatment of relapsed or refractory multiple myeloma. In 2013, the third-generation immunomodulatory drug Pomalidomide was approved for patients with relapsed/refractory multiple myeloma treated with at least two drugs including Lenalidomide and Bortezomib. Studies have shown that the primary direct target protein of Lenalidomide and its analogs is Cereblon. Subsequently, the mechanism of these drugs in the treatment of multiple myeloma was revealed. That is, immunomodulatory drugs can bind to the CRBN protein in cells, thereby causing CRL4ACRBN ubiquitin ligase to ubiquitinate transcription factors IKZF1 and IKZF3 for degradation. These two transcription factors are critical in the development and survival of B cells. In 2014, Prof. Harper discovered five endogenous ligase substrates: GRINL1A, MBOAT7, OTUD7B, C6orf141 and MEIS2 by ubiquitination analysis of protein chips. Among them, MEIS2, as a transcription factor, plays an important role in the normal development of the human body, and its increased expression can lead to shortened toe tips in chicken embryos, indicating that this molecule is a potential downstream molecule of thalidomide-induced embryonic malformation. The binding site of MEIS2 molecule in CRBN is the same as that of Thalidomide, so Thalidomide competes with MEIS2 for binding to CRBN. In 2015, Prof. Krönke discovered that lenalidomide is also highly effective in the treatment of myelodysplastic syndrome (MDS) caused by the deletion of 5q chromosome. Through using stable isotope labeling of amino acids in cell culture (SILAC) quantitative mass spectrometry to evaluate the global changes in ubiquitination and protein levels in del (5q) myeloid cell line KG-1, it was found that lenalidomide can uniquely degrade CK1α protein, while neither pomalidomide nor thalidomide has any effect in CK1α.

TNF-α (tumor necrosis factor alpha) is the most important cytokine involved in inflammatory, which is produced mainly by monocytes, macrophages and thymus-dependent lymphocytes, and can also promote tissue destruction by coordinating recruitment of immune cells into and out of tissue, and play a key role in the occurrence and development of many diseases, such as infectious diseases, inflammatory diseases, tumors, Acquired Immune Deficiency Syndrome (AIDS), anemia, hemorrhagic shock, transplant rejection, tuberculosis and diabetes. Thus, blocking TNF-α at various levels may potentially treat diseases or conditions associated with TNF-α. As immunomodulators, Lenalidomide and Pomalidomide can significantly reduce the level of TNF-α through cereblon E3 ligase and inhibit the secretion of other pro-inflammatory factors, and have anti-tumor and immunomodulatory activities (Lopez-Girona A, et al. Leukemia, 2012; Liu D, et al. Gen Comp Endocrinol, 2016; 228:1). Currently, Lenalidomide has been approved for the treatment of multiple myeloma, myelodysplastic syndrome and mantle cell lymphoma, while Pomalidomide is applicable to patients with multiple myeloma who have received at least two drugs (including Lenalidomide, Bortezomib) in the past and recently undergone treatment or progressed within 60 days after completion of treatment. In addition, in clinical trials, Lenalidomide and Pomalidomide can be used alone or in combination with other therapeutic drugs for the treatment of other diseases such as lymphomas, thyroid cancer, leukemia, melanoma, lung cancer.

In sum, Thalidomide and derivatives thereof play a vital role in the treatment of other related malignancies such as multiple myeloma and immunological diseases, but at present the diversity of this class of compounds is insufficient. Therefore, there is an urgent need to design and synthesize a series of highly active glutarimide skeleton-based drugs in order to achieve better therapeutic effect.

### Summary of Invention

Therefore, in one aspect, the present disclosure provides a compound of Formula (I): 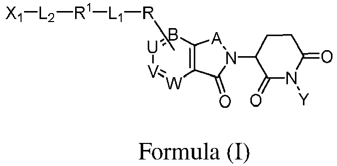 or a salt, an enantiomer, a stereoisomer (including diastereomer), a solvate, or polymorph thereof, in which
Y, A, B, U, V, W, R, L₁, R¹, L₂, and X₁ and all substituents are as defined in the Detailed Description of the Invention.

The present disclosure also provides a pharmaceutical composition comprising the compound of Formula (I) or a pharmaceutically acceptable salt, an enantiomer, a stereoisomer, a solvate, or polymorph thereof, and at least one pharmaceutically acceptable carrier.

The present disclosure also provides the compound of Formula (I), or a pharmaceutically acceptable salt, an enantiomer, a diastereomer, a solvate, or polymorph thereof for use as a medicament.

The present disclosure also provides the compound of Formula (I), or a pharmaceutically acceptable salt, an enantiomer, a diastereomer, a solvate, or polymorph thereof or the pharmaceutical composition for use in the prevention or treatment of a disease or disorder associated with TNF-α.

The present disclosure also provides the use of the compound of Formula (I) or a pharmaceutically acceptable salt, an enantiomer, a stereoisomer, a solvate, or polymorph thereof or the pharmaceutical composition for the manufacture of a medicament for the treatment or prevention of a disease or disorder associated with TNF-α.

The present disclosure also provides a method for the treatment or prevention of a disease or disorder associated with TNF-α, comprising administering to a subject a therapeutically effective amount of the compound of Formula (I), or a pharmaceutically acceptable salt, an enantiomer, a stereoisomer, a solvate, or polymorph thereof, or the pharmaceutical composition of the present disclosure.

### Brief Description of Drawings

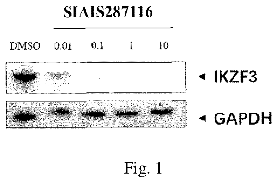

FIG. 1 shows the degradation of IKZF1 and IKZF3 proteins caused by the compounds of the present invention in multiple myeloma cells MM.1S, as detected by Western blotting.

### Detailed Description of the Invention

In one aspect, the present disclosure provides a compound of Formula (I): 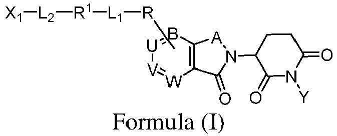 or a salt, an enantiomer, a diastereomer, a solvate, or a polymorph thereof, in which
Y represents H or C₁₋₃ alkyl (e.g., methyl, ethyl, or propyl);
A represents CH₂ or C(O);
B, U, V, W are the same or different and each independently represent CH or N, wherein B, U, V and W are not simultaneously N, and when any of B, U, V, and W is attached to group R, it is not N;
R represents O, S, N(R²), CH₂, alkenylene or alkynylene, wherein R² represents H or C₁₋₃ alkyl (e.g., methyl, ethyl or propyl), or R represents a bond;
R¹ represents optionally substituted heterocyclylene or optionally substituted heteroarylene, wherein said heterocyclylene and said heteroarylene each independently optionally substituted with a substituent(s) selected from the group consisting of C₁₋₃ alkyl (e.g., methyl, ethyl or propyl), C₁₋₃ alkoxy (e.g., methoxy, ethoxy or propoxy), C₃₋₆ cycloalkyl (e.g., cyclopropyl, cyclobutyl, cyclopentyl or cyclohexyl), halogenated C₁₋₃ alkyl (such as difluoromethyl or trifluoromethyl), halogen (such as fluorine, chlorine, bromine or iodine), hydroxy, cyano, amino, or any combination thereof;
L₁ represents Formula -L₃-L₄-^{∗}, where symbol * indicates the point of attachment of L₄ to R,
   L₃ represents O, S, S(O)₂O, OS(O)₂, S(O)₂N(R³), N(R³)S(O)₂, C(O)O, OC(O), N(R³), C(O)N(R³), or N(R³)C(O), wherein R³ represents H or C₁₋₃ alkyl (e.g., methyl, ethyl or propyl), or
   L₃ represents a bond; and
   L₄ represents optionally substituted methylene or optionally substituted linear or branched C₂₋₄₀ alkylene, wherein the linear or branched C₂₋₄₀ alkylene is optionally interrupted one or more times (e.g., 1-10 times, 1-8 times, 1-6 times, 1-5 times, 1-3 times, 1-2 times, or 1 time) by a group selected from the group consisting of: optionally substituted heterocyclylene, optionally substituted heteroarylene, O, S, S(O)₂O, OS(O)₂, S(O)₂N(R³), N(R³)S(O)₂, C(O)O, OC(O), N(R³), C(O)N(R³), N(R³)C(O) or any combination thereof, wherein R³ represents H or C₁₋₃ alkyl (e.g., methyl, ethyl or propyl), wherein the methylene, hydrogen(s) of one or more CH₂ (e.g., 1-20, 1-15, 1-10, 1-8, 1-5, 1-3 or 1-2 CH₂) of backbone of said linear or branched C₂₋₄₀ alkylene, the heterocyclylene and the heteroarylene are each independently optionally substituted by a substituent(s) selected from the group consisting of C₁₋₃ alkyl (e.g., methyl, ethyl or propyl), C₁₋₃ alkoxy (e.g., methoxy, ethoxy or propoxy), C₃₋₆ cycloalkyl (e.g. cyclopropyl, cyclobutyl, cyclopentyl or cyclohexyl), halogenated C₁₋₃ alkyl (e.g. trifluoromethyl), halogen (e.g. fluorine, chlorine, bromine or iodine), hydroxy, cyano , amino or any combination thereof, or
   L₄ represents a bond, provided that R, L₃ and L₄ do not simultaneously represent a bond, and when L₄ represents a bond, either R or L₃ represents a bond; and
L₂ represents Formula -L₅-L₆-^{∗∗}, where symbol ** indicates the point of attachment of L₆ to R¹,
   L₆ represents O, S, S(O)₂O, OS(O)₂, S(O)₂N(R³), N(R³)S(O)₂, C(O)O, OC(O), N(R³), C(O)N(R³), or N(R³)C(O), wherein R³ represents H or C₁₋₃ alkyl (e.g., methyl, ethyl or propyl), or
   L₆ represents a bond; and
   L₅ represents optionally substituted methylene or optionally substituted linear or branched C₂₋₄₀ alkylene, wherein the linear or branched C₂₋₄₀ alkylene is optionally interrupted one or more times (e.g., 1-10 times, 1-8 times, 1-6 times, 1-5 times, 1-3 times, 1-2 times, or 1 time) by a group selected from the group consisting of: optionally substituted heterocyclylene, optionally substituted heteroarylene, O, S, S(O)₂O, OS(O)₂, S(O)₂N(R³), N(R³)S(O)₂, C(O)O, OC(O), N(R³), C(O)N(R³), N(R³)C(O) or any combination thereof, wherein R³ represents H or C₁₋₃ alkyl (e.g., methyl, ethyl or propyl), wherein the methylene, hydrogen(s) of one or more CH₂ (e.g., 1-20, 1-15, 1-10, 1-8, 1-5, 1-3 or 1-2 CH₂) of backbone of said linear or branched C₂₋₄₀ alkylene, the heterocyclylene and the heteroarylene are each independently optionally substituted by a substituent(s) selected from the group consisting of C₁₋₃ alkyl (e.g., methyl, ethyl or propyl), C₁₋₃ alkoxy (e.g., methoxy, ethoxy or propoxy), C₃₋₆ cycloalkyl (e.g. cyclopropyl, cyclobutyl, cyclopentyl or cyclohexyl), halogenated C₁₋₃ alkyl (e.g. trifluoromethyl), halogen (e.g. fluorine, chlorine, bromine or iodine), hydroxy, cyano, amino or any combination thereof, or
   L₅ represents a bond; and
X₁ represents Formula X_{b}-Xₐ-, wherein
   Xₐ represents -(CH₂)₀₋₂₀-O-^{#}, -(CH₂)₀₋₂₀-S-^{#}, -(CH₂)₀₋₂₀-C(O)O-^{#}, -(CH₂)₀₋₂₀-OC(O)-^{#}, - (CH₂)₀₋₂₀-N(R³)-^{#}, -(CH₂)₀₋₂₀-C(O)N(R³)-^{#}, -(CH₂)₀₋₂₀-N(R³)C(O)-^{#}, -(CH₂)₀₋₂₀-S(O)₂N(R³)-^{#}, - (CH₂)₀₋₂₀₋N(R³)S(O)₂-^{#}, -(CH₂)₀₋₂₀-S(O)₂O-^{#}, -(CH₂)₀₋₂₀-OS(O)₂-^{#}, -(CH₂)₀₋₂₀-S(O)₂-^{#}, -(CH₂)₀₋₂₀-S(O)₂-^{#}, alkynylene, or alkenylene, wherein R³ represents H or C₁₋₃ alkyl (e.g., methyl, ethyl or propyl), and symbol # indicates the point of attachment to L₂, or
   Xₐ represents a bond, provided that when L₅ represents a bond, either Xₐ or L₆ represents a bond; and
   X_{b} represents optionally substituted adamantanyl, optionally substituted noradamantanyl, optionally substituted meta-menthanyl, optionally substituted p-menthanyl, optionally substituted quinuclidinyl, optionally substituted bicyclo[2.2.1]heptyl or optionally substituted bicyclo[2.2.1]heptenyl, wherein said adamantanyl, said noradamantanyl, said meta-menthanyl, said p-menthanyl, said quinuclidinyl, said bicyclo[2.2.1]heptyl, and said bicyclo[2.2.1]heptenyl are each independently optionally substituted by a substituent(s) selected from the group consisting of C₁₋₃ alkyl (e.g., methyl, ethyl or propyl), halogenated C₁₋₃ alkyl (e.g., trifluoromethyl), C₁₋₃ alkoxy (e.g., methoxy, ethoxy or propoxy), C₁₋₃ alkyl-NH-, amino-C₁₋₃ alkylene, amino, oxo, halogen (e.g., fluorine, chlorine, bromine or iodine), hydroxy, cyano, C₁₋₃ alkyl- NHC(O)-, mercapto, or any combination thereof.

In one embodiment of the present disclosure, B, U, V, and W of Formula (I) are the same and all are CH.

In one embodiment of the present disclosure, one of B, U, V, and W of Formula (I) is N, and the rest are CH.

In one embodiment of the present disclosure, two of B, U, V, and W of Formula (I) are N, and the rest are CH.

In one embodiment of the present disclosure, three of B, U, V, and W of Formula (I) are N, and the rest is CH.

In one embodiment of the present disclosure, Y represents H.

In one embodiment of the present disclosure, Y represents a C₁₋₃ alkyl (e.g., methyl or ethyl).

In one embodiment of the present disclosure, Y represents methyl.

In one embodiment of the present disclosure, A represents C(O).

In one embodiment of the present disclosure, A represents CH₂.

In one embodiment of the present disclosure, R represents O.

In one embodiment of the present disclosure, R represents S.

In one embodiment of the present disclosure, R represents N(R²), wherein R² represents H or C₁₋₃ alkyl (e.g., methyl, ethyl or propyl).

In one embodiment of the present disclosure, R represents CH₂.

In one embodiment of the present disclosure, R represents alkenylene.

In one embodiment of the present disclosure, R represents alkynylene.

In one embodiment of the present disclosure, R represents a bond.

In one embodiment of the present disclosure, R¹ represents the following groups optionally further substituted with a substituent(s) selected from the group consisting of C₁₋₃ alkyl (e.g., methyl, ethyl or propyl), C₁₋₃ alkoxy (e.g., methoxy, ethoxy or propoxy), C₃₋₆ cycloalkyl (e.g., cyclopropyl, cyclobutyl, cyclopentyl or cyclohexyl), halogenated C₁₋₃ alkyl (e.g., difluoromethyl or trifluoromethyl), halogen (e.g., fluorine, chlorine, bromine or iodine), hydroxy, cyano, amino, or any combination thereof:
furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzoisoxazolylene, benzothiazolylene, benzoisothiazolylene, benzotriazolylene, benzo[2,1,3]oxadiazolylene, benzo[2,1,3]thiadiazolylene, benzo[1,2,3]thiadiazolylene, quinolylene, isoquinolylene, naphthyridinylene, cinnolinylene, quinazolinylene, quinoxalinylene, phthalazinylene, pyrazolo[1,5-a]pyridinylene, pyrazolo[1,5-a]pyrimidinylene, imidazo[1,2-a]pyridinylene, 1H-pyrrolo[3,2-b]pyridinylene, 1H-pyrrolo[2,3-b]pyridinylene, 4H-fluoro[3,2-b]pyrrolylene, pyrrolo[2,1-b]thiazolylene, imidazo[2,1-b]thiazolylene, azetidinylene, oxetanylene, pyrrolidinylene, imidazolidylene, pyrazolidylene, triazolylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxanylene, and diazepanylene (e.g., 1,4-diazepanylene, 4,5-diazepanylene, or 1,3-diazepanylene).

In one embodiment of the present disclosure, wherein L₁ represents Formula -L₃-L₄-^{∗}, wherein symbol * indicates the point of attachment of L₄ to R,
L₃ represents a bond; and
L₄ represents optionally substituted methylene or optionally substituted linear or branched C₂₋₄₀ alkylene, wherein the linear or branched C₂₋₄₀ alkylene is optionally interrupted one or more times (e.g., 1-10 times, 1-8 times, 1-6 times, 1-5 times, 1-3 times, 1-2 times, or 1 time) by a group selected from the group consisting of: optionally substituted heterocyclylene, optionally substituted heteroarylene, O, S, S(O)₂O, OS(O)₂, S(O)₂N(R³), N(R³)S(O)₂, C(O)O, OC(O), N(R³), C(O)N(R³), N(R³)C(O) or any combination thereof, wherein R³ represents H or C₁₋₃ alkyl (e.g., methyl, ethyl or propyl), wherein the methylene, hydrogen(s) of one or more CH₂ (e.g., 1-20, 1-15, 1-10, 1-8, 1-5, 1-3 or 1-2 CH₂) of backbone of said linear or branched C₂₋₄₀ alkylene, the heterocyclylene and the heteroarylene are each independently optionally substituted by a substituent(s) selected from the group consisting of C₁₋₃ alkyl (e.g., methyl, ethyl or propyl), C₁₋₃ alkoxy (e.g., methoxy, ethoxy or propoxy), C₃₋₆ cycloalkyl (e.g. cyclopropyl, cyclobutyl, cyclopentyl or cyclohexyl), halogenated C₁₋₃ alkyl (e.g. difluoromethyl or trifluoromethyl), halogen (e.g. fluorine, chlorine, bromine or iodine), hydroxy, cyano, amino or any combination thereof. In a sub-embodiment, the optionally substituted heteroarylene is: furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzoisoxazolylene, benzothiazolylene, benzoisothiazolylene, benzotriazolylene, benzo[2,1,3]oxadiazolylene, benzo[2,1,3]thiadiazolylene, benzo[1,2,3]thiadiazolylene, quinolylene, isoquinolylene, naphthyridinylene, cinnolinylene, quinazolinylene, quinoxalinylene, phthalazinylene, pyrazolo[1,5-a]pyridinylene, pyrazolo[1,5-a]pyrimidinylene, imidazo[1,2-a]pyridinylene, 1H-pyrrolo[3,2-b]pyridinylene, 1H-pyrrolo[2,3-b]pyridinylene, 4H-fluoro[3,2-b]pyrrolylene, pyrrolo[2,1-b]thiazolylene or imidazo[2,1-b]thiazolylene, wherein the heteroarylene is optionally further substituted by a substituent(s) selected from the group consisting of C₁₋₃ alkyl (e.g., methyl, ethyl or propyl), C₁₋₃ alkoxy (e.g., methoxy, ethoxy or propoxy), C₃₋₆ cycloalkyl (e.g., cyclopropyl, cyclobutyl, cyclopentyl or cyclohexyl), halogenated C₁₋₃ alkyl (e.g., bifluoromethyl or trifluoromethyl), halogen (e.g., fluorine, chlorine, bromine or iodine), hydroxy, cyano, amino, or any combination thereof. In a sub-embodiment, the optionally substituted heterocyclylene is: azetidinylene, oxetanylene, pyrrolidinylene, imidazolidylene, pyrazolidylene, triazolylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxanylene, and diazepanylene (e.g., 1,4-diazepanylene, 4,5-diazepanylene, or 1,3-diazepanylene), wherein the heterocyclylene is optionally further substituted by a substituent(s) selected from the group consisting of C₁₋₃ alkyl (e.g., methyl, ethyl or propyl), C₁₋₃ alkoxy (e.g., methoxy, ethoxy or propoxy), C₃₋₆ cycloalkyl (e.g., cyclopropyl, cyclobutyl, cyclopentyl or cyclohexyl), halogenated C₁₋₃ alkyl (e.g., bifluoromethyl or trifluoromethyl), halogen (e.g., fluorine, chlorine, bromine or iodine), hydroxy, cyano, amino, or any combination thereof.

In one embodiment of the present disclosure, L₁ represents Formula -L₃-L₄-^{∗}, wherein symbol * indicates the point of attachment of L₄ to R, L₃ represents a bond; and L₄ represents linear or branched C₁₋₄₀ alkylene (e.g. C₁₋₃₅ alkylene, C₁₋₃₀ alkylene, C₁₋₂₅ alkylene, C₁₋₂₃ alkylene, C₁₋₂₂ alkylene, C₁₋₂₁ alkylene, C₁₋₂₀ alkylene, C₁₋₁₉ alkylene, C₁₋₁₈ alkylene, C₁₋₁₇ alkylene, C₁₋₁₆ alkylene, C₁₋₁₅ alkylene, C₁₋₁₀ alkylene, C₁₋₉ alkylene, C₁₋₈ alkylene, C₁₋₇ alkylene, C₁₋₆ alkylene, C₁₋₅ alkylene, C₂₋₄ alkylene or C₁₋₃ alkylene) optionally substituted by a substituent(s) selected from the group consisting of C₁₋₃ alkyl (e.g., methyl, ethyl or propyl), C₁₋₃ alkoxy (e.g., methoxy, ethoxy or propoxy), C₃₋₆ cycloalkyl (e.g., cyclopropyl, cyclobutyl, cyclopentyl or cyclohexyl), halogenated C₁₋₃ alkyl (e.g., difluoromethyl or trifluoromethyl), halogen (e.g., fluorine, chlorine, bromine or iodine), hydroxyl, cyano, amino or any combination thereof.

In one embodiment of the present disclosure, L₄ represents following groups: -CH₂-, -(CH₂)₂-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₁₂-, - (CH₂)₁₃-, -(CH₂)₁₄-, -(CH₂)₁₅-, -(CH₂)₁₆-, -(CH₂)₁₇-, -(CH₂)₁₈-, -(CH₂)₁₉-, or -(CH₂)₂₀-; wherein hydrogen(s) of one or more CH₂ (e.g., 1-20, 1-15, 1-10, 1-8, 1-5, 1-3 or 1-2 CH₂) of the groups is optionally further replaced with a substituent(s) selected from the group consisting of C₁₋₃ alkyl (e.g., methyl, ethyl or propyl), C₁₋₃ alkoxy (e.g., methoxy, ethoxy or propoxy), C₃₋₆ cycloalkyl (e.g., cyclopropyl, cyclobutyl, cyclopentyl or cyclohexyl), halogenated C₁₋₃ alkyl (e.g., difluoromethyl or trifluoromethyl), halogen (e.g., fluorine, chlorine, bromine, or iodine), hydroxy, cyano, amino, or any combination thereof.

In one embodiment of the present disclosure, L₂ represents Formula -L₅-L₆-^{∗∗}, wherein symbol ** indicates the point of attachment of L₆ to R¹, and both L₅ and L₆ represent a bond.

In one embodiment of the present disclosure, L₂ represents Formula -L₅-L₆-^{∗∗}, wherein symbol ** indicates the point of attachment of L₆ to R¹,
L₆ represents a bond; and
L₅ represents optionally substituted methylene or optionally substituted linear or branched C₂₋₄₀ alkylene (e.g., C₂₋₃₅ alkylene, C₂₋₃₀ alkylene, C₂₋₂₅ alkylene, C₂₋₂₃ alkylene, C₂₋₂₂ alkylene, C₂₋₂₁ alkylene, C₂₋₂₀ alkylene, C₂₋₁₉ alkylene, C₂₋₁₈ alkylene, C₂₋₁₇ alkylene, C₂₋₁₆ alkylene, C₂₋₁₅ alkylene, C₂₋₁₀ alkylene, C₂₋₉ alkylene, C₂₋₈ alkylene, C₂₋₇ alkylene, C₂₋₆ alkylene, C₂₋₅ alkylene, C₂₋₄ alkylene or C₂₋₃ alkylene), wherein the linear or branched C₂₋₄₀ alkylene is optionally interrupted one or more times (e.g. 1-10 times, 1-8 times, 1-6 times, 1-5 times, 1-3 times, 1-2 times or 1 time) by a group selected from the group consisting of: optionally substituted heterocyclylene, optionally substituted heteroarylene, O, S, S(O)₂O, OS(O)₂, S(O)₂N(R³), N(R³)S(O)₂, C(O)O, OC(O), N(R³), C(O)N(R³), N(R³)C(O) or any combination thereof, wherein R³ represents H or C₁₋₃ alkyl (e.g., methyl, ethyl or propyl), wherein the methylene, hydrogen(s) of one or more CH₂ (e.g., 1-20, 1-15, 1-10, 1-8, 1-5, 1-3 or 1-2 CH₂) of backbone of said linear or branched C₂₋₄₀ alkylene, the heterocyclylene and the heteroarylene are each independently optionally substituted by a substituent(s) selected from the group consisting of C₁₋₃ alkyl (e.g., methyl, ethyl or propyl), C₁₋₃ alkoxy (e.g., methoxy, ethoxy or propoxy), C₃₋₆ cycloalkyl (e.g., cyclopropyl, cyclobutyl, cyclopentyl or cyclohexyl), halogenated C₁₋₃ alkyl (e.g., difluoromethyl or trifluoromethyl), halogen (e.g., fluorine, chlorine, bromine, or iodine), hydroxy, cyano, amino, or any combination thereof. In a sub-embodiment, the optionally substituted heteroarylene is, for example, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzoisoxazolylene, benzothiazolylene, benzoisothiazolylene, benzotriazolylene, benzo[2,1,3]oxadiazolylene, benzo[2,1,3]thiadiazolylene, benzo[1,2,3]thiadiazolylene, quinolylene, isoquinolylene, naphthyridinylene, cinnolinylene, quinazolinylene, quinoxalinylene, phthalazinylene, pyrazolo[1,5-a]pyridinylene, pyrazolo[1,5-a]pyrimidinylene, imidazo[1,2-a]pyridinylene, 1H-pyrrolo[3,2-b]pyridinylene, 1H-pyrrolo[2,3-b]pyridinylene, 4H-fluoro[3,2-b]pyrrolylene, pyrrolo[2,1-b]thiazolylene or imidazo[2,1-b]thiazolylene, wherein the heteroarylene is optionally further substituted by a substituent(s) selected from the group consisting of C₁₋₃ alkyl (e.g., methyl, ethyl or propyl), C₁₋₃ alkoxy (e.g., methoxy, ethoxy or propoxy), C₃₋₆ cycloalkyl (e.g., cyclopropyl, cyclobutyl, cyclopentyl or cyclohexyl), halogenated C₁₋₃ alkyl (e.g., bifluoromethyl or trifluoromethyl), halogen (e.g., fluorine, chlorine, bromine or iodine), hydroxy, cyano, amino, or any combination thereof. In a sub-embodiment, the optionally substituted heterocyclylene is, for example, azetidinylene, oxetanylene, pyrrolidinylene, imidazolidylene, pyrazolidylene, triazolylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxanylene, and diazepanylene (e.g., 1,4-diazepanylene, 4,5-diazepanylene, or 1,3-diazepanylene), wherein the heterocyclylene is optionally further substituted by a substituent(s) selected from the group consisting of C₁₋₃ alkyl (e.g., methyl, ethyl or propyl), C₁₋₃ alkoxy (e.g., methoxy, ethoxy or propoxy), C₃₋₆ cycloalkyl (e.g., cyclopropyl, cyclobutyl, cyclopentyl or cyclohexyl), halogenated C₁₋₃ alkyl (e.g., bifluoromethyl or trifluoromethyl), halogen (e.g., fluorine, chlorine, bromine or iodine), hydroxy, cyano, amino, or any combination thereof.

In one embodiment of the present disclosure, L₂ represents Formula -L₅-L₆-^{∗∗}, wherein symbol ** indicates the point of attachment of L₆ to R¹,
L₆ represents a bond; and
L₅ represents a group having the following general formula: -(CH₂)ₙ₁-N(R³)-(CH₂)ₙ₂-^{∗∗∗}, - (CH₂)ₙ₁-C(O)O-(CH₂)ₙ₂-^{∗∗∗}, -(CH₂)ₙ₁-OC(O)-(CH₂)ₙ₂-^{∗∗∗}, -(CH₂)ₙ₁-C(O)N(R³)-(CH₂)ₙ₂-^{∗∗∗}, - (CH₂)ₙ₁-N(R³)C(O)-(CH₂)ₙ₂-^{∗∗∗}, -(CH₂)ₙ₁-S(O)₂O-(CH₂)ₙ₂-^{∗∗∗}, -(CH₂)ₙ₁-OS(O)₂-(CH₂)ₙ₂-^{∗∗∗}, - (CH₂)ₙ₁-S(O)₂N(R³)-(CH₂)ₙ₂-^{∗∗∗}, or -(CH₂)ₙ₁-N(R³)S(O)₂-(CH₂)ₙ₂-^{∗∗∗},
   wherein symbol *** indicates the point of attachment of L₅ to L₆, R³ represents H or C₁₋₃ alkyl, and hydrogen(s) of one or more CH₂ (e.g., 1-10, 1-8, 1-5, 1-3 or 1-2 CH₂) of said group is optionally further replaced by a substituent(s) selected from the group consisting of C₁₋₃ alkyl (e.g., methyl, ethyl or propyl), C₁₋₃ alkoxy (e.g., methoxy, ethoxy or propoxy), C₃₋₆ cycloalkyl (e.g., cyclopropyl, cyclobutyl, cyclopentyl or cyclohexyl), halogenated C₁₋₃ alkyl (e.g., difluoromethyl or trifluoromethyl), halogen (e.g., fluorine, chlorine, bromine or iodine), hydroxy, cyano, amino, or any combination thereof, and
   n1 and n2 each independently represent an integer of 1, 2, 3, 4, 5, 6, 7, 8, 9, 10, 11, 12, 13, 14, or 15.

In one embodiment of the present disclosure, L₂ represents Formula -L₅-L₆-^{∗∗}, wherein symbol ** indicates the point of attachment of L₆ to R¹,
L₆ represents a bond; and
L₅ represents the following groups: -CH(CH₃)C(O)OCH₂-^{∗∗∗}, -CH₂CH(CH₃)C(O)OCH₂-^{∗∗∗}, - (CH₂)₂CH(CH₃)C(O)OCH₂-^{∗∗∗}, -(CH₂)₃CH(CH₃)C(O)OCH₂-^{∗∗∗}, -(CH₂)₄CH(CH₃)C(O)OCH₂-^{∗∗∗}, - (CH₂)₅CH(CH₃)C(O)OCH₂-^{∗∗∗}, -(CH₂)₆CH(CH₃)C(O)OCH₂-^{∗∗∗}, -(CH₂)₇CH(CH₃)C(O)OCH₂-^{∗∗∗}, - CH(CH₃)OC(O)CH₂-^{∗∗∗}, -CH₂CH(CH₃)OC(O)CH₂-^{∗∗∗}, -(CH₂)₂CH(CH₃)OC(O)CH₂-^{∗∗∗}, - (CH₂)₃CH(CH₃)OC(O)CH₂-^{∗∗∗}, -(CH₂)₄CH(CH₃)OC(O)CH₂-^{∗∗∗}, -(CH₂)₅CH(CH₃)OC(O)CH₂-^{∗∗∗}, - (CH₂)₆CH(CH₃)OC(O)CH₂-^{∗∗∗}, -(CH₂)₇CH(CH₃)OC(O)CH₂-^{∗∗∗}, -(CH₂)₈CH(CH₃)OC(O)CH₂-^{∗∗∗}, - CH(CH₃)C(O)NHCH₂-^{∗∗∗}, -CH₂CH(CH₃)C(O)NHCH₂-^{∗∗∗}, -(CH₂)₂CH(CH₃)C(O)NHCH₂-^{∗∗∗}, - (CH₂)₃CH(CH₃)C(O)NHCH₂-^{∗∗∗}, -(CH₂)₄CH(CH₃)C(O)NHCH₂-^{∗∗∗}, -(CH₂)₅CH(CH₃)C(O)NHCH₂-^{∗∗∗}, -(CH₂)₆CH(CH₃)C(O)NHCH₂-^{∗∗∗}, -(CH₂)₇CH(CH₃)C(O)NHCH₂-^{∗∗∗}, - (CH₂)₈CH(CH₃)C(O)NHCH₂-^{∗∗∗}, -(CH₂)₉CH(CH₃)C(O)NHCH₂-^{∗∗∗} or - (CH₂)₁₀CH(CH₃)C(O)NHCH₂-^{∗∗∗}, wherein hydrogen(s) of one or more CH₂ (e.g., 1-5, 1-3 or 1-2 CH₂) of said group is optionally further replaced by a substituent(s) selected from the group consisting of C₁₋₃ alkyl (e.g., methyl, ethyl or propyl), C₁₋₃ alkoxy (e.g., methoxy, ethoxy or propoxy), C₃₋₆ cycloalkyl (e.g., cyclopropyl, cyclobutyl, cyclopentyl or cyclohexyl), halogenated C₁₋₃ alkyl (e.g., difluoromethyl or trifluoromethyl), halogen (e.g., fluorine, chlorine, bromine or iodine), hydroxy, cyano, amino, or any combination thereof; and symbol *** indicates the point of attachment of L₅ to L₆.

In one embodiment of the present disclosure, the -Xₐ-L₅-L₆- moiety in Formula (I) represents the following groups:
-CH(CH₃)C(O)OCH₂-, -CH₂CH(CH₃)C(O)OCH₂-, -(CH₂)₂CH(CH₃)C(O)OCH₂-, - (CH₂)₃CH(CH₃)C(O)OCH₂-, -(CH₂)₄CH(CH₃)C(O)OCH₂-, -(CH₂)₅CH(CH₃)C(O)OCH₂-, - (CH₂)₆CH(CH₃)C(O)OCH₂-, -(CH₂)₇CH(CH₃)C(O)OCH₂-, -CH(CH₃)OC(O)CH₂-, - CH₂CH(CH₃)OC(O)CH₂-, -(CH₂)₂CH(CH₃)OC(O)CH₂-, -(CH₂)₃CH(CH₃)OC(O)CH₂-, - (CH₂)₄CH(CH₃)OC(O)CH₂-, -(CH₂)₅CH(CH₃)OC(O)CH₂-, -(CH₂)₆CH(CH₃)OC(O)CH₂-, - (CH₂)₇CH(CH₃)OC(O)CH₂-, -(CH₂)₈CH(CH₃)OC(O)CH₂-, -CH(CH₃)C(O)NHCH₂-, - CH₂CH(CH₃)C(O)NHCH₂-, -(CH₂)₂CH(CH₃)C(O)NHCH₂-, -(CH₂)₃CH(CH₃)C(O)NHCH₂-, - (CH₂)₄CH(CH₃)C(O)NHCH₂-, -(CH₂)₅CH(CH₃)C(O)NHCH₂-, -(CH₂)₆CH(CH₃)C(O)NHCH₂-, - (CH₂)₇CH(CH₃)C(O)NHCH₂-, -(CH₂)₈CH(CH₃)C(O)NHCH₂-, -(CH₂)₉CH(CH₃)C(O)NHCH₂- or - (CH₂)₁₀CH(CH₃)C(O)NHCH₂-, wherein hydrogen(s) of one or more CH₂ (e.g., 1-5, 1-3 or 1-2 CH₂) of said group is optionally further replaced by a substituent(s) selected from the group consisting of C₁₋₃ alkyl (e.g., methyl, ethyl or propyl), C₁₋₃ alkoxy (e.g., methoxy, ethoxy or propoxy), C₃₋₆ cycloalkyl (e.g., cyclopropyl, cyclobutyl, cyclopentyl or cyclohexyl), halogenated C₁₋₃ alkyl (e.g., difluoromethyl or trifluoromethyl), halogen (e.g., fluorine, chlorine, bromine or iodine), hydroxy, cyano, amino, or any combination thereof. Herein, unless otherwise specified, either one of the two ends of the group represented by the -Xₐ-L₅-L₆- moiety may be attached to the group R¹, and the other end may be attached to X_{b}.

In one embodiment of the present disclosure, L₂ represents Formula -L₅-L₆-^{∗∗}, wherein symbol ** indicates the point of attachment of L₆ to R¹,
L₆ represents a bond; and
L₅ represents linear or branched C₁₋₄₀ alkylene (e.g., C₁₋₃₅ alkylene, C₁₋₃₀ alkylene, C₁₋₂₅ alkylene, C₁₋₂₃ alkylene, C₁₋₂₂ alkylene, C₁₋₂₁ alkylene, C₁₋₂₀ alkylene, C₁₋₁₉ alkylene, C₁₋₁₈ alkylene, C₁₋₁₇ alkylene, C₁₋₁₆ alkylene, C₁₋₁₅ alkylene, C₁₋₁₀ alkylene, C₁₋₉ alkylene, C₁₋₈ alkylene chain, C₁₋₇ alkylene, C₁₋₆ alkylene, C₁₋₅ alkylene, C₂₋₄ alkylene or C₁₋₃ alkylene) optionally substituted by a substituent(s) selected from the group consisting of C₁₋₃ alkyl (e.g., methyl, ethyl or propyl), C₁₋₃ alkoxy (e.g., methoxy, ethoxy or propoxy), C₃₋₆ cycloalkyl (e.g., cyclopropyl, cyclobutyl, cyclopentyl or cyclohexyl), halogenated C₁₋₃ alkyl (e.g., difluoromethyl or trifluoromethyl), halogen (e.g., fluorine, chlorine, bromine or iodine), hydroxy, cyano, amino or any combination thereof.

In one embodiment of the present disclosure, L₅ represents the following groups: -CH₂-, - (CH₂)₂-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, - (CH₂)₁₂-, -(CH₂)₁₃-, -(CH₂)₁₄-, -(CH₂)₁₅-, -(CH₂)₁₆-, -(CH₂)₁₇-, -(CH₂)₁₈-, -(CH₂)₁₉-, or -(CH₂)₂₀-; wherein hydrogen(s) of one or more CH₂ (e.g., 1-20, 1-15, 1-10, 1-8, 1-5, 1-3 or 1-2 CH₂) of the group is optionally further replaced by a substituent(s) selected from the group consisting of C₁₋₃ alkyl (e.g., methyl, ethyl or propyl), C₁₋₃ alkoxy (e.g., methoxy, ethoxy or propoxy), C₃₋₆ cycloalkyl (e.g., cyclopropyl, cyclobutyl, cyclopentyl or cyclohexyl), halogenated C₁₋₃ alkyl (e.g., difluoromethyl or trifluoromethyl), halogen (e.g., fluorine, chlorine, bromine or iodine), hydroxy, cyano, amino, or any combination thereof.

In one embodiment of the present disclosure, the L₂-R¹-L₁ moiety in Formula (I) can represent the following groups: 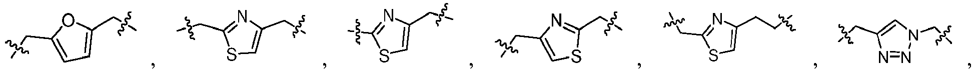 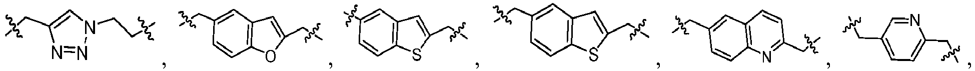 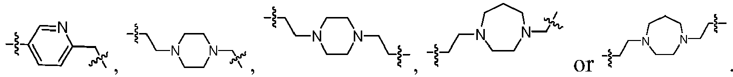 Herein, unless otherwise specified, either one of the two ends of the group represented by the L₂-R¹-L₁ moiety may be attached to the group R, and the other end may be attached to X₁.

In one embodiment of the present disclosure, X₁ represents Formula X_{b}-Xₐ-, wherein
Xₐ represents -(CH₂)₀₋₁₅-O-^{#}, -(CH₂)₀₋₁₅-S-^{#}, -(CH₂)₀₋₁₅-C(O)O-^{#}, -(CH₂)₀₋₁₅-OC(O)-^{#}, -(CH₂)₀₋₁₅-N(R³)-^{#}, -(CH₂)₀₋₁₅-C(O)N(R³)-^{#}, -(CH₂)₀₋₁₅-N(R³)C(O)-^{#}, -(CH₂)₀₋₁₅-S(O)₂N(R³)-^{#}, -(CH₂)₀₋₁₅-N(R³)S(O)₂-^{#}, -(CH₂)₀₋₁₅-S(O)₂O-^{#}, -(CH₂)₀₋₁₅-OS(O)₂-^{#}, -(CH₂)₀₋₁₅-S(O)₂-^{#}, -(CH₂)₀₋₁₅-S(O)₂-^{#}, alkynylene or alkenylene, wherein R³ represents H or C₁₋₃ alkyl, and symbol # represents the point of attachment to L₂, or
Xₐ represents a bond, provided that Xₐ, L₅ and L₆ do not simultaneously represent a bond, and when L₅ represents a bond, either Xₐ or L₆ represents a bond; and
X_{b} represents optionally substituted adamantanyl, optionally substituted noradamantanyl, optionally substituted meta-menthanyl, optionally substituted p-menthanyl, optionally substituted quinuclidinyl, optionally substituted bicyclo[2.2.1]heptyl or optionally substituted bicyclo[2.2.1]heptenyl, wherein said adamantanyl, said noradamantanyl, said meta-menthanyl, said *p-*menthanyl, said quinuclidinyl, said bicyclo[2.2.1]heptyl, and said bicyclo[2.2.1]heptenyl are each independently optionally substituted by a substituent(s) selected from the group consisting of C₁₋₃ alkyl (e.g., methyl, ethyl or propyl), halogenated C₁₋₃ alkyl (e.g., bifluoromethyl or trifluoromethyl), C₁₋₃ alkoxy (e.g., methoxy, ethoxy or propoxy), C₁₋₃ alkyl-NH- (e.g., CH₃-NH- or CH₃CH₂-NH-), amino-C₁₋₃ alkylene (e.g., NH₂-CH₂- or NH₂-CH₂CH₂-), amino, oxo, halogen (e.g., fluorine, chlorine, bromine or iodine), hydroxy, cyano, C₁₋₃ alkyl-NHC(O)- (e.g., CH₃NHC(O)-), mercapto, or any combination thereof.

In one embodiment of the present disclosure, X₁ represents Formula X_{b}-Xₐ-, wherein
Xₐ represents -(CH₂)₀₋₁₅-O-^{#}, -(CH₂)₀₋₁₅-S-^{#}, -(CH₂)₀₋₁₅-C(O)O-^{#}, -(CH₂)₀₋₁₅-OC(O)-^{#}, -(CH₂)₀₋₁₅-N(R³)-^{#}, -(CH₂)₀₋₁₅-C(O)N(R³)-^{#}, -(CH₂)₀₋₁₅-N(R³)C(O)-^{#}, -(CH₂)₀₋₁₅-S(O)₂N(R³)-^{#}, -(CH₂)₀₋₁₅-N(R³)S(O)₂-^{#}, -(CH₂)₀₋₁₅-S(O)₂O-^{#}, -(CH₂)₀₋₁₅-OS(O)₂-^{#}, -(CH₂)₀₋₁₅-S(O)₂-^{#}, -(CH₂)₀₋₁₅-S(O)₂-^{#}, alkynylene or alkenylene, wherein R³ represents H or C₁₋₃ alkyl (e.g., methyl, ethyl or propyl), and symbol # represents the point of attachment to L₂, and
X_{b} represents optionally substituted adamantanyl, optionally substituted noradamantanyl, optionally substituted meta-menthanyl, optionally substituted p-menthanyl, optionally substituted quinuclidinyl, optionally substituted bicyclo[2.2.1]heptyl or optionally substituted bicyclo[2.2.1]heptenyl, wherein said adamantanyl, said noradamantanyl, said meta-menthanyl, said *p-*menthanyl, said quinuclidinyl, said bicyclo[2.2.1]heptyl, and said bicyclo[2.2.1]heptenyl are each independently optionally substituted by a substituent(s) selected from the group consisting of C₁₋₃ alkyl (e.g., methyl, ethyl or propyl), halogenated C₁₋₃ alkyl (e.g., bifluoromethyl or trifluoromethyl), C₁₋₃ alkoxy (e.g., methoxy, ethoxy or propoxy), C₁₋₃ alkyl-NH- (e.g., CH₃-NH- or CH₃CH₂-NH-), amino-C₁₋₃ alkylene (e.g., NH₂-CH₂- or NH₂-CH₂CH₂-), amino, oxo, halogen (e.g., fluorine, chlorine, bromine or iodine), hydroxy, cyano, C₁₋₃ alkyl-NHC(O)- (e.g., CH₃NHC(O)-), mercapto, or any combination thereof.

In one embodiment of the present disclosure, Xₐ represents the following groups: O, -CH₂O-^{#}, -(CH₂)₂O-^{#}, -(CH₂)₃O-^{#}, -(CH₂)₄O-^{#}, -(CH₂)₅O-^{#}, -(CH₂)₆O-^{#}, -(CH₂)₇O-^{#}, -(CH₂)₈O-^{#}, -(CH₂)₉O-^{#}, - (CH₂)₁₀O-^{#}, S, -CH₂S-^{#}, -(CH₂)₂S-^{#}, -(CH₂)₃S-^{#}, -(CH₂)₄S-^{#}, -(CH₂)₅S-^{#}, -(CH₂)₆S-^{#}, -C(O)O-^{#}, - CH₂C(O)O-^{#}, -(CH₂)₃C(O)O-^{#}, -(CH₂)₄C(O)O-^{#}, -(CH₂)₅C(O)O-^{#}, -(CH₂)₆C(O)O-^{#}, -(CH₂)₇C(O)O-^{#}, - (CH₂)₈C(O)O-^{#}, -(CH₂)₉C(O)O-^{#}, -(CH₂)₁₀C(O)O-^{#}, -(CH₂)₂CH(CH₃)C(O)O-^{#}, - (CH₂)₃CH(CH₃)C(O)O-^{#}, -(CH₂)₄CH(CH₃)C(O)O-^{#}, -(CH₂)₅CH(CH₃)C(O)O-^{#}, - (CH₂)₆CH(CH₃)C(O)O-^{#}, -(CH₂)₇CH(CH₃)C(O)O-^{#}, -OC(O)-^{#}, -CH₂OC(O)-^{#}, -(CH₂)₂OC(O)-^{#}, - (CH₂)₃OC(O)-^{#}, -(CH₂)₄OC(O)-^{#}, -(CH₂)₅OC(O)-^{#}, -(CH₂)₆OC(O)-^{#}, -(CH₂)₇OC(O)-^{#}, -(CH₂)₈OC(O)-^{#}, -(CH₂)₉OC(O)-^{#}, -(CH₂)₁₀OC(O)-^{#}, -CH₂CH(CH₃)OC(O)-^{#}, -(CH₂)₂CH(CH₃)OC(O)-^{#}, - (CH₂)₃CH(CH₃)OC(O)-^{#}, -(CH₂)₄CH(CH₃)OC(O)-^{#}, -(CH₂)₅CH(CH₃)OC(O)-^{#}, - (CH₂)₆CH(CH₃)OC(O)-^{#}, -(CH₂)₇CH(CH₃)OC(O)-^{#}, -(CH₂)₈CH(CH₃)OC(O)-^{#}, NH, -CH₂NH-^{#}, - (CH₂)₂NH-^{#}, -(CH₂)₃NH-^{#}, -(CH₂)₄NH-^{#}, -(CH₂)₅NH-^{#}, -(CH₂)₆NH-^{#}, -(CH₂)₇NH-^{#}, -(CH₂)₈NH-^{#}, - (CH₂)₉NH-^{#}, -(CH₂)₁₀NH-^{#}, N(CH₃), -CH₂N(CH₃)-^{#}, -(CH₂)₂N(CH₃)-^{#}, -(CH₂)₃N(CH₃)-^{#}, - (CH₂)₄N(CH₃)-^{#}, -(CH₂)₅N(CH₃)-^{#}, -(CH₂)₆N(CH₃)-^{#}, -(CH₂)₇N(CH₃)-^{#}, -(CH₂)₈N(CH₃)-^{#}, - (CH₂)₉N(CH₃)-^{#}, -(CH₂)₁₀N(CH₃)-^{#}, -C(O)NH-^{#}, -CH₂C(O)NH-^{#}, -(CH₂)₂C(O)NH-^{#}, -(CH₂)₃C(O)NH-^{#}, -(CH₂)₄C(O)NH-^{#}, -(CH₂)₅C(O)NH-^{#}, -(CH₂)₆C(O)NH-^{#}, -(CH₂)₇C(O)NH-^{#}, -(CH₂)₈C(O)NH-^{#}, - (CH₂)₉C(O)NH-^{#}, -(CH₂)₁₀C(O)NH-^{#}, -CH₂CH(CH₃)C(O)NH-^{#}, -(CH₂)₂CH(CH₃)C(O)NH-^{#}, - (CH₂)₃CH(CH₃)C(O)NH-^{#}, -(CH₂)₄CH(CH₃)C(O)NH-^{#}, -(CH₂)₅CH(CH₃)C(O)NH-^{#}, - (CH₂)₆CH(CH₃)C(O)NH-^{#}, -(CH₂)₇CH(CH₃)C(O)NH-^{#}, -(CH₂)₈CH(CH₃)C(O)NH-^{#}, - (CH₂)₉CH(CH₃)C(O)NH-^{#}, -(CH₂)₁₀CH(CH₃)C(O)NH-^{#}, -NHC(O)-^{#}, -CH₂NHC(O)-^{#}, - (CH₂)₂NHC(O)-^{#}, -(CH₂)₃NHC(O)-^{#}, -(CH₂)₄NHC(O)-^{#}, -(CH₂)₅NHC(O)-^{#}, -(CH₂)₆NHC(O)-^{#}, - (CH₂)₇NHC(O)-^{#}, -(CH₂)₈NHC(O)-^{#}, -(CH₂)₉NHC(O)-^{#}, -(CH₂)₁₀NHC(O)-^{#}, -CH₂S(O)₂NH-^{#}, - (CH₂)₂S(O)₂NH-^{#}, -(CH₂)₃S(O)₂NH-^{#}, -(CH₂)₄S(O)₂NH-^{#}, -(CH₂)₅S(O)₂NH-^{#}, -(CH₂)₆S(O)₂NH-^{#}, - (CH₂)₇S(O)₂NH-^{#}, -(CH₂)₈S(O)₂NH-^{#}, -(CH₂)₉S(O)₂NH-^{#}, -(CH₂)₁₀S(O)₂NH-^{#}, -CH₂NHS(O)₂-^{#}, - (CH₂)₂NHS(O)₂-^{#}, -(CH₂)₃NHS(O)₂-^{#}, -(CH₂)₄NHS(O)₂-^{#}, -(CH₂)₅NHS(O)₂-^{#}, -(CH₂)₆NHS(O)₂-^{#}, - (CH₂)₇NHS(O)₂-^{#}, -(CH₂)₈NHS(O)₂-^{#}, -(CH₂)₉NHS(O)₂-^{#}, -(CH₂)₁₀NHS(O)₂-^{#}, -CH₂S(O)₂O-^{#}, - (CH₂)₂S(O)₂O-^{#}, -(CH₂)₃S(O)₂O-^{#}, -(CH₂)₄S(O)₂O-^{#}, -(CH₂)₅S(O)₂O-^{#}, -(CH₂)₆S(O)₂O-^{#}, - (CH₂)₇S(O)₂O-^{#}, -(CH₂)₈S(O)₂O-^{#}, -(CH₂)₉S(O)₂O-^{#}, -(CH₂)₁₀S(O)₂O-^{#}, -CH₂OS(O)₂-^{#}, - (CH₂)₂OS(O)₂-^{#}, -(CH₂)₃OS(O)₂-^{#}, -(CH₂)₄OS(O)₂-^{#}, -(CH₂)₅OS(O)₂-^{#}, -(CH₂)₆OS(O)₂-^{#}, - (CH₂)₇OS(O)₂-^{#}, -(CH₂)₈OS(O)₂-^{#}, -(CH₂)₉OS(O)₂-^{#}, -(CH₂)₁₀OS(O)₂-^{#}, ethynylene or vinylene; wherein symbol # indicates the point of attachment to L₂, and hydrogen(s) of one or more CH₂ (e.g., 1-20, 1-15, 1-10, 1-8, 1-5, 1-3 or 1-2 CH₂) of said group is optionally further replaced by a substituent(s) selected from the group consisting of C₁₋₃ alkyl (e.g., methyl, ethyl or propyl), C₁₋₃ alkoxy (e.g., methoxy, ethoxy or propoxy), C₃₋₆ cycloalkyl (e.g., cyclopropyl, cyclobutyl, cyclopentyl or cyclohexyl), halogenated C₁₋₃ alkyl (e.g., difluoromethyl or trifluoromethyl), halogen (e.g., fluorine, chlorine, bromine or iodine), hydroxy, cyano, amino, or any combination thereof.

In one embodiment of the present disclosure, X_{b} represents:
1-adamantanyl, 2-adamantanyl, 3-adamantanyl, 4-adamantanyl, 5-adamantanyl, 6-adamantanyl, 7-adamantanyl, 8-adamantanyl, 9-adamantanyl, 10-adamantanyl, haloadamantanyl, oxoadamantanyl, hydroxyadamantanyl, methyladamantanyl, dimethyladamantanyl, 1-noradamantanyl, 2-noradamantanyl, 3-noradamantanyl, 4-noradamantanyl, 5-noradamantanyl, 6-noradamantanyl, 7-noradamantanyl, 8-noradamantanyl, 9-noradamantanyl, p-menthanyl, meta-menthanyl, quinuclidinyl, bicyclo[2.2.1]heptan-2-yl, bicyclo[2.2.1]heptan-3-yl, bicyclo[2.2.1]heptan-4-yl, bicyclo[2.2.1]heptan-5-yl, bicyclo[2.2.1]heptan-6-yl, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl, 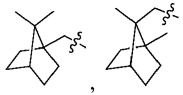 , 7,7-dimethyl-2-oxobicyclo[2.2.1]heptan-1-yl, 7,7-dimethyl-2-oxobicyclo[2.2.1]heptan-3-yl, 7,7-dimethyl-2-oxobicyclo[2.2.1]heptan-4-yl, 7,7-dimethyl-2-oxobicyclo[2.2.1]heptan-5-yl, 7,7-dimethyl-2-oxobicyclo[2.2.1]heptan-6-yl, bicyclo[2.2.1]hept-5-en-2-yl, bicyclo[2.2.1]hept-5-en-3-yl, or bicyclo[2.2.1]hept-5-en-7-yl.

In one embodiment of the present disclosure, X_{b} represents:

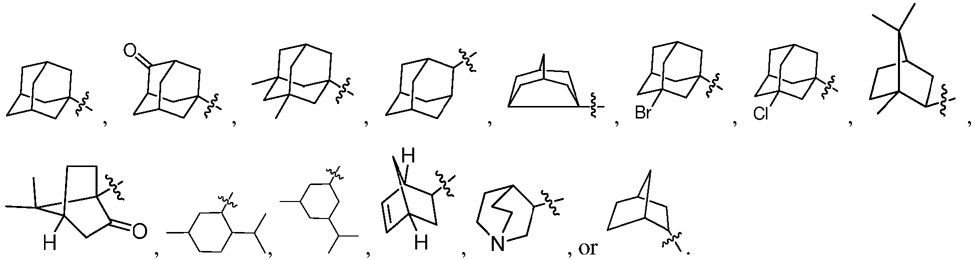

In one embodiment of the present disclosure, the compound of Formula (I) is also a compound of Formula (Ia): 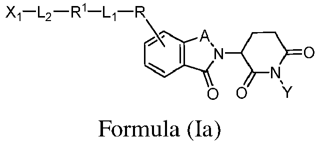 wherein Y, A, B, U, V, W, R, L₁, R¹, L₂ and X₁ are as defined in Formula (I) above, and also as defined in various embodiments thereof.

In one embodiment of the present disclosure, the compound of Formula (I) is also a compound of Formula (Ib): 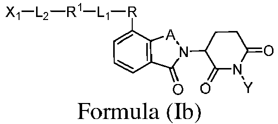 wherein Y, A, B, U, V, W, R, L₁, R¹, L₂ and X₁ are as defined in Formula (I) above, and also as defined in various embodiments thereof.

In one embodiment of the present disclosure, the compound of Formula (I) is also a compound of Formula (Ic): 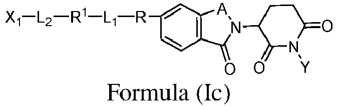 wherein Y, A, B, U, V, W, R, L₁, R¹, L₂ and X₁ are as defined in Formula (I) above, and also as defined in various embodiments thereof.

In one embodiment of the present disclosure, the compound of Formula (I) is also a compound of Formula (Id): 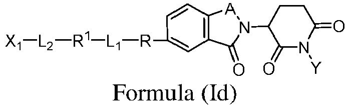 wherein Y, A, B, U, V, W, R, L₁, R¹, L₂ and X₁ are as defined in Formula (I) above, and also as defined in various embodiments thereof.

In one embodiment of the present disclosure, the compound of Formula (I) is also a compound of Formula (Ie): 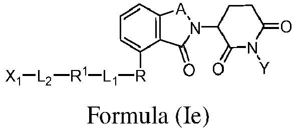 wherein Y, A, B, U, V, W, R, L₁, R¹, L₂ and X₁ are as defined in Formula (I) above, and also as defined in various embodiments thereof.

Particularly preferred are the compounds of the present invention and their salts (especially pharmaceutically acceptable salts, such as hydrochloride, etc.), enantiomers, stereoisomers (including diastereomers), solvates, or polymorphs thereof in Table 1:

**Table 1. The compounds of the present invention**

| Compound No. | Structure of the compounds | The compounds' name | Chinese translation of the compounds' name |
|---|---|---|---|
| SIAIS313 118 | 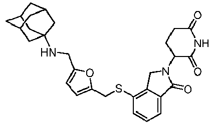 | 3-(4-(((5-((adamantan-1-ylamino)methyl)furan-2-yl)methyl)thio)-1-oxoisoindolin-2-yl)piperidine-2,6-dione | 3-(4-(((5-((adamantan-1-ylamino)methyl)furan-2-yl)methyl)thio)-1-oxoisoindolin-2-yl)piperidine-2,6-dione |
| | 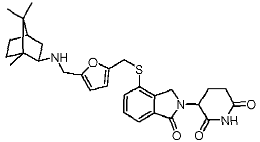 | 3-(1-oxo-4-(((5-(((1,7,7-trimethylbicyclo[2.2.1]hep tan-2-yl)amino)methyl)furan-2-yl)methyl)thio)isoindolin-2-yl)piperidine-2,6-dione | 3-(1-oxo-4-(((5-(((1,7,7-trimethylbicyclo[2.2.1]h eptan-2-yl)amino)methyl)furan-2-yl)methyl)thio)isoindolin -2-yl)piperidine-2,6-dione |
| | 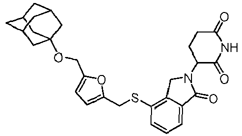 | 3-(4-(((5-((adamantan-1-yloxy)methyl)furan-2-yl)methyl)thio)-1-oxoisoindolin-2-yl)piperidine-2,6-dione | 3-(4-(((5-((adamantan-1-yloxy)methyl)furan-2-yl)methyl)thio)-1-oxoisoindolin-2-yl)piperidine-2,6-dione |
| | 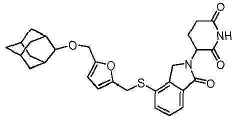 | 3-(4-(((5-((adamantan-2-yloxy)methyl)furan-2-yl)methyl)thio)-1-oxoisoindolin-2-yl)piperidine-2,6-dione | 3-(4-(((5-((adamantan-2-yloxy)methyl)furan-2-yl)methyl)thio)-1-oxoisoindolin-2-yl)piperidine-2,6-dione |
| | 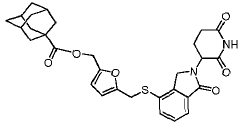 | (5-(((2-(2,6-dioxopiperidin-3-yl)-1-oxoisoindolin-4-yl)thio)methyl)furan-2-yl)methyl adamantane-1-carboxylate | (5-(((2-(2,6-dioxopiperidin-3-yl)-1-oxoisoindolin-4-yl)thio)methyl)furan-2-yl)methyl adamantane-1-carboxylate |
| | 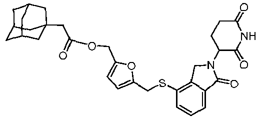 | (5-(((2-(2,6-dioxopiperidin-3-yl)-1-oxoisoindolin-4-yl)thio)methyl)furan-2-yl)methyl 2-(adamantan-1-yl)acetate | (5-(((2-(2,6-dioxopiperidin-3-yl)-1 - oxoisoindolin-4-yl)thio)methyl)furan-2-yl)methyl 2-(adamantan-1-yl)acetate |
| | 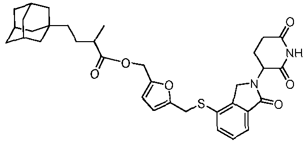 | (5-(((2-(2,6-dioxopiperidin-3-yl)-1 - oxoisoindolin-4-yl)thio)methyl)furan-2-yl)methyl 4-(adamantan-1-yl)-2-methylbutanoate | (5-(((2-(2,6-dioxopiperidin-3-yl)-1 - oxoisoindolin-4-yl)thio)methyl)furan-2-yl)methyl 4-(adamantan-1-yl)-2-methylbutanoate |
| | 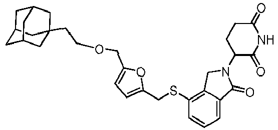 | 3-(4-(((5-((2-(adamantan-1 -yl)ethoxy)methyl)furan-2-yl)methyl)thio)-1-oxoisoindolin-2-yl)piperidine-2,6-dione | 3-(4-(((5-((2-(adamantan-1-yl)ethoxy)methyl)furan-2-yl)methyl)thio)-1-oxoisoindolin-2-yl)piperidine-2,6-dione |
| | 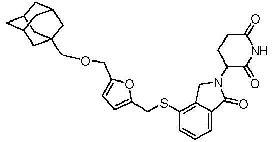 | 3-(4-(((5-((adamantan-1-ylmethoxy)methyl)furan-2-yl)methyl)thio)-1-oxoisoindolin-2-yl)piperidine-2,6-dione | 3-(4-(((5-((adamantan-1-ylmethoxy)methyl)furan -2-yl)methyl)thio)-1-oxoisoindolin-2-yl)piperidine-2,6-dione |
| | 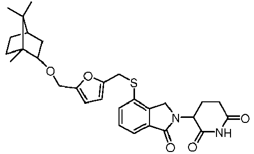 | 3-(1-oxo-4-(((5-(((1,7,7-trimethylbicyclo[2.2.1]hep tan-2-yl)oxy)methyl)furan-2-yl)methyl)thio)isoindolin-2-yl)piperidine-2,6-dione | 3-(1-oxo-4-(((5-(((1,7,7-trimethylbicyclo[2.2.1]h eptan-2-yl)oxy)methyl)furan-2-yl)methyl)thio)isoindolin -2-yl)piperidine-2,6-dione |
| | 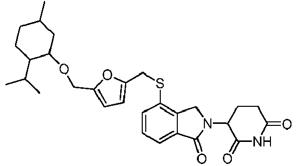 | 3-(4-(((5-(((2-isopropyl-5-methylcyclohexyl)oxy)met hyl)furan-2-yl)methyl)thio)-1-oxoisoindolin-2-yl)piperidine-2,6-dione | 3-(4-(((5-(((2-isopropyl-5-methylcyclohexyl)oxy)m ethyl)furan-2-yl)methyl)thio)-1-oxoisoindolin-2-yl)piperidine-2,6-dione |
| | 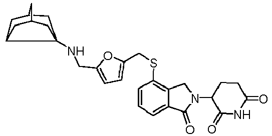 | 3-(4-(((5-(((hexahydro-2,5-methanopentalen-3a(1H)-yl)amino)methyl)furan-2-yl)methyl)thio)-1-oxoisoindolin-2-yl)piperidine-2,6-dione | 3-(4-(((5-(((hexahydro-2,5-methanopentalen-3a(1H)-yl)amino)methyl)furan-2-yl)methyl)thio)-1-oxoisoindolin-2-yl)piperidine-2,6-dione |
| SIAIS313 160 | 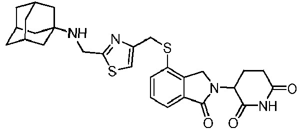 | 3-(4-(((2-((adamantan-1-ylamino)methyl)thiazol-4-yl)methyl)thio)-1-oxoisoindolin-2-yl)piperidine-2,6-dione | 3-(4-(((2-((adamantan-1-ylamino)methyl)thiazol-4-yl)methyl)thio)-1-oxoisoindolin-2-yl)piperidine-2,6-dione |
| | 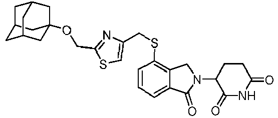 | 3-(4-(((2-((adamantan-1-yloxy)methyl)thiazol-4-yl)methyl)thio)-1-oxoisoindolin-2-yl)piperidine-2,6-dione | 3-(4-(((2-((adamantan-1-yloxy)methyl)thiazol-4-yl)methyl)thio)-1-oxoisoindolin-2-yl)piperidine-2,6-dione |
| | 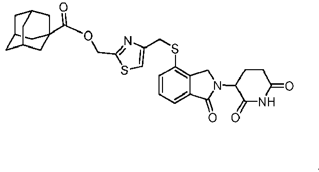 | (4-(((2-(2,6-dioxopiperidin-3-yl)-1 - oxoisoindolin-4-yl)thio)methyl)thiazol-2-yl)methyl adamantane-1-carboxylate | (4-(((2-(2,6-dioxopiperidin-3-yl)-1-oxoisoindolin-4-yl)thio)methyl)thiazol-2-yl)methyl adamantane-1-carboxylate |
| | 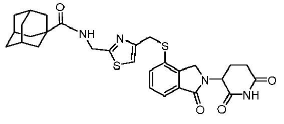 | N-((4-(((2-(2,6-dioxopiperidin-3-yl)-1 - oxoisoindolin-4-yl)thio)methyl)thiazol-2-yl)methyl)adamantane-1-carboxamide | N-((4-(((2-(2,6-dioxopiperidin-3-yl)-1-oxoisoindolin-4-yl)thio)methyl)thiazol-2-yl)methyl)adamantane-1-carboxamide |
| | 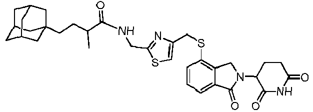 | 4-(adamantan-1-yl)-N-((4-(((2-(2,6-dioxopiperidin-3-yl)-1-oxoisoindolin-4-yl)thio)methyl)thiazol-2-yl)methyl)-2-methylbutanamide | 4-(adamantan-1-yl)-N-((4-(((2-(2,6-dioxopiperidin-3-yl)-1-oxoisoindolin-4-yl)thio)methyl)thiazol-2-yl)methyl)-2-methylbutanamide |
| | 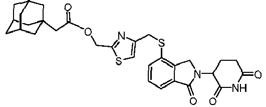 | (4-(((2-(2,6-dioxopiperidin-3-yl)-1-oxoisoindolin-4-yl)thio)methyl)thiazol-2-yl)methyl 2-(adamantan-1-yl)acetate | (4-(((2-(2,6-dioxopiperidin-3-yl)-1-oxoisoindolin-4-yl)thio)methyl)thiazol-2-yl)methyl 2-(adamantan-1-yl)acetate |
| | 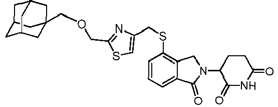 | 3-(4-(((2-((adamantan-1-ylmethoxy)methyl)thiazol-4-yl)methyl)thio)-1-oxoisoindolin-2-yl)piperidine-2,6-dione | 3-(4-(((2-((adamantan-1-ylmethoxy)methyl)thiaz ol-4-yl)methyl)thio)-1 - oxoisoindolin-2-yl)piperidine-2,6-dione |
| | 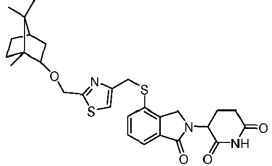 | 3-(1-oxo-4-(((2-(((1,7,7-trimethylbicyclo[2.2.1]hep tan-2-yl)oxy)methyl)thiazol-4-yl)methyl)thio)isoindolin-2-yl)piperidine-2,6-dione | 3-(1-oxo-4-(((2-(((1,7,7-trimethylbicyclo[2.2.1]h eptan-2-yl)oxy)methyl)thiazol-4-yl)methyl)thio)isoindolin -2-yl)piperidine-2,6-dione |
| | 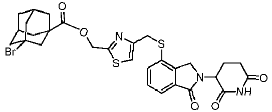 | (4-(((2-(2,6-dioxopiperidin-3-yl)-1-oxoisoindolin-4-yl)thio)methyl)thiazol-2-yl)methyl 3-bromoadamantane-1 - carboxylate | (4-(((2-(2,6-dioxopiperidin-3-yl)-1-oxoisoindolin-4-yl)thio)methyl)thiazol-2-yl)methyl 3-bromoadamantane-1- carboxylate |
| SIAIS355 072 | 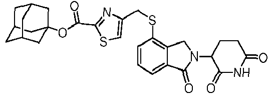 | adamantan-1-yl 4-(((2-(2,6-dioxopiperidin-3-yl)-1-oxoisoindolin-4-yl)thio)methyl)thiazole-2-carboxylate | adamantan-1-yl 4-(((2-(2,6-dioxopiperidin-3-yl)-1 -oxoisoindolin-4-yl)thio)methyl)thiazole-2-carboxylate |
| | 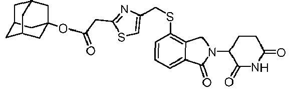 | adamantan-1-yl 2-(4-(((2-(2,6-dioxopiperidin-3-yl)-1 -oxoisoindolin-4-yl)thio)methyl)thiazol-2-yl)acetate | adamantan-1-yl 2-(4-(((2-(2,6-dioxopiperidin-3-yl)-1 -oxoisoindolin-4-yl)thio)methyl)thiazol-2-yl)acetate |
| | 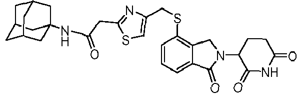 | N-(adamantan-1-yl)-2-(4-(((2-(2,6-dioxopiperidin-3-yl)-1-oxoisoindolin-4-yl)thio)methyl)thiazol-2-yl)acetamide | N-(adamantan-1-yl)-2-(4-(((2-(2,6-dioxopiperidin-3-yl)-1-oxoisoindolin-4-yl)thio)methyl)thiazol-2-yl)acetamide |
| SIAIS355 073 | 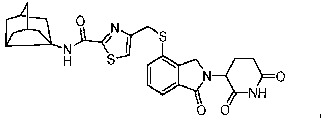 | 4-(((2-(2,6-dioxopiperidin-3-yl)-1-oxoisoindolin-4-yl)thio)methyl)-N-(octahydro-2,5-methanopentalen-6a-yl)thiazole-2-carboxamide | 4-(((2-(2,6-dioxopiperidin-3-yl)-1-oxoisoindolin-4-yl)thio)methyl)-N-(octahydro-2,5-methanopentalen-6a-yl)thiazole-2-carboxamide |
| | 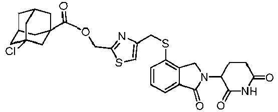 | (4-(((2-(2,6-dioxopiperidin-3-yl)-1 - oxoisoindolin-4-yl)thio)methyl)thiazol-2-yl)methyl 3-chloroadamantane-1-carboxylate | (4-(((2-(2,6-dioxopiperidin-3-yl)-1-oxoisoindolin-4-yl)thio)methyl)thiazol-2-yl)methyl 3-chloroadamantane-1- carboxylate |
| | 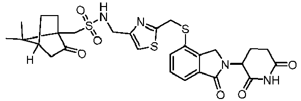 | 1-(7,7-dimethyl-2-oxobicyclo[2.2.1]heptan-1-yl)-N-((2-(((2-(2,6-dioxopiperidin-3-yl)-1- oxoisoindolin-4-yl)thio)methyl)thiazol-4-yl)methyl)methanesulfona mide | 1-(7,7-dimethyl-2-oxobicyclo[2.2.1]heptan-1-yl)-N-((2-(((2-(2,6-dioxopiperidin-3-yl)-1-oxoisoindolin-4-yl)thio)methyl)thiazol-4-yl)methyl)methanesulfon amide |
| | 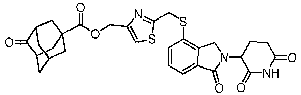 | (2-(((2-(2,6-dioxopiperidin-3-yl)-1-oxoisoindolin-4-yl)thio)methyl)thiazol-4-yl)methyl 4-oxoadamantane-1 - carboxylate | (2-(((2-(2,6-dioxopiperidin-3-yl)-1-oxoisoindolin-4-yl)thio)methyl)thiazol-4-yl)methyl 4-oxoadamantane-1- carboxylate |
| SIAIS313 185 | 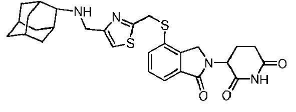 | 3-(4-(((4-((adamantan-2-ylamino)methyl)thiazol-2-yl)methyl)thio)-1-oxoisoindolin-2-yl)piperidine-2,6-dione | 3-(4-(((4-((adamantan-2-ylamino)methyl)thiazol-2-yl)methyl)thio)-1-oxoisoindolin-2-yl)piperidine-2,6-dione |
| | 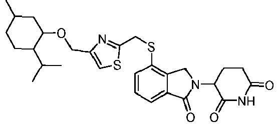 | 3-(4-(((4-(((2-isopropyl-5-methylcyclohexyl)oxy)met hyl)thiazol-2-yl)methyl)thio)-1-oxoisoindolin-2-yl)piperidine-2,6-dione | 3-(4-(((4-(((2-isopropyl-5-methylcyclohexyl)oxy)m ethyl)thiazol-2-yl)methyl)thio)-1-oxoisoindolin-2-yl)piperidine-2,6-dione |
| | 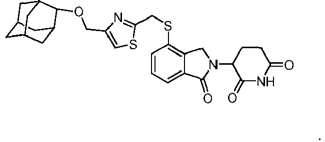 | 3-(4-(((4-((adamantan-2-yloxy)methyl)thiazol-2-yl)methyl)thio)-1-oxoisoindolin-2-yl)piperidine-2,6-dione | 3-(4-(((4-((adamantan-2-yloxy)methyl)thiazol-2-yl)methyl)thio)-1-oxoisoindolin-2-yl)piperidine-2,6-dione |
| | 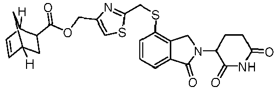 | (2-(((2-(2,6-dioxopiperidin-3-yl)-1-oxoisoindolin-4-yl)thio)methyl)thiazol-4-yl)methyl bicyclo[2.2.1]hept-5-ene-2-carboxylate | (2-(((2-(2,6-dioxopiperidin-3-yl)-1-oxoisoindolin-4-yl)thio)methyl)thiazol-4-yl)methyl bicyclo[2.2.1]hept-5-ene-2-carboxylate |
| | 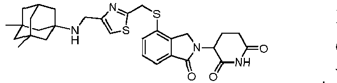 | 3-(4-(((4-(((3,5-dimethyladamantan-1-yl)amino)methyl)thiazol-2-yl)methyl)thio)-1-oxoisoindolin-2-yl)piperidine-2,6-dione | 3-(4-(((4-(((3,5-dimethyladamantan-1-yl)amino)methyl)thiazol-2-yl)methyl)thio)-1-oxoisoindolin-2-yl)piperidine-2,6-dione |
| | 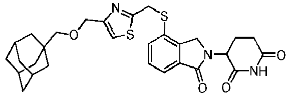 | 3-(4-(((4-((adamantan-1 - ylmethoxy)methyl)thiazol-2-yl)methyl)thio)-1-oxoisoindolin-2-yl)piperidine-2,6-dione | 3-(4-(((4-((adamantan-1- ylmethoxy)methyl)thiaz ol-2-yl)methyl)thio)-1-oxoisoindolin-2-yl)piperidine-2,6-dione |
| | 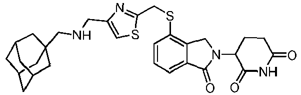 | 3-(4-(((4-(((adamantan-1-ylmethyl)amino)methyl)th iazol-2-yl)methyl)thio)-1 - oxoisoindolin-2-yl)piperidine-2,6-dione | 3-(4-(((4-(((adamantan-1-ylmethyl)amino)methyl)t hiazol-2-yl)methyl)thio)-1-oxoisoindolin-2-yl)piperidine-2,6-dione |
| SIAIS287 129 | 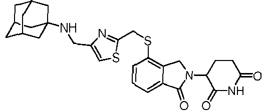 | 3-(4-(((4-((adamantan-1-ylamino)methyl)thiazol-2-yl)methyl)thio)-1-oxoisoindolin-2-yl)piperidine-2,6-dione | 3-(4-(((4-((adamantan-1-ylamino)methyl)thiazol-2-yl)methyl)thio)-1-oxoisoindolin-2-yl)piperidine-2,6-dione |
| | 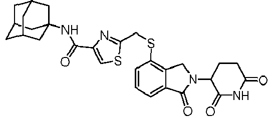 | N-(adamantan-1-yl)-2-(((2-(2,6-dioxopiperidin-3-yl)-1-oxoisoindolin-4-yl)thio)methyl)thiazole-4-carboxamide | N-(adamantan-1-yl)-2-(((2-(2,6-dioxopiperidin-3-yl)-1-oxoisoindolin-4-yl)thio)methyl)thiazole-4-carboxamide |
| | 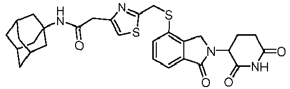 | N-(adamantan-1-yl)-2-(2-(((2-(2,6-dioxopiperidin-3-yl)-1 -oxoisoindolin-4-yl)thio)methyl)thiazol-4-yl)acetamide | N-(adamantan-1-yl)-2-(2-(((2-(2,6-dioxopiperidin-3-yl)-1-oxoisoindolin-4-yl)thio)methyl)thiazol-4-yl)acetamide |
| | 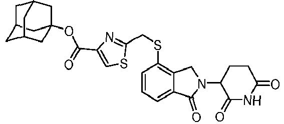 | adamantan-1-yl 2-(((2-(2,6-dioxopiperidin-3-yl)-1 -oxoisoindolin-4-yl)thio)methyl)thiazole-4-carboxylate | adamantan-1-yl 2-(((2-(2,6-dioxopiperidin-3-yl)-1 -oxoisoindolin-4-yl)thio)methyl)thiazole-4-carboxylate |
| SIAIS287 097 | 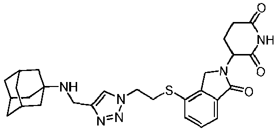 | 3-(4-((2-(4-((adamantan-1-ylamino)methyl)-1H-1,2,3-triazol-1-yl)ethyl)thio)-1-oxoisoindolin-2-yl)piperidine-2,6-dione | 3-(4-((2-(4-((adamantan-1-ylamino)methyl)-1H-1,2,3-triazol-1-yl)ethyl)thio)-1-oxoisoindolin-2-yl)piperidine-2,6-dione |
| | 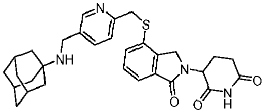 | 3-(4-(((5-(((adamantan-1-yl)amino)methyl)pyridin-2-yl)methyl)thio)-1-oxoisoindolin-2-yl)piperidine-2,6-dione | 3-(4-(((5-(((adamantan-1-yl)amino)methyl)pyridin -2-yl)methyl)thio)-1-oxoisoindolin-2-yl)piperidine-2,6-dione |
| | 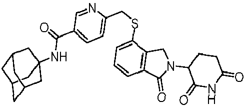 | N-(adamantan-1-yl)-6-(((2-(2,6-dioxopiperidin-3-yl)-1-oxoisoindolin-4-yl)thio)methyl)nicotinami de | N-(adamantan-1-yl)-6-(((2-(2,6-dioxopiperidin-3-yl)-1-oxoisoindolin-4-yl)thio)methyl)nicotinam ide |
| | 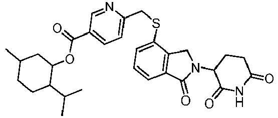 | 2-isopropyl-5-methylcyclohexyl 6-(((2-(2,6-dioxopiperidin-3-yl)-1-oxoisoindolin-4-yl)thio)methyl)nicotinate | 2-isopropyl-5-methylcyclohexyl 6-(((2-(2,6-dioxopiperidin-3-yl)-1-oxoisoindolin-4-yl)thio)methyl)nicotinate |
| | 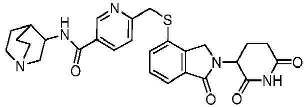 | 6-(((2-(2,6-dioxopiperidin-3-yl)-1-oxoisoindolin-4-yl)thio)methyl)-N-(quinuclidin-3-yl)nicotinamide | 6-(((2-(2,6-dioxopiperidin-3-yl)-1-oxoisoindolin-4-yl)thio)methyl)-N-(quinuclidin-3-yl)nicotinamide |
| | 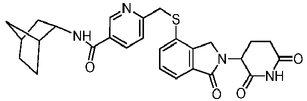 | N-(bicyclo[2.2.1]heptan-2-yl)-6-(((2-(2,6-dioxopiperidin-3-yl)-1-oxoisoindolin-4-yl)thio)methyl)nicotinami de | N-(bicyclo[2.2.1]heptan-2-yl)-6-(((2-(2,6-dioxopiperidin-3-yl)-1-oxoisoindolin-4-yl)thio)methyl)nicotinam ide |
| | 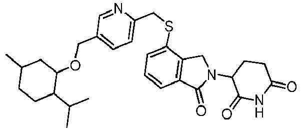 | 3-(4-(((5-(((2-isopropyl-5-methylcyclohexyl)oxy)met hyl)pyridin-2-yl)methyl)thio)-1-oxoisoindolin-2-yl)piperidine-2,6-dione | 3-(4-(((5-(((2-isopropyl-5-methylcyclohexyl)oxy)m ethyl)pyridin-2-yl)methyl)thio)-1-oxoisoindolin-2-yl)piperidine-2,6-dione |
| | 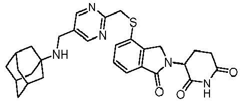 | 3-(4-(((5-(((adamantan-1-yl)amino)methyl)pyrimidi n-2-yl)methyl)thio)-1-oxoisoindolin-2-yl)piperidine-2,6-dione | 3-(4-(((5-(((adamantan-1-yl)amino)methyl)pyrimi din-2-yl)methyl)thio)-1 - oxoisoindolin-2-yl)piperidine-2,6-dione |
| SIAIS287 116 | 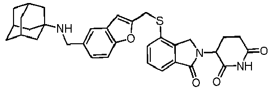 | 3-(4-(((5-((adamantan-1-ylamino)methyl)benzofura n-2-yl)methyl)thio)-1 - oxoisoindolin-2-yl)piperidine-2,6-dione | 3-(4-(((5-((adamantan-1-ylamino)methyl)benzofu ran-2-yl)methyl)thio)-1-oxoisoindolin-2-yl)piperidine-2,6-dione |
| SIAIS338 048 | 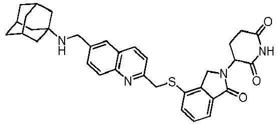 | 3-(4-(((6-((adamantan-1 - ylamino)methyl)quinolin-2-yl)methyl)thio)-1-oxoisoindolin-2-yl)piperidine-2,6-dione | 3-(4-(((6-((adamantan-1 - ylamino)methyl)quinolin -2-yl)methyl)thio)-1-oxoisoindolin-2-yl)piperidine-2,6-dione |
| SIAIS338 091 | 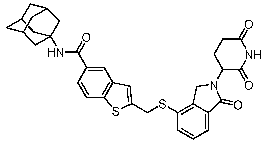 | N-(adamantan-1 -yl)-2-(((2-(2,6-dioxopiperidin-3-yl)-1-oxoisoindolin-4-yl)thio)methyl)benzo[b]thi ophene-5-carboxamide | N-(adamantan-1-yl)-2-(((2-(2,6-dioxopiperidin-3-yl)-1-oxoisoindolin-4-yl)thio)methyl)benzo[b]t hiophene-5-carboxamide |
| | 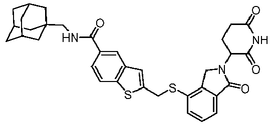 | N-((adamantan-1-yl)methyl)-2-(((2-(2,6-dioxopiperidin-3-yl)-1-oxoisoindolin-4-yl)thio)methyl)benzo[b]thi ophene-5-carboxamide | N-((adamantan-1-yl)methyl)-2-(((2-(2,6-dioxopiperidin-3-yl)-1-oxoisoindolin-4-yl)thio)methyl)benzo[b]t hiophene-5-carboxamide |
| | 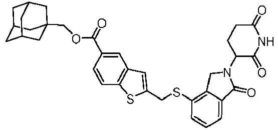 | (adamantan-1-yl)methyl 2-(((2-(2,6-dioxopiperidin-3-yl)-1 -oxoisoindolin-4-yl)thio)methyl)benzo[b]thi ophene-5-carboxylate | (adamantan-1-yl)methyl 2-(((2-(2,6-dioxopiperidin-3-yl)-1-oxoisoindolin-4-yl)thio)methyl)benzo[b]t hiophene-5-carboxylate |
| | 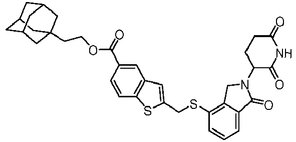 | 2-(adamantan-1-yl)ethyl 2-(((2-(2,6-dioxopiperidin-3-yl)-1 -oxoisoindolin-4-yl)thio)methyl)benzo[b]thi ophene-5-carboxylate | 2-(adamantan-1-yl)ethyl 2-(((2-(2,6-dioxopiperidin-3-yl)-1-oxoisoindolin-4-yl)thio)methyl)benzo[b]t hiophene-5-carboxylate |
| | 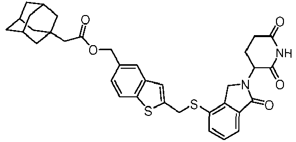 | (2-(((2-(2,6-dioxopiperidin-3-yl)-1-oxoisoindolin-4-yl)thio)methyl)benzo[b]thi ophen-5-yl)methyl 2-(adamantan-1-yl)acetate | (2-(((2-(2,6-dioxopiperidin-3-yl)-1-oxoisoindolin-4-yl)thio)methyl)benzo[b]t hiophen-5-yl)methyl 2-(adamantan-1-yl)acetate |
| | 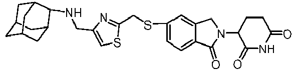 | 3-(5-(((4-(((adamantan-2-yl)amino)methyl)thiazol-2-yl)methyl)thio)-1-oxoisoindolin-2-yl)piperidine-2,6-dione | 3-(5-(((4-(((adamantan-2-yl)amino)methyl)thiazol-2-yl)methyl)thio)-1-oxoisoindolin-2-yl)piperidine-2,6-dione |
| | 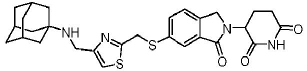 | 3-(6-(((4-(((adamantan-1 - yl)amino)methyl)thiazol-2-yl)methyl)thio)-1-oxoisoindolin-2-yl)piperidine-2,6-dione | 3-(6-(((4-(((adamantan-1-yl)amino)methyl)thiazol-2-yl)methyl)thio)-1-oxoisoindolin-2-yl)piperidine-2,6-dione |
| | 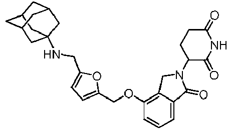 | 3-(4-((5-((adamantan-1-ylamino)methyl)furan-2-yl)methoxy)-1-oxoisoindolin-2-yl)piperidine-2,6-dione | 3-(4-((5-((adamantan-1-ylamino)methyl)furan-2-yl)methoxy)-1-oxoisoindolin-2-yl)piperidine-2,6-dione |
| | 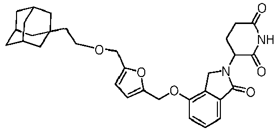 | 3-(4-((5-((2-(adamantan-1-yl)ethoxy)methyl)furan-2-yl)methoxy)-1-oxoisoindolin-2-yl)piperidine-2,6-dione | 3-(4-((5-((2-(adamantan-1-yl)ethoxy)methyl)furan-2-yl)methoxy)-1-oxoisoindolin-2-yl)piperidine-2,6-dione |
| | 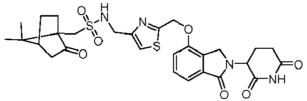 | 1-(7,7-dimethyl-2-oxobicyclo[2.2.1]heptan-1-yl)-N-((2-(((2-(2,6-dioxopiperidin-3-yl)-1-oxoisoindolin-4-yl)oxy)methyl)thiazol-4-yl)methyl)methanesulfona mide | 1-(7,7-dimethyl-2-oxobicyclo[2.2.1]heptan-1-yl)-N-((2-(((2-(2,6-dioxopiperidin-3-yl)-1-oxoisoindolin-4-yl)oxy)methyl)thiazol-4-yl)methyl)methanesulfon amide |
| | 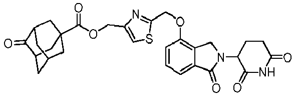 | (2-(((2-(2,6-dioxopiperidin-3-yl)-1-oxoisoindolin-4-yl)oxy)methyl)thiazol-4-yl)methyl 4-oxoadamantane-1 - carboxylate | (2-(((2-(2,6-dioxopiperidin-3-yl)-1-oxoisoindolin-4-yl)oxy)methyl)thiazol-4-yl)methyl 4-oxoadamantane-1 - carboxylate |
| | 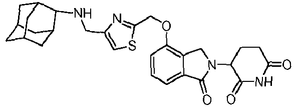 | 3-(4-((4-((adamantan-2-ylamino)methyl)thiazol-2-yl)methoxy)-1-oxoisoindolin-2-yl)piperidine-2,6-dione | 3-(4-((4-((adamantan-2-ylamino)methyl)thiazol-2-yl)methoxy)-1-oxoisoindolin-2-yl)piperidine-2,6-dione |
| | 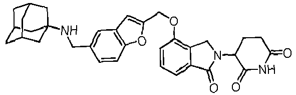 | 3-(4-((5-((adamantan-1-ylamino)methyl)benzofura n-2-yl)methoxy)-1-oxoisoindolin-2-yl)piperidine-2,6-dione | 3-(4-((5-((adamantan-1-ylamino)methyl)benzofu ran-2-yl)methoxy)-1-oxoisoindolin-2-yl)piperidine-2,6-dione |
| | 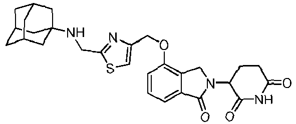 | 3-(4-((2-((adamantan-1-ylamino)methyl)thiazol-4-yl)methoxy)-1-oxoisoindolin-2-yl)piperidine-2,6-dione | 3-(4-((2-((adamantan-1-ylamino)methyl)thiazol-4-yl)methoxy)-1-oxoisoindolin-2-yl)piperidine-2,6-dione |
| | 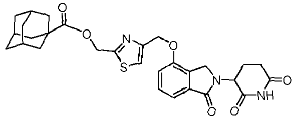 | (4-(((2-(2,6-dioxopiperidin-3-yl)-1-oxoisoindolin-4-yl)oxy)methyl)thiazol-2-yl)methyl adamantane-1-carboxylate | (4-(((2-(2,6-dioxopiperidin-3-yl)-1-oxoisoindolin-4-yl)oxy)methyl)thiazol-2-yl)methyl adamantane-1-carboxylate |
| | 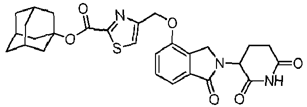 | adamantan-1-yl 4-(((2-(2,6-dioxopiperidin-3-yl)-1-oxoisoindolin-4-yl)oxy)methyl)thiazole-2-carboxylate | adamantan-1-yl 4-(((2-(2,6-dioxopiperidin-3-yl)-1-oxoisoindolin-4-yl)oxy)methyl)thiazole-2-carboxylate |
| | 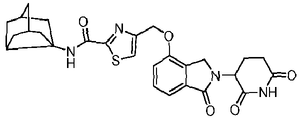 | 4-(((2-(2,6-dioxopiperidin-3-yl)-1-oxoisoindolin-4-yl)oxy)methyl)-N-(octahydro-2,5-methanopentalen-6a-yl)thiazole-2-carboxamide | 4-(((2-(2,6-dioxopiperidin-3-yl)-1-oxoisoindolin-4-yl)oxy)methyl)-N-(octahydro-2,5-methanopentalen-6a-yl)thiazole-2-carboxamide |
| | 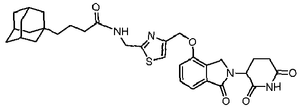 | 4-(adamantan-1-yl)-N-((4-(((2-(2,6-dioxopiperidin-3-yl)-1-oxoisoindolin-4-yl)oxy)methyl)thiazol-2-yl)methyl)-2-methylbutanamide | 4-(adamantan-1-yl)-N-((4-(((2-(2,6-dioxopiperidin-3-yl)-1- oxoisoindolin-4-yl)oxy)methyl)thiazol-2-yl)methyl)-2-methylbutanamide |
| | 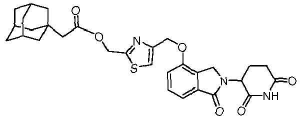 | (4-(((2-(2,6-dioxopiperidin-3-yl)-1-oxoisoindolin-4-yl)oxy)methyl)thiazol-2-yl)methyl 2-(adamantan-1-yl)acetate | (4-(((2-(2,6-dioxopiperidin-3-yl)-1-oxoisoindolin-4-yl)oxy)methyl)thiazol-2-yl)methyl 2-(adamantan-1-yl)acetate |
| | 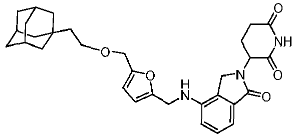 | 3-(4-(((5-((2-(adamantan-1 -yl)ethoxy)methyl)furan-2-yl)methyl)amino)-1- oxoisoindolin-2-yl)piperidine-2,6-dione | 3-(4-(((5-((2-(adamantan-1-yl)ethoxy)methyl)furan-2-yl)methyl)amino)-1- oxoisoindolin-2-yl)piperidine-2,6-dione |
| | 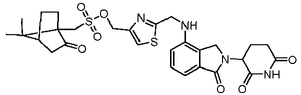 | (2-(((2-(2,6-dioxopiperidin-3-yl)-1-oxoisoindolin-4-yl)amino)methyl)thiazol-4-yl)methyl 7,7-dimethyl-2-oxobicyclo[2.2.1]heptan-1-yl)methanesulfonate | (2-(((2-(2,6-dioxopiperidin-3-yl)-1-oxoisoindolin-4-yl)amino)methyl)thiazol-4-yl)methyl 7,7-dimethyl-2-oxobicyclo[2.2.1]heptan-1-yl)methanesulfonate |
| | 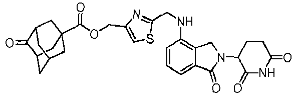 | (2-(((2-(2,6-dioxopiperidin-3-yl)-1-oxoisoindolin-4-yl)amino)methyl)thiazol-4-yl)methyl 4-oxoadamantane-1- carboxylate | (2-(((2-(2,6-dioxopiperidin-3-yl)-1-oxoisoindolin-4-yl)amino)methyl)thiazol-4-yl)methyl 4-oxoadamantane-1-carboxylate |
| | 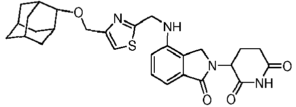 | 3-(4-(((4-((adamantan-2-yloxy)methyl)thiazol-2-yl)methyl)amino)-1 - oxoisoindolin-2-yl)piperidine-2,6-dione | 3-(4-(((4-((adamantan-2-yloxy)methyl)thiazol-2-yl)methyl)amino)-1 - oxoisoindolin-2-yl)piperidine-2,6-dione |
| | 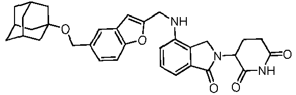 | 3-(4-(((5-((adamantan-1-yloxy)methyl)benzofuran-2-yl)methyl)amino)-1 - oxoisoindolin-2-yl)piperidine-2,6-dione | 3-(4-(((5-((adamantan-1-yloxy)methyl)benzofura n-2-yl)methyl)amino)-1-oxoisoindolin-2-yl)piperidine-2,6-dione |
| | 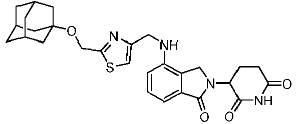 | 3-(4-(((2-((adamantan-1-yloxy)methyl)thiazol-4-yl)methyl)amino)-1-oxoisoindolin-2-yl)piperidine-2,6-dione | 3-(4-(((2-((adamantan-1-yloxy)methyl)thiazol-4-yl)methyl)amino)-1 - oxoisoindolin-2-yl)piperidine-2,6-dione |
| | 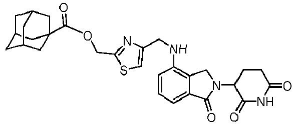 | (4-(((2-(2,6-dioxopiperidin-3-yl)-1 - oxoisoindolin-4-yl)amino)methyl)thiazol-2-yl)methyl adamantane-1-carboxylate | (4-(((2-(2,6-dioxopiperidin-3-yl)-1-oxoisoindolin-4-yl)amino)methyl)thiazol-2-yl)methyl adamantane-1-carboxylate |
| | 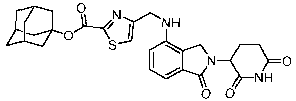 | adamantan-1-yl 4-(((2-(2,6-dioxopiperidin-3-yl)-1-oxoisoindolin-4-yl)amino)methyl)thiazole-2-carboxylate | adamantan-1-yl 4-(((2-(2,6-dioxopiperidin-3-yl)-1-oxoisoindolin-4-yl)amino)methyl)thiazol e-2-carboxylate |
| | 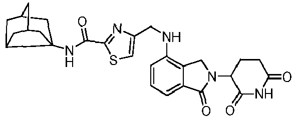 | 4-(((2-(2,6-dioxopiperidin-3-yl)-1-oxoisoindolin-4-yl)amino)methyl)-N-(octahydro-2,5-methanopentalen-6a-yl)thiazole-2-carboxamide | 4-(((2-(2,6-dioxopiperidin-3-yl)-1-oxoisoindolin-4-yl)amino)methyl)-N-(octahydro-2,5-methanopentalen-6a-yl)thiazole-2-carboxamide |
| SIAIS355 067 | 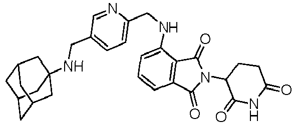 | 4-(((5-((adamantan-1-ylamino)methyl)pyridin-2-yl)methyl)amino)-2-(2,6-dioxopiperidin-3-yl)isoindoline-1,3-dione | 4-(((5-((adamantan-1 - ylamino)methyl)pyridin-2-yl)methyl)amino)-2-(2,6-dioxopiperidin-3-yl)isoindoline-1,3-dione |
| | 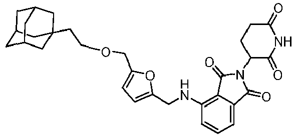 | 4-(((5-((2-(adamantan-1-yl)ethoxy)methyl)furan-2-yl)methyl)amino)-2-(2,6-dioxopiperidin-3-yl)isoindoline-1,3-dione | 4-(((5-((2-(adamantan-1-yl)ethoxy)methyl)furan-2-yl)methyl)amino)-2-(2,6-dioxopiperidin-3-yl)isoindoline-1,3-dione |
| | 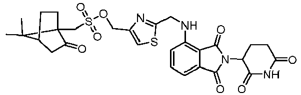 | (2-(((2-(2,6-dioxopiperidin-3-yl)-1,3-dioxoisoindolin-4-yl)amino)methyl)thiazol-4-yl)methyl (7,7-dimethyl-2-oxobicyclo[2.2.1]heptan-1-yl)methanesulfonate | (2-(((2-(2,6-dioxopiperidin-3-yl)-1,3-dioxoisoindolin-4-yl)amino)methyl)thiazol-4-yl)methyl (7,7-dimethyl-2-oxobicyclo[2.2.1]heptan-1-yl)methanesulfonate |
| | 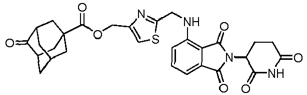 | (2-(((2-(2,6-dioxopiperidin-3-yl)-1,3-dioxoisoindolin-4-yl)amino)methyl)thiazol-4-yl)methyl 4-oxoadamantane-1 - carboxylate | (2-(((2-(2,6-dioxopiperidin-3-yl)-1,3-dioxoisoindolin-4-yl)amino)methyl)thiazol-4-yl)methyl 4-oxoadamantane-1 - carboxylate |
| | 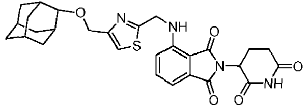 | 4-(((4-((adamantan-2-yloxy)methyl)thiazol-2-yl)methyl)amino)-2-(2,6-dioxopiperidin-3-yl)isoindoline-1,3-dione | 4-(((4-((adamantan-2-yloxy)methyl)thiazol-2-yl)methyl)amino)-2-(2,6-dioxopiperidin-3-yl)isoindoline-1,3-dione |
| | 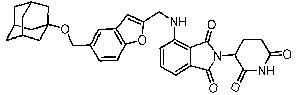 | 4-(((5-((adamantan-1-yloxy)methyl)benzofuran-2-yl)methyl)amino)-2-(2,6-dioxopiperidin-3-yl)isoindoline-1,3-dione | 4-(((5-((adamantan-1-yloxy)methyl)benzofura n-2-yl)methyl)amino)-2-(2,6-dioxopiperidin-3-yl)isoindoline-1,3-dione |
| | 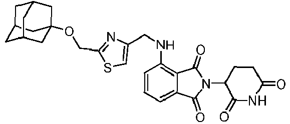 | 4-(((2-((adamantan-1-yloxy)methyl)thiazol-4-yl)methyl)amino)-2-(2,6-dioxopiperidin-3-yl)isoindoline-1,3-dione | 4-(((2-((adamantan-1-yloxy)methyl)thiazol-4-yl)methyl)amino)-2-(2,6-dioxopiperidin-3-yl)isoindoline-1,3-dione |
| | 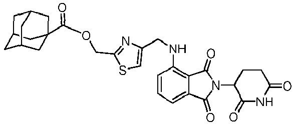 | (4-(((2-(2,6-dioxopiperidin-3-yl)-1,3-dioxoisoindolin-4-yl)amino)methyl)thiazol-2-yl)methyl adamantane-1-carboxylate | (4-(((2-(2,6-dioxopiperidin-3-yl)-1,3-dioxoisoindolin-4-yl)amino)methyl)thiazol-2-yl)methyl adamantane-1-carboxylate |
| | 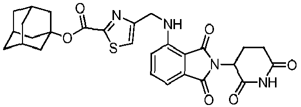 | adamantan-1-yl 4-(((2-(2,6-dioxopiperidin-3-yl)-1,3-dioxoisoindolin-4-yl)amino)methyl)thiazole-2-carboxylate | adamantan-1-yl 4-(((2-(2,6-dioxopiperidin-3-yl)-1,3-dioxoisoindolin-4-yl)amino)methyl)thiazole-2-carboxylate |
| | 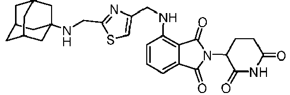 | 4-(((2-(((adamantan-1-yl)amino)methyl)thiazol-4-yl)methyl)amino)-2-(2,6-dioxopiperidin-3-yl)isoindoline-1,3-dione | 4-(((2-(((adamantan-1-yl)amino)methyl)thiazol-4-yl)methyl)amino)-2-(2,6-dioxopiperidin-3-yl)isoindoline-1,3-dione |
| | 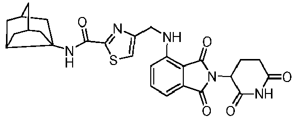 | 4-(((2-(2,6-dioxopiperidin-3-yl)-1,3-dioxoisoindolin-4-yl)amino)methyl)-N-(octahydro-2,5-methanopentalen-6a-yl)thiazole-2-carboxamide | 4-(((2-(2,6-dioxopiperidin-3-yl)-1,3-dioxoisoindolin-4-yl)amino)methyl)-N-(octahydro-2,5-methanopentalen-6a-yl)thiazole-2-carboxamide |
| | 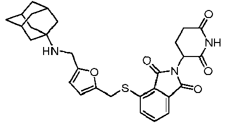 | 4-(((5-((adamantan-1-ylamino)methyl)furan-2-yl)methyl)thio)-2-(2,6-dioxopiperidin-3-yl)isoindoline-1,3-dione | 4-(((5-((adamantan-1-ylamino)methyl)furan-2-yl)methyl)thio)-2-(2,6-dioxopiperidin-3-yl)isoindoline-1,3-dione |
| | 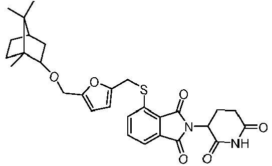 | 2-(2,6-dioxopiperidin-3-yl)-4-(((5-(((1,7,7-trimethylbicyclo[2.2.1]hep tan-2-yl)oxy)methyl)furan-2-yl)methyl)thio)isoindoline -1,3-dione | 2-(2,6-dioxopiperidin-3-yl)-4-(((5-(((1,7,7-trimethylbicyclo[2.2.1]h eptan-2-yl)oxy)methyl)furan-2-yl)methyl)thio)isoindolin e-1,3-dione |
| | 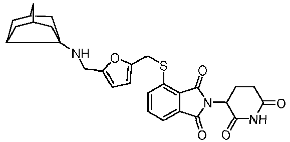 | 2-(2,6-dioxopiperidin-3-yl)-4-(((5-(((hexahydro-2,5-methanopentalen-3a(1H)-yl)amino)methyl)furan-2-yl)methyl)thio)isoindoline -1,3-dione | 2-(2,6-dioxopiperidin-3-yl)-4-(((5-(((hexahydro-2,5-methanopentalen-3a(1H)-yl)amino)methyl)furan-2-yl)methyl)thio)isoindolin e-1,3-dione |
| | 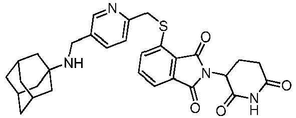 | 4-(((5-((adamantan-1-ylamino)methyl)pyridin-2-yl)methyl)thio)-2-(2,6-dioxopiperidin-3-yl)isoindoline-1,3-dione | 4-(((5-((adamantan-1-ylamino)methyl)pyridin-2-yl)methyl)thio)-2-(2,6-dioxopiperidin-3-yl)isoindoline-1,3-dione |
| | 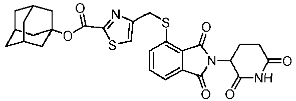 | adamantan-1-yl 4-(((2-(2,6-dioxopiperidin-3-yl)-1,3-dioxoisoindolin-4-yl)thio)methyl)thiazole-2-carboxylate | adamantan-1-yl 4-(((2-(2,6-dioxopiperidin-3-yl)-1,3-dioxoisoindolin-4-yl)thio)methyl)thiazole-2-carboxylate |
| | 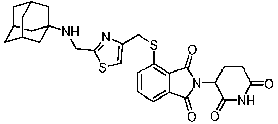 | 4-(((2-((adamantan-1-ylamino)methyl)thiazol-4-yl)methyl)thio)-2-(2,6-dioxopiperidin-3-yl)isoindoline-1,3-dione | 4-(((2-((adamantan-1-ylamino)methyl)thiazol-4-yl)methyl)thio)-2-(2,6-dioxopiperidin-3-yl)isoindoline-1,3-dione |
| | 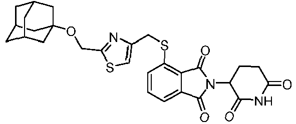 | 4-(((2-(((adamantan-1-yl)oxy)methyl)thiazol-4-yl)methyl)thio)-2-(2,6-dioxopiperidin-3-yl)isoindoline-1,3-dione | 4-(((2-(((adamantan-1-yl)oxy)methyl)thiazol-4-yl)methyl)thio)-2-(2,6-dioxopiperidin-3-yl)isoindoline-1,3-dione |
| | 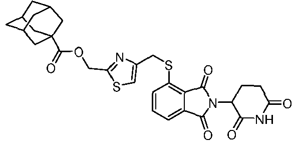 | (4-(((2-(2,6-dioxopiperidin-3-yl)-1,3-dioxoisoindolin-4-yl)thio)methyl)thiazol-2-yl)methyl adamantane-1-carboxylate | (4-(((2-(2,6-dioxopiperidin-3-yl)-1,3-dioxoisoindolin-4-yl)thio)methyl)thiazol-2-yl)methyl adamantane-1-carboxylate |
| | 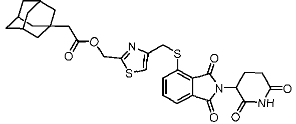 | (4-(((2-(2,6-dioxopiperidin-3-yl)-1,3-dioxoisoindolin-4-yl)thio)methyl)thiazol-2-yl)methyl 2-(adamantan-1-yl)acetate | (4-(((2-(2,6-dioxopiperidin-3-yl)-1,3-dioxoisoindolin-4-yl)thio)methyl)thiazol-2-yl)methyl 2-(adamantan-1-yl)acetate |
| | 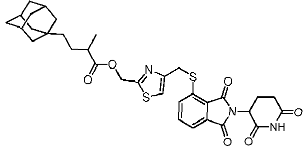 | (4-(((2-(2,6-dioxopiperidin-3-yl)-1,3-dioxoisoindolin-4-yl)thio)methyl)thiazol-2-yl)methyl 4-(adamantan-1-yl)-2-methylbutanoate | (4-(((2-(2,6-dioxopiperidin-3-yl)-1,3-dioxoisoindolin-4-yl)thio)methyl)thiazol-2-yl)methyl 4-(adamantan-1-yl)-2-methylbutanoate |
| | 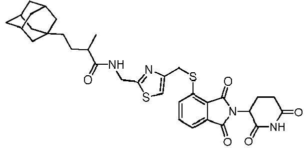 | 4-(adamantan-1-yl)-N-((4-(((2-(2,6-dioxopiperidin-3-yl)-1,3-dioxoisoindolin-4-yl)thio)methyl)thiazol-2-yl)methyl)-2-methylbutanamide | 4-(adamantan-1-yl)-N-((4-(((2-(2,6-dioxopiperidin-3-yl)-1,3-dioxoisoindolin-4-yl)thio)methyl)thiazol-2-yl)methyl)-2-methylbutanamide |
| | 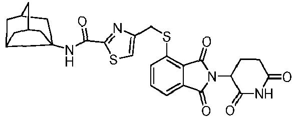 | 4-(((2-(2,6-dioxopiperidin-3-yl)-1,3-dioxoisoindolin-4-yl)thio)methyl)-N-(-octahydro-2,5-methanopentalen-6a-yl)thiazole-2-carboxamide | 4-(((2-(2,6-dioxopiperidin-3-yl)-1,3-dioxoisoindolin-4-yl)thio)methyl)-N-(-octahydro-2,5-methanopentalen-6a-yl)thiazole-2-carboxamide |
| | 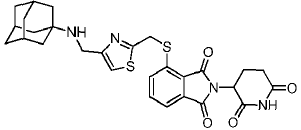 | 4-(((4-((adamantan-1 - ylamino)methyl)thiazol-2-yl)methyl)thio)-2-(2,6-dioxopiperidin-3-yl)isoindoline-1,3-dione | 4-(((4-((adamantan-1 - ylamino)methyl)thiazol-2-yl)methyl)thio)-2-(2,6-dioxopiperidin-3-yl)isoindoline-1,3-dione |
| | 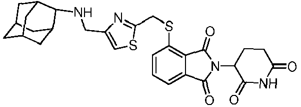 | 4-(((4-((adamantan-2-ylamino)methyl)thiazol-2-yl)methyl)thio)-2-(2,6-dioxopiperidin-3-yl)isoindoline-1,3-dione | 4-(((4-((adamantan-2-ylamino)methyl)thiazol-2-yl)methyl)thio)-2-(2,6-dioxopiperidin-3-yl)isoindoline-1,3-dione |
| | 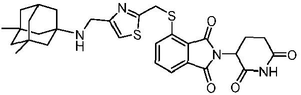 | 4-(((4-(((3,5-dimethyladamantan-1-yl)amino)methyl)thiazol-2-yl)methyl)thio)-2-(2,6-dioxopiperidin-3-yl)isoindoline-1,3-dione | 4-(((4-(((3,5-dimethyladamantan-1-yl)amino)methyl)thiazol-2-yl)methyl)thio)-2-(2,6-dioxopiperidin-3-yl)isoindoline-1,3-dione |
| | 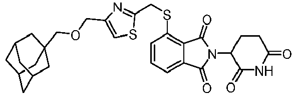 | 4-(((4-(((adamantan-1- yl)methoxy)methyl)thiazol -2-yl)methyl)thio)-2-(2,6-dioxopiperidin-3-yl)isoindoline-1,3-dione | 4-(((4-(((adamantan-1- yl)methoxy)methyl)thiaz ol-2-yl)methyl)thio)-2-(2,6-dioxopiperidin-3-yl)isoindoline-1,3-dione |
| | 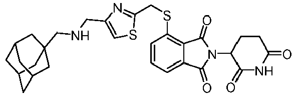 | 4-(((4-((((adamantan-1- yl)methyl)amino)methyl)t hiazol-2-yl)methyl)thio)-2-(2,6-dioxopiperidin-3-yl)isoindoline-1,3 -dione | 4-(((4-((((adamantan-1- yl)methyl)amino)methyl )thiazol-2-yl)methyl)thio)-2-(2,6-dioxopiperidin-3-yl)isoindoline-1,3-dione |
| | 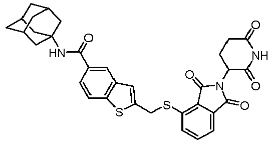 | N-(adamantan-1-yl)-2-(((2-(2,6-dioxopiperidin-3-yl)-1,3-dioxoisoindolin-4-yl)thio)methyl)benzo[b]thi ophene-5-carboxamide | N-(adamantan-1-yl)-2-(((2-(2,6-dioxopiperidin-3-yl)-1,3-dioxoisoindolin-4-yl)thio)methyl)benzo[b]t hiophene-5-carboxamide |
| | 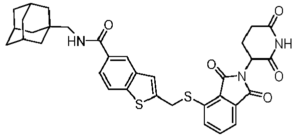 | N-((adamantan-1-yl)methyl)-2-(((2-(2,6-dioxopiperidin-3-yl)-1,3-dioxoisoindolin-4-yl)thio)methyl)benzo[b]thi ophene-5-carboxamide | N-((adamantan-1-yl)methyl)-2-(((2-(2,6-dioxopiperidin-3-yl)-1,3-dioxoisoindolin-4-yl)thio)methyl)benzo[b]t hiophene-5-carboxamide |
| | 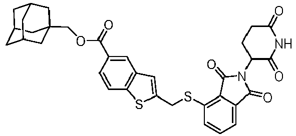 | (adamantan-1-yl)methyl2-(((2-(2,6-dioxopiperidin-3-yl)-1,3-dioxoisoindolin-4-yl)thio)methyl)benzo[b]thi ophene-5-carboxylate | (adamantan-1-yl)methyl2-(((2-(2,6-dioxopiperidin-3-yl)-1,3-dioxoisoindolin-4-yl)thio)methyl)benzo[b]t hiophene-5-carboxylate |
| | 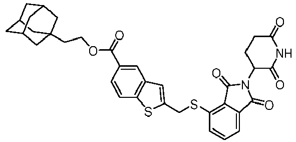 | 2-(adamantan-1-yl)ethyl 2-(((2-(2,6-dioxopiperidin-3-yl)-1,3-dioxoisoindolin-4-yl)thio)methyl)benzo[b]thi ophene-5-carboxylate | 2-(adamantan-1-yl)ethyl 2-(((2-(2,6-dioxopiperidin-3-yl)-1,3-dioxoisoindolin-4-yl)thio)methyl)benzo[b]t hiophene-5-carboxylate |
| | 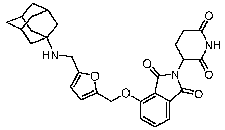 | 4-((5-((adamantan-1-ylamino)methyl)furan-2-yl)methoxy)-2-(2,6-dioxopiperidin-3-yl)isoindoline-1,3-dione | 4-((5-((adamantan-1-ylamino)methyl)furan-2-yl)methoxy)-2-(2,6-dioxopiperidin-3-yl)isoindoline-1,3-dione |
| | 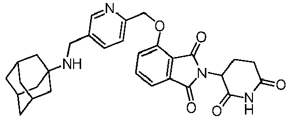 | 4-((5-((adamantan-1-ylamino)methyl)pyridin-2-yl)methoxy)-2-(2,6-dioxopiperidin-3-yl)isoindoline-1,3-dione | 4-((5-((adamantan-1-ylamino)methyl)pyridin-2-yl)methoxy)-2-(2,6-dioxopiperidin-3-yl)isoindoline-1,3-dione |
| | 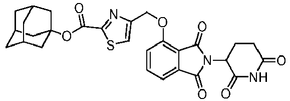 | adamantan-1-yl 4-(((2-(2,6-dioxopiperidin-3-yl)-1,3-dioxoisoindolin-4-yl)oxy)methyl)thiazole-2-carboxylate | adamantan-1-yl 4-(((2-(2,6-dioxopiperidin-3-yl)-1,3-dioxoisoindolin-4-yl)oxy)methyl)thiazole-2-carboxylate |
| | 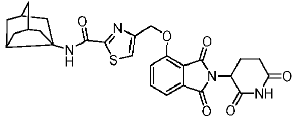 | 4-(((2-(2,6-dioxopiperidin-3-yl)-1,3-dioxoisoindolin-4-yl)oxy)methyl)-N-(octahydro-2,5-methanopentalen-6a-yl)thiazole-2-carboxamide | 4-(((2-(2,6-dioxopiperidin-3-yl)-1,3-dioxoisoindolin-4-yl)oxy)methyl)-N-(octahydro-2,5-methanopentalen-6a-yl)thiazole-2-carboxamide |
| | 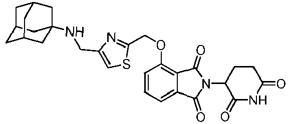 | 4-((4-((adamantan-1-ylamino)methyl)thiazol-2-yl)methoxy)-2-(2,6-dioxopiperidin-3-yl)isoindoline-1,3-dione | 4-((4-((adamantan-1-ylamino)methyl)thiazol-2-yl)methoxy)-2-(2,6-dioxopiperidin-3-yl)isoindoline-1,3-dione |
| | 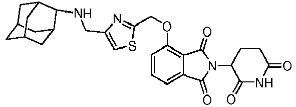 | 4-((4-((adamantan-2-ylamino)methyl)thiazol-2-yl)methoxy)-2-(2,6-dioxopiperidin-3-yl)isoindoline-1,3-dione | 4-((4-((adamantan-2-ylamino)methyl)thiazol-2-yl)methoxy)-2-(2,6-dioxopiperidin-3-yl)isoindoline-1,3-dione |
| | 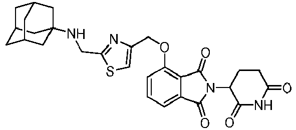 | 4-((2-((adamantan-1-ylamino)methyl)thiazol-4-yl)methoxy)-2-(2,6-dioxopiperidin-3-yl)isoindoline-1,3-dione | 4-((2-((adamantan-1-ylamino)methyl)thiazol-4-yl)methoxy)-2-(2,6-dioxopiperidin-3-yl)isoindoline-1,3-dione |
| | 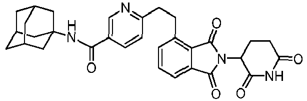 | N-(adamantan-1-yl)-6-(2-(2-(2,6-dioxopiperidin-3-yl)-1,3-dioxoisoindolin-4-yl)ethyl)nicotinamide | N-(adamantan-1-yl)-6-(2-(2-(2,6-dioxopiperidin-3-yl)-1,3-dioxoisoindolin-4-yl)ethyl)nicotinamide |
| | 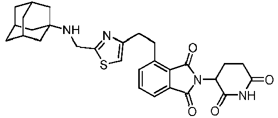 | 4-(2-(2-((adamantan-1-ylamino)methyl)thiazol-4-yl)ethyl)-2-(2,6-dioxopiperidin-3-yl)isoindoline-1,3-dione | 4-(2-(2-((adamantan-1-ylamino)methyl)thiazol-4-yl)ethyl)-2-(2,6-dioxopiperidin-3-yl)isoindoline-1,3-dione |
| | 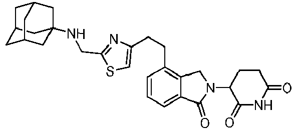 | 3-(4-(2-(2-((adamantan-1-ylamino)methyl)thiazol-4-yl)ethyl)-1-oxoisoindolin-2-yl)piperidine-2,6-dione | 3-(4-(2-(2-((adamantan-1-ylamino)methyl)thiazol-4-yl)ethyl)-1-oxoisoindolin-2-yl)piperidine-2,6-dione |
| | 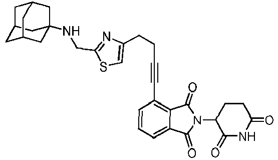 | 4-(4-(2-((adamantan-1-ylamino)methyl)thiazol-4-yl)but-1-yn-1-yl)-2-(2,6-dioxopiperidin-3-yl)isoindoline-1,3 -dione | 4-(4-(2-((adamantan-1-ylamino)methyl)thiazol-4-yl)but-1-yn-1-yl)-2-(2,6-dioxopiperidin-3-yl)isoindoline-1,3-dione |
| | 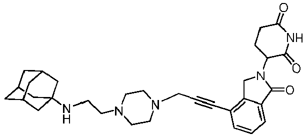 | 3-(4-(3-(4-(2-(adamantan-1-ylamino)ethyl)piperazin-1-yl)prop-1-yn-1-yl)-1- oxoisoindolin-2-yl)piperidine-2,6-dione | 3-(4-(3-(4-(2-(adamantan-1-ylamino)ethyl)piperazin-1-yl)prop-1-yn-1-yl)-1-oxoisoindolin-2-yl)piperidine-2,6-dione |
| | 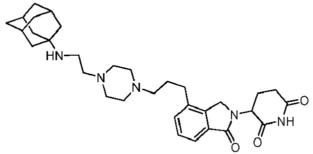 | 3-(4-(3-(4-(2-(adamantan-1 -ylamino)ethyl)piperazin-1-yl)propyl)-1-oxoisoindolin-2-yl)piperidine-2,6-dione | 3-(4-(3-(4-(2-(adamantan-1-ylamino)ethyl)piperazin-1-yl)propyl)-1-oxoisoindolin-2-yl)piperidine-2,6-dione |
| | 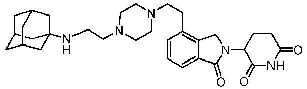 | 3-(4-(2-(4-(2-(adamantan-1-ylamino)ethyl)piperazin-1-yl)ethyl)-1-oxoisoindolin-2-yl)piperidine-2,6-dione | 3-(4-(2-(4-(2-(adamantan-1-ylamino)ethyl)piperazin-1-yl)ethyl)-1-oxoisoindolin-2-yl)piperidine-2,6-dione |
| | 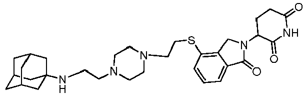 | 3-(4-((2-(4-(2-(adamantan-1-ylamino)ethyl)piperazin-1-yl)ethyl)thio)-1-oxoisoindolin-2-yl)piperidine-2,6-dione | 3-(4-((2-(4-(2-(adamantan-1-ylamino)ethyl)piperazin-1-yl)ethyl)thio)-1-oxoisoindolin-2-yl)piperidine-2,6-dione |
| | 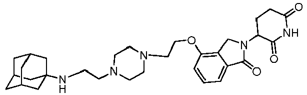 | 3-(4-(2-(4-(2-(adamantan-1-ylamino)ethyl)piperazin-1-yl)ethoxy)-1-oxoisoindolin-2-yl)piperidine-2,6-dione | 3-(4-(2-(4-(2-(adamantan-1-ylamino)ethyl)piperazin-1-yl)ethoxy)-1-oxoisoindolin-2-yl)piperidine-2,6-dione |
| | 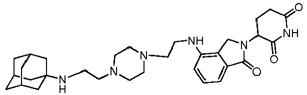 | 3-(4-((2-(4-(2-(adamantan-1-ylamino)ethyl)piperazin-1-yl)ethyl)amino)-1-oxoisoindolin-2-yl)piperidine-2,6-dione | 3-(4-((2-(4-(2-(adamantan-1-ylamino)ethyl)piperazin-1-yl)ethyl)amino)-1-oxoisoindolin-2-yl)piperidine-2,6-dione |
| | 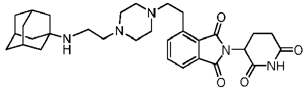 | 4-(2-(4-(2-(adamantan-1-ylamino)ethyl)piperazin-1- yl)ethyl)-2-(2,6-dioxopiperidin-3-yl)isoindoline-1,3-dione | 4-(2-(4-(2-(adamantan-1-ylamino)ethyl)piperazin-1-yl)ethyl)-2-(2,6-dioxopiperidin-3-yl)isoindoline-1,3-dione |
| | 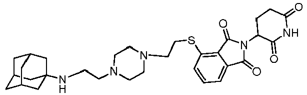 | 4-((2-(4-(2-(adamantan-1-ylamino)ethyl)piperazin-1- yl)ethyl)thio)-2-(2,6-dioxopiperidin-3-yl)isoindoline-1,3-dione | 4-((2-(4-(2-(adamantan-1-ylamino)ethyl)piperazin-1-yl)ethyl)thio)-2-(2,6-dioxopiperidin-3-yl)isoindoline-1,3-dione |
| | 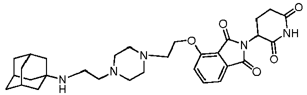 | 4-(2-(4-(2-(adamantan-1-ylamino)ethyl)piperazin-1 - yl)ethoxy)-2-(2,6-dioxopiperidin-3-yl)isoindoline-1,3-dione | 4-(2-(4-(2-(adamantan-1-ylamino)ethyl)piperazin-1-yl)ethoxy)-2-(2,6-dioxopiperidin-3-yl)isoindoline-1,3-dione |
| | 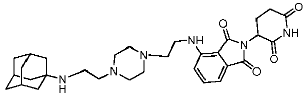 | 4-((2-(4-(2-(adamantan-1-ylamino)ethyl)piperazin-1- yl)ethyl)amino)-2-(2,6-dioxopiperidin-3-yl)isoindoline-1,3-dione | 4-((2-(4-(2-(adamantan-1-ylamino)ethyl)piperazin-1-yl)ethyl)amino)-2-(2,6-dioxopiperidin-3-yl)isoindoline-1,3-dione |
| | 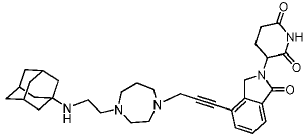 | 3-(4-(3-(4-(2-(adamantan-1-ylamino)ethyl)-1,4-diazepan-1-yl)prop-1 -yn-1-yl)-1-oxoisoindolin-2-yl)piperidine-2,6-dione | 3-(4-(3-(4-(2-(adamantan-1-ylamino)ethyl)-1,4-diazepan-1-yl)prop-1-yn-1-yl)-1-oxoisoindolin-2-yl)piperidine-2,6-dione |
| | 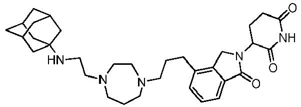 | 3-(4-(3-(4-(2-(adamantan-1-ylamino)ethyl)-1,4-diazepan-1-yl)propyl)-1- oxoisoindolin-2-yl)piperidine-2,6-dione | 3-(4-(3-(4-(2-(adamantan-1-ylamino)ethyl)-1,4-diazepan-1-yl)propyl)-1- oxoisoindolin-2-yl)piperidine-2,6-dione |
| | 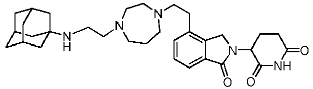 | 3-(4-(2-(4-(2-(adamantan-1-ylamino)ethyl)-1,4-diazepan-1-yl)ethyl)-1-oxoisoindolin-2-yl)piperidine-2,6-dione | 3-(4-(2-(4-(2-(adamantan-1-ylamino)ethyl)-1,4-diazepan-1-yl)ethyl)-1-oxoisoindolin-2-yl)piperidine-2,6-dione |
| | 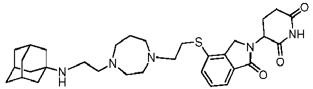 | 3-(4-((2-(4-(2-(adamantan-1-ylamino)ethyl)-1,4-diazepan-1-yl)ethyl)thio)-1 -oxoisoindolin-2-yl)piperidine-2,6-dione | 3-(4-((2-(4-(2-(adamantan-1-ylamino)ethyl)-1,4-diazepan-1-yl)ethyl)thio)-1-oxoisoindolin-2-yl)piperidine-2,6-dione |
| | 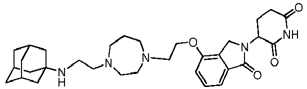 | 3-(4-(2-(4-(2-(adamantan-1-ylamino)ethyl)-1,4-diazepan-1-yl)ethoxy)-1-oxoisoindolin-2-yl)piperidine-2,6-dione | 3-(4-(2-(4-(2-(adamantan-1-ylamino)ethyl)-1,4-diazepan-1-yl)ethoxy)-1-oxoisoindolin-2-yl)piperidine-2,6-dione |

It is to be understood that the compounds of Formula (I), (Ia), (Ib), (Ic), (Id), (Ie) of the present invention may have a stereo configuration and thus can be in more than one stereoisomeric form. The present invention also relates to compounds having a stereo configuration in substantially pure isomeric form, e.g., greater than about 90% enantiomeric/diastereomeric excess ("ee"), such as about 95% ee or 97% ee, or greater than 99% ee, and mixtures thereof, including racemic mixtures. These isomers can be prepared by using asymmetric synthesis (e.g., by using chiral intermediates) or by chiral resolution.

In another aspect, the present invention also provides a pharmaceutical composition comprising, as an active ingredient, the compound of Formula (I) of the present invention or pharmaceutically acceptable salts, racemates, enantiomers, stereoisomers, solvates, or polymorphs thereof, and a pharmaceutically acceptable carrier.

The pharmaceutical composition of the present invention further comprises at least one additional therapeutic agent for the treatment or prevention of a disease or disorder associated with TNF-α. The additional therapeutic agent can be combined with the compound of Formula (I) of the present invention to treat diseases or disorders associated with TNF-α, including but not limited to chemotherapeutic agents, immunotherapy agents, gene therapy agents and the like. In one embodiment, the diseases or disorders associated with TNF-α is selected from the group consisting of: tumors (including cancer), infectious diseases, inflammatory diseases, autoimmune diseases, anemia, hemorrhagic shock, transplant rejection, multiple organ dysfunction syndrome (MODS), sarcoidosis, adult respiratory distress syndrome, congestive heart failure, myocardial infarction, acute liver failure, or diabetes. The diseases or disorders associated with TNF-α include, but are not limited to:
tumors (including cancers), including multiple myeloma, myelodysplastic syndrome (MDS), previously treated myelodysplastic syndrome, plasma cell myeloma, transplantation-related cancer, myelofibrosis, plasma cell myeloma, smoldering myeloma, smoldering multiple myeloma; bone marrow disease; neutropenia; leukemia, including acute myeloid leukemia, chronic myelogenous leukemia, B-cell chronic lymphocytic leukemia, acute myeloid leukemia (AML); lymphoma, including lymphoma CD20 positive, mantle cell lymphoma, primary lymphoma, B-cell lymphoma, recurrent B-cell non-Hodgkin's lymphoma, recurrent diffuse large B-cell lymphoma, recurrent primary mediastinal (thymus) large B-cell, relapsed transformed non-Hodgkin's lymphoma, refractory B-cell non-Hodgkin's lymphoma, refractory diffuse large B-cell lymphoma, refractory primary mediastinal (thymus) large B-cell, refractory transformed non-Hodgkin's lymphoma; thyroid cancer; melanoma; and lung cancer;
autoimmune diseases, including, for example, Unverricht Syndrome; sepsis syndrome; Rheumatoid arthritis; autoimmune encephalomyelitis; ankylosing spondylitis; psoriasis; systemic lupus erythematosus; recurrent oral ulcer; multiple sclerosis; inflammatory bowel disease including Crohn's disease and ulcerative colitis; Kawasaki disease;
infectious diseases, including, for example, COVID-19 novel coronavirus infection, septic shock, bacterial meningitis, cerebral malaria, AIDS, tuberculosis;
inflammatory diseases, including, for example, pneumonia, osteoarthritis, synovitis, systemic inflammatory response syndrome, airway inflammation, bronchitis, chronic obstructive pulmonary disease, asthma;
multiple organ dysfunction syndrome, including, for example, multiple organ failure due to cachexia and septic shock; and
anemia; hemorrhagic shock; organ (including kidney, heart, lung) or tissue transplant rejection; diabetes; sarcoidosis; adult respiratory distress syndrome; congestive heart failure; myocardial infarction; and acute liver failure.

The pharmaceutical composition of the present invention comprising, as an active ingredient, the compound of Formula (I) of the present invention or a pharmaceutically acceptable salt thereof can be formulated into any suitable formulations such as sprays, patches, tablets (such as conventional tablets, dispersible tablets, oral disintegrating tablets), capsules (such as soft capsules, hard capsules, enteric-coated capsules), dragees, troches, powders, granules, powder injections, or liquid formulations (such as suspensions (e.g., aqueous or oily suspensions), solutions, emulsions, or syrups), or conventional injectable dosage forms such as injectable solutions (e.g., sterile injectable solutions formulated as a vehicle or solvent according to methods known in the art using water, Ringer's solution, or isotonic sodium chloride solution or the like) or lyophilized compositions and the like, depending upon a suitable route of administration (including, but not limited to, nasal administration, inhalation administration, topical administration, oral administration, oral mucosal administration, rectal administration, intrapleural administration, intraperitoneal administration, vaginal administration, intramuscular administration, subcutaneous administration, transdermal administration, epidural administration, intrathecal administration, and intravenous administration. The compounds of Formula (I) can also be formulated into conventional, dispersible, chewable, oral disintegrating, or rapidly dissolving formulations as required by those skilled in the art.

In another aspect, the present invention provides the compound of Formula (I), or pharmaceutically acceptable salts, racemates, enantiomers, stereoisomers, solvates, or polymorphs thereof for use as a medicament.

In another aspect, the present invention provides the compound of Formula (I), or pharmaceutically acceptable salts, racemates, enantiomers, stereoisomers, solvates, or polymorphs thereof for use in the prevention and/or treatment of diseases or disorders associated with TNF-α. In one embodiment, the diseases or disorders associated with TNF-α are selected from the group consisting of tumors (including cancer), infectious diseases, inflammatory diseases, autoimmune diseases, anemia, hemorrhagic shock, transplant rejection, multiple organ dysfunction syndrome (MODS), sarcoidosis, adult respiratory distress syndrome, congestive heart failure, myocardial infarction, acute liver failure, or diabetes. In one embodiment, the diseases or disorders associated with TNF-α include, but are not limited to, tumors (including cancers), including multiple myeloma, myelodysplastic syndrome (MDS), previously treated myelodysplastic syndrome, plasma cell myeloma, transplantation-related cancer, myelofibrosis, plasma cell myeloma, smoldering myeloma, smoldering multiple myeloma; bone marrow disease; neutropenia; leukemia, including acute myeloid leukemia, chronic myelogenous leukemia, B-cell chronic lymphocytic leukemia, acute myeloid leukemia (AML); lymphoma, including lymphoma CD20 positive, mantle cell lymphoma, primary lymphoma, B-cell lymphoma, recurrent B-cell non-Hodgkin's lymphoma, recurrent diffuse large B-cell lymphoma, recurrent primary mediastinal (thymus) large B-cell, relapsed transformed non-Hodgkin's lymphoma, refractory B-cell non-Hodgkin's lymphoma, refractory diffuse large B-cell lymphoma, refractory primary mediastinal (thymus) large B-cell, refractory transformed non-Hodgkin's lymphoma; thyroid cancer; melanoma; and lung cancer; autoimmune diseases, including, for example, Unverricht Syndrome; sepsis syndrome; Rheumatoid arthritis; autoimmune encephalomyelitis; ankylosing spondylitis; psoriasis; systemic lupus erythematosus; recurrent oral ulcer; multiple sclerosis; inflammatory bowel disease including Crohn's disease and ulcerative colitis; Kawasaki disease; infectious diseases, including, for example, COVID-19 novel coronavirus infection, septic shock, bacterial meningitis, cerebral malaria, AIDS, tuberculosis; inflammatory diseases, including, for example, pneumonia, osteoarthritis, synovitis, systemic inflammatory response syndrome, airway inflammation, bronchitis, chronic obstructive pulmonary disease, asthma; multiple organ dysfunction syndrome, including, for example, multiple organ failure due to cachexia and septic shock; anemia; hemorrhagic shock; organ (including kidney, heart, lung) or tissue transplant rejection; diabetes; sarcoidosis; adult respiratory distress syndrome; congestive heart failure; myocardial infarction; and acute liver failure.

In another aspect, the present invention provides the use of the compound of Formula (I) or pharmaceutically acceptable salts, racemates, enantiomers, stereoisomers, solvates, or polymorphs thereof, for the manufacture of a medicament for the prevention and/or treatment of diseases or disorders associated with TNF-α. In one embodiment, the diseases or disorders associated with TNF-α are selected from the group consisting of tumors (including cancer), infectious diseases, inflammatory diseases, autoimmune diseases, anemia, hemorrhagic shock, transplant rejection, multiple organ dysfunction syndrome (MODS), sarcoidosis, adult respiratory distress syndrome, congestive heart failure, myocardial infarction, acute liver failure, or diabetes. In one embodiment, the diseases or disorders associated with TNF-α include, but are not limited to, tumors (including cancers), including multiple myeloma, myelodysplastic syndrome (MDS), previously treated myelodysplastic syndrome, plasma cell myeloma, transplantation-related cancer, myelofibrosis, plasma cell myeloma, smoldering myeloma, smoldering multiple myeloma; bone marrow disease; neutropenia; leukemia, including acute myeloid leukemia, chronic myelogenous leukemia, B-cell chronic lymphocytic leukemia, acute myeloid leukemia (AML); lymphoma, including lymphoma CD20 positive, mantle cell lymphoma, primary lymphoma, B-cell lymphoma, recurrent B-cell non-Hodgkin's lymphoma, recurrent diffuse large B-cell lymphoma, recurrent primary mediastinal (thymus) large B-cell, relapsed transformed non-Hodgkin's lymphoma, refractory B-cell non-Hodgkin's lymphoma, refractory diffuse large B-cell lymphoma, refractory primary mediastinal (thymus) large B-cell, refractory transformed non-Hodgkin's lymphoma; thyroid cancer; melanoma; and lung cancer; autoimmune diseases, including, for example, Unverricht Syndrome; sepsis syndrome; Rheumatoid arthritis; autoimmune encephalomyelitis; ankylosing spondylitis; psoriasis; systemic lupus erythematosus; recurrent oral ulcer; multiple sclerosis; inflammatory bowel disease including Crohn's disease and ulcerative colitis; Kawasaki disease; infectious diseases, including, for example, COVID-19 novel coronavirus infection, septic shock, bacterial meningitis, cerebral malaria, AIDS, tuberculosis; inflammatory diseases, including, for example, pneumonia, osteoarthritis, synovitis, systemic inflammatory response syndrome, airway inflammation, bronchitis, chronic obstructive pulmonary disease, asthma; multiple organ dysfunction syndrome, including, for example, multiple organ failure due to cachexia and septic shock; anemia; hemorrhagic shock; organ (including kidney, heart, lung) or tissue transplant rejection; diabetes; sarcoidosis; adult respiratory distress syndrome; congestive heart failure; myocardial infarction; and acute liver failure.

In another aspect, the present invention also provides a method for treating or preventing diseases or disorders associated with TNF-α, comprising administering to a subject a therapeutically effective amount of the compound of Formula (I) of the present invention or a pharmaceutically acceptable salt, racemate, enantiomer, stereoisomer, solvate, or polymorph thereof, or the pharmaceutical composition of the present invention. In one embodiment, the diseases or disorders associated with TNF-α are selected from the group consisting of tumors (including cancer), infectious diseases, inflammatory diseases, autoimmune diseases, anemia, hemorrhagic shock, transplant rejection, multiple organ dysfunction syndrome (MODS), sarcoidosis, adult respiratory distress syndrome, congestive heart failure, myocardial infarction, acute liver failure, or diabetes. In one embodiment, the diseases or disorders associated with TNF-α include, but are not limited to, tumors (including cancers), including multiple myeloma, myelodysplastic syndrome (MDS), previously treated myelodysplastic syndrome, plasma cell myeloma, transplantation-related cancer, myelofibrosis, plasma cell myeloma, smoldering myeloma, smoldering multiple myeloma; bone marrow disease; neutropenia; leukemia, including acute myeloid leukemia, chronic myelogenous leukemia, B-cell chronic lymphocytic leukemia, acute myeloid leukemia (AML); lymphoma, including lymphoma CD20 positive, mantle cell lymphoma, primary lymphoma, B-cell lymphoma, recurrent B-cell non-Hodgkin's lymphoma, recurrent diffuse large B-cell lymphoma, recurrent primary mediastinal (thymus) large B-cell, relapsed transformed non-Hodgkin's lymphoma, refractory B-cell non-Hodgkin's lymphoma, refractory diffuse large B-cell lymphoma, refractory primary mediastinal (thymus) large B-cell, refractory transformed non-Hodgkin's lymphoma; thyroid cancer; melanoma; and lung cancer; autoimmune diseases, including, for example, Unverricht Syndrome; sepsis syndrome; Rheumatoid arthritis; autoimmune encephalomyelitis; ankylosing spondylitis; psoriasis; systemic lupus erythematosus; recurrent oral ulcer; multiple sclerosis; inflammatory bowel disease including Crohn's disease and ulcerative colitis; Kawasaki disease; infectious diseases, including, for example, COVID-19 novel coronavirus infection, septic shock, bacterial meningitis, cerebral malaria, AIDS, tuberculosis; inflammatory diseases, including, for example, pneumonia, osteoarthritis, synovitis, systemic inflammatory response syndrome, airway inflammation, bronchitis, chronic obstructive pulmonary disease, asthma; multiple organ dysfunction syndrome, including, for example, multiple organ failure due to cachexia and septic shock; anemia; hemorrhagic shock; organ (including kidney, heart, lung) or tissue transplant rejection; diabetes; sarcoidosis; adult respiratory distress syndrome; congestive heart failure; myocardial infarction; and acute liver failure.

The method for treating or preventing diseases or disorders associated with TNF-α of the present invention comprises that: the compound of Formula (I) of the present invention, or a pharmaceutically acceptable salt, racemate, enantiomer, stereoisomer, solvate, or polymorph thereof, or said pharmaceutical composition is administered to the subject by at least one mode of administration selected from the group consisting of: nasal, inhalation, topical, oral, oral mucosal, rectal, pleural, peritoneal, vaginal, intramuscular, subcutaneous, transdermal, epidural, intrathecal, and intravenous administration.

### Definition

Unless otherwise specified, the following words, phrases and symbols used herein generally have the meanings as described below.

In general, the nomenclature used herein (such as the IUPAC nomenclature) and the laboratory procedures described below (including those used in cell culture, organic chemistry, analytical chemistry, and pharmacology, etc.) are those well known and commonly used in the art. Unless otherwise defined, all scientific and technical terms used herein in connection with the present disclosure described herein have the same meaning as commonly understood by one skill in the art. In addition, the use of the word "a" or "an" when used in conjunction with the term "comprising" or nouns in the claims and/or the specification may mean "one", but it is also consistent with the meaning of "one or more", "at least one", and "one or more than one". Similarly, the terms "another" or "other" can mean at least a second or more.

It is to be understood that whenever the terms "comprising" or "containing" are used herein to describe various aspects, other similar aspects recited by "consisting of" and/or "consisting essentially of" are also provided.

The term "about" used herein refers to approximately, roughly, nearly, or around. When the term "about" is used in conjunction with a numerical range, it modifies that range by extending the boundaries above and below the stated numerical value. In general, the term "about" can modify a numerical value above and below the stated value by an upward or downward (increasing or decreasing) variation, e.g., 10%, 5%, 2%, or 1%.

The wording "...represents a bond" used herein means that it is a chemical bond linker (i.e., that it is absent). For example, the term "R represents a bond" means that R is a bond linker. In other words, when R represents a bond, the L₁ of the compound of Formula (I) is directly connected to any one of B, U, V and W in Formula (I).

As used herein, the term "interrupted" of the wording "linear or branched C₂₋₄₀ alkylene is interrupted one or more times by..." used alone or in combination has the definition known in the art, i.e., can mean that there is a group as defined herein (e.g., a group selected from the group consisting of optionally substituted heterocyclylene, optionally substituted heteroarylene, O, S, S(O)₂O, OS(O)₂, S(O)₂N(R³), N(R³)S(O)₂, C(O)O, OC(O), N(R³), C(O)N(R³), N(R³)C(O) or any combination thereof, as defined herein) inserted between any two adjacent carbon atoms in the backbone of the linear or branched C₂₋₄₀ alkylene. Herein, examples of the above phrase "interrupted one or more times" may include, but are not limited to, being interrupted 1-20 times, or 1-15, 1-10, 1-9, 1-8, 1-7, 1-6 , 1-5, 1-4, 1-3 or 1-2 times, or 1 time. For example, the wording "the linear or branched C₂₋₄₀ alkylene is optionally interrupted one or more times by C(O)O" refers to that there is a C(O)O group inserted between any one or more pairs of two adjacent carbon atoms of the main backbone of the linear or branched C₂₋₄₀ alkylene, such that a linear or branched C₂₋₄₀ alkylene containing one or more (e.g., 1-10, 1-8, 1-7, 1-6, 1-5, 1-4, 1-3. 1-2, or 1) fragment "-CH₂-C(O)O-CH₂-" is formed.

Herein, bonds interrupted by wavy lines show the point of attachment of the depicted group to the rest of the molecule. For example, the group represented by X_{b} depicted below 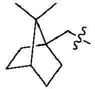
means the methylene in said group is bonded to Xₐ in the group X₁ of the compound of Formula (I). For example, unless otherwise specified, the combined group represented by the L₂-R¹-L₁ moiety in Formula (I) depicted herein 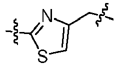
means that either of the two ends of the group (e.g., methylene) can be attached to the group R, the other end is attached to X₁, and vice versa.

As used herein, the singular forms "a", "an", and "the" include plural forms unless the context clearly dictates otherwise.

In some embodiments, the term "one or more" when used in conjunction with "the hydrogen(s) of one or more CH₂ of C₂₋₄₀ alkylene" may refer to 1-80 hydrogen atom(s) of the plurality of hydrogen atoms of C₂₋₄₀ alkylene. In some embodiments, the term "one or more" may refer to 1-30, optionally 1-25, 1-20, 1-15, 1-10, 1-5, 1-4, 1-3, 1-1, or 1 of the plurality of hydrogen atoms of the referenced C₂₋₄₀ alkylene mentioned. In some embodiments, the term "one or more" may refer to 1-3 of the plurality of hydrogen atoms of the referenced alkylene.

As used herein, the term "halogen atom" or "halogen", used alone or in combination, refers to fluorine, chlorine, bromine, or iodine, and optionally F, Cl, or Br.

As used herein, the term "alkyl", used alone or in combination, refers to a linear or branched alkyl group. The term "Cₓ-C_{y} alkyl" or "C_{x-y} alkyl" (x and y each being an integer) refers to a linear or branched alkyl group containing from x to y carbon atoms. The term "C₁₋₁₀ alkyl" used alone or in combination in the present disclosure refers to a linear or branched alkyl group containing from 1 to 10 carbon atoms. The C₁₋₁₀ alkyl of the present disclosure is optionally a C₁₋₉ alkyl, such as C₁₋₈ alkyl, C₂₋₈ alkyl, C₁₋₇ alkyl, C₁₋₆ alkyl, C₁₋₅ alkyl, or C₁₋₄ alkyl. Representative examples include methyl, ethyl, n-propyl, isopropyl, n-butyl, isobutyl, sec-butyl, tert-butyl, pentyl, isopentyl, neopentyl, tert-pentyl, hexyl, heptyl, octyl, nonyl, and decyl. The term "C₁₋₃ alkyl" in the present disclosure refers to an alkyl group containing from 1 to 3 carbon atoms, and representative examples thereof include methyl, ethyl, n-propyl, and isopropyl. In the present invention, the "alkyl" is optionally substituted, and the substituent may be one or more selected from halogen, hydroxyl, cyano, C₁₋₃ alkyl, C₁₋₃ alkoxy, trifluoromethyl, heterocyclyl, or a combination thereof.

As used herein, the term "halogenated alkyl" or "haloalkyl", used alone or in combination, refers to a linear or branched alkyl group substituted with one or more halogens, wherein one or more hydrogen atom(s) of the alkyl group is replaced with one or more halogens. The term "halogenated C_{x-Cy} alkyl" or "halogenated C_{x-y} alkyl" (x and y are each an integer) refers to a linear or branched alkyl containing from x to y carbon atoms substituted with one or more halogens. The term "halogenated C₁₋₁₀ alkyl" used alone or in combination in the present invention refers to a linear or branched alkyl group containing from 1 to 10 carbon atoms substituted with one or more halogens. The halogenated C₁₋₁₀ alkyl group of the present invention is optionally a halogenated C₁₋₉ alkyl group, such as a halogenated C₁₋₈ alkyl group, a halogenated C₂₋₈ alkyl group, a halogenated C₁₋₇ alkyl group, a halogenated C₁₋₆ alkyl, halogenated C₁₋₅ alkyl, or halogenated C₁₋₄ alkyl. Representative examples include halomethyl, haloethyl, halo-n-propyl, haloisopropyl, halo-n-butyl, haloisobutyl, halo-sec-butyl, halo-tert-butyl, halopentyl, haloisoamyl, haloneopentyl, halo-tert-pentyl, halohexyl, haloheptyl, halooctyl, halononyl, and halodecyl. The term "halo-C₁₋₃ alkyl" of the present invention refers to an alkyl group containing from 1 to 3 carbon atoms substituted by one or more halogens, and its representative examples include halomethyl, haloethyl, halo-n-propyl and haloisopropyl.

As used herein, the term "the hydrogen(s) of one or more CH₂ of the linear or branched C_{x-y} alkylene group is replaced by...", used alone or in combination, means that the hydrogen atom(s) of any one or more CH₂ of the linear or branched C_{x-y} alkylene group is replaced with a substituent as defined herein.

As used herein, the term "alkylene" (which is used interchangeably with "alkylene chain"), used alone or in combination, refers to a linear or branched divalent saturated hydrocarbon group composed of carbon and hydrogen atoms. The term "Cₓ-C_{y} alkylene" or "C_{x-y} alkylene" (x and y each being an integer) refers to a linear or branched alkylene group containing from x to y carbon atoms. The C₁-C₄₀ alkylene in the present disclosure can optionally be C₁-C₃₅ alkylene, C₁-C₃₀ alkylene, C₁-C₂₉ alkylene, C₁-C₂₈ alkylene, C₁-C₂₇ alkylene, C₁-C₂₆ alkylene, C₁-C₂₅ alkylene, C₁-C₂₄ alkylene, C₁-C₂₃ alkylene, C₁-C₂₂ alkylene, C₁-C₂₁ alkylene, C₁-C₂₀ alkylene, C₁-C₁₉ alkylene, Ci-Cis alkylene, C₁-C₁₇ alkylene, C₁-C₁₆ alkylene, C₁-C₁₅ alkylene, C₁-C₁₄ alkylene, C₁-C₁₃ alkylene, C₁-C₁₂ alkylene, C₁-C₁₁ alkylene, C₁-C₁₀ alkylene , C₁-C₉ alkylene, Ci-Cs alkylene, C₁-C₇ alkylene, C₁-C₆ alkylene, C₁-C₅ alkylene, C₁-C₄ alkylene, C₁-C₃ alkylene, or C₁-C₂ alkylene. Representative examples include, but are not limited to, methylene, ethylene, propylene, isopropylene, butylene, isobutylene, sec-butylene, tert-butylene, n-pentylene, isopentylene, neopentylidene, tert-pentylene, hexylene, heptylene, octylene, nonylene, decylene, undecylene, dodecylene, tridecylene, tetradecylene, pentadecylene, hexadecylene, heptadecylene, octadecylene, nonadecylene, eicosylene, heneicosylene, docosylene, tricosylene, tetracosylene, pentacosylene, hexacosylene, peptacosylene, octacosylene, nonacosylene, and triacontylene. In the present invention, the "alkylene" is optionally substituted, and the substituent may be one or more selected from C₁₋₃ alkyl, C₁₋₃ alkoxy, C₃₋₆ cycloalkyl, halogen, halogenated C₁₋₃ alkyl, hydroxyl, cyano, or any combination thereof.

As used herein, the term "heteroaryl" used alone or in combination refers to a 5- to 10-membered monocyclic or bicyclic aromatic ring group containing one or more (e.g., from 1 to 6, or from 1 to 5, or from 1 to 4, or from 1 to 3, or from 1 to 2, or 1) heteroatoms independently selected from the group consisting of oxygen, nitrogen, and sulfur. Non-limiting examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2a]pyridyl, 1H-pyrrolo[3,2-b]pyridyl, 1H-pyrrolo[2,3-b]pyridyl, 4H-fluoro[3,2b]pyrrolyl, pyrrolo[2,1-b]thiazolyl and imidazo[2,1-b]thiazolyl. The heteroaryl group may be unsubstituted or substituted as explicitly defined (e.g., mono-, di-, tri-, or poly-substituted), and the substituent(s) of the heteroaryl can be optionally selected from the group consisting of C₁₋₃ alkyl, C₁₋₃ alkoxy, C₃₋₆ cycloalkyl, halogenated C₁₋₃ alkyl, halogen, hydroxyl, cyano, amino, or any combination thereof.

As used herein, the term "heteroarylene" used alone or in combination refers to a 5- to 10-membered monocyclic or bicyclic divalent aromatic ring group containing one or more (e.g., from 1 to 6, or from 1 to 5, or from 1 to 4, or from 1 to 3, or from 1 to 2, or 1) heteroatoms independently selected from the group consisting of oxygen, nitrogen, and sulfur. Non-limiting examples of heteroarylene include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2a]pyridylene, 1H-pyrrolo[3,2-b]pyridylene, 1H-pyrrolo[2,3-b]pyridylene, 4H-fluoro[3,2b]pyrrolylene, pyrrolo[2,1-b]thiazolylene and imidazo[2,1-b]thiazolylene. The heteroarylene group may be unsubstituted or substituted as explicitly defined (e.g., mono-, di-, tri-, or poly-substituted), and the substituent(s) of the heteroarylene can be optionally selected from the group consisting of C₁₋₃ alkyl, C₁₋₃ alkoxy, C₃₋₆ cycloalkyl, halogenated C₁₋₃ alkyl, halogen, hydroxyl, cyano, amino, or any combination thereof.

As used herein, the term "phenyl" used alone or in combination is optionally substituted. A substituted phenyl refers to phenyl optionally substituted with 1 to 3 substituent(s), wherein the substituent(s) is optionally selected from C₁₋₃ alkyl, C₁₋₃ alkoxy, C₃₋₆ cycloalkyl, halogenated C₁₋₃ alkyl, halogen, hydroxyl, cyano, amino, or any combination thereof.

As used herein, the term "phenylene" used alone or in combination is optionally substituted. A substituted phenylene refers to phenyl optionally substituted with 1 to 3 substituent(s), wherein the substituent(s) is optionally selected from C₁₋₃ alkyl, C₁₋₃ alkoxy, C₃₋₆ cycloalkyl, halogenated C₁₋₃ alkyl, halogen, hydroxyl, cyano, amino, or any combination thereof.

As used herein, the term "alkoxy" used alone or in combination refers to a linear or branched alkoxy group having the Formula of -O-alkyl. The alkyl of the alkoxy may optionally contain 1-10 carbon atoms. Representative examples of "alkoxy" include, but are not limited to, methoxy, ethoxy, propoxy, isopropoxy, n-butoxy, isobutoxy, tert-butoxy, pentoxy, 2-pentyloxy, isopentyloxy, neopentyloxy, hexyloxy, 2-hexyloxy, 3-hexyloxy, 3-methylpentyloxy, etc. The term "C₁-C₃ alkoxy" or "C₁₋₃ alkoxy" used alone or in combination refers to a linear or branched alkoxy group containing from 1 to 3 carbon atoms. Representative examples of C₁₋₃ alkoxy include, but are not limited to, methoxy, ethoxy, n-propoxy, and isopropoxy.

As used herein, the term "cycloalkyl", used alone or in combination, refers to a saturated or partially unsaturated (i.e., containing one or more double bonds, but not having a fully conjugated π-electron system) monocyclic or bicyclic or polycyclic cyclic hydrocarbon radical, which in some embodiments has from 3 to 20 carbon atoms (i.e., C₃₋₂₀ cycloalkyl), or from 3 to 15 carbon atoms (i.e., C₃₋₁₅ cycloalkyl), or from 3 to 12 carbon atoms (i.e., C₃₋₁₂ cycloalkyl), or from 3 to 11 carbon atoms (i.e., C₃₋₁₁ cycloalkyl), or from 3 to 10 carbon atoms (i.e., C₃₋₁₀ cycloalkyl), or from 3 to 8 carbon atoms ( i.e., C₃₋₈ cycloalkyl), or from 3 to 7 carbon atoms (i.e., C₃₋₇ cycloalkyl), or from 3 to 6 carbon atoms (i.e., C₃₋₆ cycloalkyl). The term "cycloalkyl" includes monocyclic, bicyclic or tricyclic cyclic hydrocarbon radical having from 3 to 20 carbon atoms. Representative examples of monocyclic cycloalkyl groups include, but are not limited to, cyclopropyl, cyclobutyl, cyclopentyl, cyclopentenyl, cyclohexyl, cyclohexenyl, cycloheptyl, and cyclooctyl. Bicyclic and tricyclic cycloalkyl groups include bridged cycloalkyl, fused cycloalkyl and spiro-cycloalkyl groups such as, but not limited to, decalinyl, octahydropentalenyl, octahydro-1H-indenyl, spiro-cycloalkyl, adamantanyl, noradamantanyl, bornyl, norbornyl (also named as bicyclo[2.2.1]heptyl by the IUPAC system). As used herein, the "cycloalkyl" is optionally mono- or poly-substituted, such as, but not limited to, 2,2-, 2,3-, 2,4-, 2,5-, or 2,6-disubstituted cyclohexyl. The substituents of the substituted "cycloalkyl" can be optionally one or more (e.g., 1-5, 1-4, 1-3, 1-2, or 1) selected from C₁₋₃ alkyl, C₁₋₃ alkoxy, C₃₋₆ cycloalkyl, halogenated C₁₋₃ alkyl, halogen, hydroxyl, cyano, amino, or any combination thereof.

As used herein, the term "heterocyclyl" or "heterocyclic group" used alone or in combination refers to a 3- to 20-membered saturated or partially unsaturated (i.e., containing one or more double bonds, but not having a fully conjugated π-electron system) monocyclic, bicyclic, or tricyclic cyclic hydrocarbon group containing one or more (e.g., from 1 to 5, or from 1 to 4, or from 1 to 3, or from 1 to 2, or 1) heteroatoms independently selected from sulfur, oxygen, and nitrogen. In some embodiments, "heterocyclyl" may preferably refer to a 3- to 15-membered (e.g., 3- to 14-membered, 3- to 12-membered, 3- to 11-membered, 3- to 10-membered, 3- to 9-membered, 3- to 8-membered, 3-to 7-membered, 3- to 6-membered, or 3- to 5-membered) saturated or partially unsaturated (i.e., containing one or more double bonds, but not having a fully conjugated π-electron system) monocyclic cyclic hydrocarbon group containing one or more (e.g., from 1 to 5, or from 1 to 4, or from 1 to 3, or from 1 to 2, or 1) heteroatoms independently selected from sulfur, oxygen, and nitrogen. Representative examples of the heterocyclyl include, but are not limited to, azetidinyl, oxetanyl, pyrrolidinyl, imidazolidinyl, pyrazolidyl, triazolyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, 1,4-diazacycloheptan-1-yl, 3,8-diazabicyclo[3.2.1]octan-3-yl, 2,5-diazabicyclo[2.2.2]octan-2-yl, and azaspirocycloalkyl (e.g., 3-azaspiro[5.5]undecane-3-yl). The heterocyclyl may be unsubstituted or substituted as explicitly defined (e.g., mono-, di-, tri-, or poly-substituted), and the substituent(s) of the heterocyclyl can be preferably selected from C₁₋₃ alkyl, C₁₋₃ alkoxy, deuterated C₁₋₃ alkoxy, halogen, halogenated C₁₋₃ alkyl, hydroxy, cyano, oxo, C₁₋₃ alkyl-NH- amino, or any combination thereof.

As used herein, the term "heterocyclylene" used alone or in combination refers to a 3- to 20-membered saturated or partially unsaturated (i.e., containing one or more double bonds, but not having a fully conjugated π-electron system) monocyclic, bicyclic, or tricyclic bivalent cyclic hydrocarbon group containing one or more (e.g., from 1 to 5, or from 1 to 4, or from 1 to 3, or from 1 to 2, or 1) heteroatoms independently selected from sulfur, oxygen, and nitrogen. In some embodiments, "heterocyclylene" may preferably refer to a 3- to 15-membered (e.g., 3- to 14-membered, 3- to 12-membered, 3- to 11-membered, 3- to 10-membered, 3- to 9-membered, 3- to 8-membered, 3- to 7-membered, 3- to 6-membered, or 3- to 5-membered) saturated or partially unsaturated (i.e., containing one or more double bonds, but not having a fully conjugated π-electron system) monocyclic bivalent cyclic hydrocarbon group containing one or more (e.g., from 1 to 5, or from 1 to 4, or from 1 to 3, or from 1 to 2, or 1) heteroatoms independently selected from sulfur, oxygen, and nitrogen. Representative examples of the heterocyclylene include, but are not limited to, azetidinylene, oxetanylene, pyrrolidinylene, imidazolidinylene, pyrazolidylene, triazolylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, 1,4-diazacycloheptan-1-ylene, 3,8-diazabicyclo[3.2.1]octan-3-ylene, 2,5-diazabicyclo[2.2.2]octan-2-ylene, and azaspirocycloalkylene (e.g., 3-azaspiro[5.5]undecane-3-ylene). The heterocyclylene may be unsubstituted or substituted as explicitly defined (e.g., mono-, di-, tri-, or poly-substituted), and the substituent(s) of the heterocyclylene can be preferably selected from C₁₋₃ alkyl, C₁₋₃ alkoxy, deuterated C₁₋₃ alkoxy, halogen, halogenated C₁₋₃ alkyl, hydroxy, cyano, oxo, C₁₋₃ alkyl-NH- amino, or any combination thereof.

As used herein, the term "alkynylene", used alone or in combination, refers to a linear or branched divalent hydrocarbon group containing from 2 to 6 (e.g., from 2 to 5, from 2 to 4, preferably 2) carbon atoms and having one or more (e.g., from 1 to 3, from 1 to 2, or 1) carbon-carbon triple bonds. Examples of alkynylene include, but are not limited to, ethynylene, 1-propynylene, 1-butynylene, and 1,3-diynylene.

As used herein, the term "alkenylene", used alone or in combination, refers to a linear or branched divalent hydrocarbon group containing from 2 to 6 (e.g., from 2 to 5, from 2 to 4, from 2 to 3, or 2) carbon atoms and having one or more (e.g., from 1 to 3, from 1 to 2, or 1) carbon-carbon double bonds. Examples of alkenylene groups include, but are not limited to, vinylene (e.g., - CH=CH-), 1-propenylene, allylidene, 1-butenylene, 2-butenylene, 3-butenylene, isobutenylene, pentenylene, n-pent-2,4-dienylene, 1-methyl-but-1-enylene, 2-methyl-but-1-enylene, 3-methyl-but-1-enylene, 1-methyl-but-2-enylene, 2-methyl-but-2-enylene, 3-methyl-but-2-enylene, 1-methyl-but-3-enylene, 2-methyl-but-3-enylene, 3-methyl-but-3-enylene, and hexenylene.

As used herein, "bornylane" or "bornane" (also known as 1,7,7-trimethylbicyclo[2.2.1]heptane; camphane; bornylane) has a definition known to those skilled in the art. As used herein, "camphanyl" or "bornyl" refers to a monovalent group of bornane, i.e., the group remaining after any one of the hydrogens in bornane is removed. Representative examples of "bornyl" include, but are not limited to, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, or 1,7,7-trimethylbicyclo[2.2.1]heptan-6-yl, 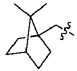 , or

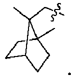

As used herein, "bicyclo[2.2.1]heptane" also known as bicyclo[2.2.1]heptane or "norbornane", has a definition known to those skilled in the art. As used herein, "bicyclo[2.2.1]heptyl" or "norbornyl" refers to a monovalent group of bicyclo[2.2.1]heptane, i.e., the group remaining after any hydrogen in bicyclo[2.2.1]heptane is removed. Representative examples of "bicyclo[2.2.1]heptyl" include, but are not limited to, bicyclo[2.2.1]heptan-2-yl, bicyclo[2.2.1]heptan-3-yl, bicyclo[2.2.1]heptan-4-yl, bicyclo[2.2.1]heptan-4-yl, bicyclo[2.2.1]heptan-5-yl, or bicyclo[2.2.1]heptan-6-yl.

As used herein, the term "bicyclo[2.2.1]heptene" has a definition known to those skilled in the art. As used herein, "bicyclo[2.2.1]heptenyl" refers to a monovalent group of bicyclo[2.2.1]heptene, i.e., the group remaining after any hydrogen in bicyclo[2.2.1]heptene is removed. Representative examples of "bicyclo[2.2.1]heptenyl" include, but are not limited to, bicyclo[2.2.1]hept-5-en-2-yl, bicyclo[2.2.1]hept-5-en- 3-yl, or bicyclo[2.2.1]hept-5-en-7-yl.

As used herein, "adamantane" (also known as tricyclo[3.3.1.1^{3,7}]decane) has a definition known to those skilled in the art, and its structural formula is as follows: 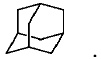 As used herein, "adamantanyl" refers to a monovalent group of adamantane, that is, the group remaining after any hydrogen in adamantane is removed. Representative examples of "adamantanyl" include, but are not limited to, 1-adamantanyl, 2-adamantanyl, 3-adamantanyl, 4-adamantanyl, 5-adamantanyl, 6-adamantanyl, 7-adamantanyl, 8-adamantanyl, 9-adamantanyl, or 10-adamantanyl.

As used herein, "noradamantane" has a definition known to those skilled in the art, and its structural formula is as follows: 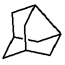 or 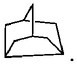 As used herein, "noradamantanyl" refers to a monovalent group of noradamantane, that is, the group remaining after any hydrogen in noradamantane is removed. Representative examples of "noradamantanyl" include, but are not limited to, 1-noradamantanyl, 2-noradamantanyl, 3-noradamantanyl, 4-noradamantanyl, 5-noradamantanyl, 6-noradamantanyl, 7-noradamantanyl, 8-noradamantanyl or 9-noradamantanyl.

As used herein, "adamantanamine" has the definitions known to those skilled in the art, namely referring to an adamantane having an amino substituent, wherein the amino substituent can replace a hydrogen on a carbon at any position in the adamantane. An example of "adamantanamine" can be adamantan-1-amine (corresponding English chemical name is adamantan-1-amine or Tricyclo[3.3.1.1^{3,7}]decan-1-amine; CAS No.: 768-94-5), with the following structural Formula:

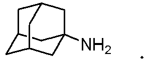

As used herein, "p-menthane" (also known as p-methane) has a definition known to those skilled in the art, and its structural formula is as follows: 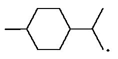 As used herein, "p-menthanyl" refers to a monovalent group of p-menthane, that is, the group remaining after any hydrogen in *p-*menthane is removed. Representative examples of "p-menthanyl" include, but are not limited to,

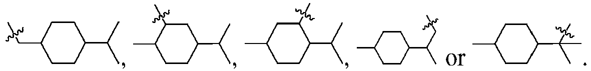

As used herein, "meta-menthane" (also known as meta-methane) has a definition known to those skilled in the art, and its structural formula is as follows: 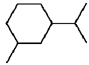 . As used herein, "meta-menthanyl" refers to a monovalent group of meta-menthane, that is, the group remaining after any hydrogen in meta-menthane is removed. Representative examples of "meta-menthanyl" include, but are not limited to, 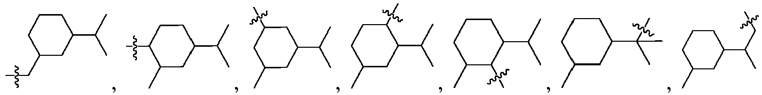 or

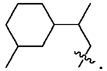

As used herein, "quinuclidine" (also known as 1 -azabicyclo [2.2.2] octane) has a definition known to those skilled in the art, and its structural formula is as follows: 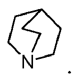 As used herein, "quinuclidinyl" refers to a monovalent group of quinuclidine, that is, the group remaining after any hydrogen in quinuclidine is removed. Representative examples of "quinuclidinyl" include, but are not limited to,

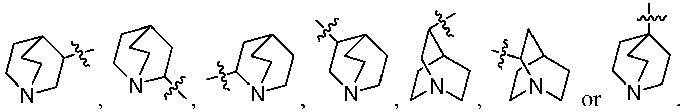

Salts or pharmaceutically acceptable salts, enantiomers, stereisomers, solvates, polymorphs of the compounds of Formula (I) of the present disclosure are also encompassed within the scope of the present invention.

In all embodiments of the present disclosure, the salts or pharmaceutically acceptable salts of the compounds of Formula (I) refer to non-toxic inorganic or organic acid and/or base addition salts. Examples include: sulfate, hydrochloride, citrate, maleate, sulfonate, citrate, lactate, tartrate, fumarate, phosphate, dihydrogenphosphate, pyrophosphate, metaphosphate, oxalate, malonate, benzoate, mandelate, succinate, glycolate, or p-toluenesulfonate, etc.

"Pharmaceutically acceptable carrier" refers to a pharmaceutically acceptable material, such as a filler, stabilizer, dispersant, suspending agent, diluent, excipient, thickener, solvent, or encapsulating material, with which the useful compounds according to the present disclosure are carried or transported into or administered to a patient so that they can perform their intended function. Generally, such constructs are carried or transported from one organ or part of the body to another organ or part of the body. The carrier is compatible with the other ingredients of the formulation, including the compounds useful in the present disclosure, and is not harmful to the patient, and the carrier must be "acceptable". Some examples of materials that can be used as pharmaceutically acceptable carriers include, but are not limited to, sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; and other common non-toxic compatible substances used in pharmaceutical formulations.

The term "treatment" or "treating" refers to the administration of the compound of Formula (I) or a pharmaceutically acceptable salt thereof according to the present disclosure, or the pharmaceutical composition containing, as an active ingredient, the compound of Formula I or a pharmaceutically acceptable salt thereof, to a subject to mitigate (alleviate) undesirable diseases or conditions, such as the development of a cancer or tumor. The beneficial or desired clinical results of the present disclosure include, but are not limited to: alleviating symptoms, reducing the severity of the disease, stabilizing the state of the disease, slowing down or delaying the progression of the disease, improving or alleviating the condition, and alleviating the disease.

A "therapeutically effective amount" of a compound of the present disclosure depends on a variety of factors, including the activity of the particular compound used; the metabolic stability and length of action of the compound; the age, sex, and weight of the patient; the patient's current medical condition,; mode and timing of administration; excretion rates; concomitant medications; the disease or disorder progression of the patient being treated. Those skilled in the art will be able to determine appropriate dosages based on these and other factors.

It should be understood that the choice of using one or more active compounds and/or compositions and their dosage depends on the basic situations of the individual (which should generally render the individual situation to achieve the best effect). Dosing and dosing regimens should be within the ability of those skilled in the art, and the appropriate dosage depends on many factors including the knowledge and ability of the physicians, veterinarians or researchers (see Wells et al. eds., Pharmacotherapy Handbook, 2nd Edition, Appleton and Lange, Stamford, Conn. (2000); PDR Pharmacopoeia, Tarascon Pocket Pharmacopoeia 2000, Deluxe Edition, Tarascon Publishing, Loma Linda, Calif. (2000)).

As used herein, the term "patient" or "subject" refers to animal, for example mammal, including but not limited to primate (such as human being), cow, sheep, goat, horse, dog, cat, rabbit, guinea pig, rat, mice, etc.

The term "room temperature" used herein refers to the ambient temperature, such as a temperature of 20-30 °C.

### Examples

In the following description, numerous specific details are set forth in order to provide a thorough understanding of the present disclosure. The present disclosure may be practiced without some or all of these specific details. In other cases, well-known process operations have not been described in detail in order not to unnecessarily obscure the present disclosure. Although the present disclosure will be described in conjunction with specific embodiments, it should be understood that this is not intended to limit the present disclosure to these embodiments.

The following abbreviations are used throughout the specification and examples:
- Ar: Argon
- ACN: Acetonitrile
- AIBN: Azobisisobutyronitrile
- Bipy: bipyridine
- BnCl: benzyl chloride
- Boc: tert-butoxycarbonyl
- BPO: dibenzoyl peroxide
- DCM: dichloromethane
- DIPEA: N,N-diisopropylethylamine
- DMF: N,N-dimethylformamide
- DMAP: N,N-dimethyl pyridine-4-amine
- DMSO: dimethyl sulfoxide
- EDCI: 1-(3-dimethylaminopropyl)-3-ethylcarbodiimide
- equiv or eq: equivalent
- ESI: electrospray ionization
- EtOAc: ethyl acetate
- h: hour
- HATU: 2-(7-azabenzotriazole)-N,N,N',N'-tetramethylurea hexafluorophosphate
- HPLC: high performance liquid chromatography
- HRMS: high-resolution mass spectrometry
- i-PrOH: isopropyl alcohol
- LAH: lithium aluminum tetrahydrogenide
- LC-MS: liquid chromatography-mass spectrometry
- LDA: lithium diisopropylamide
- LRMS: low resolution mass spectrometry
- LC: liquid chromatography
- MeOH: methanol
- MeCN: acetonitrile
- MsCl: methylsulfonyl chloride
- MsO: methylsulfonyloxy
- MW: microwave
- NaAc: sodium acetate
- NBS: N-bromosuccinimide
- NMP: N-methylpyrrolidone
- ¹H NMR: H NMR spectroscopy
- -OMe: methoxy
- PE: petroleum ether
- PhMe: toluene
- rt: room temperature
- tBuONO: tert-butyl nitrite
- TEA: triethylamine
- TFA: trifluoroacetate
- THF: tetrahydrofuran
- TLC: thin layer chromatography
- TMS: tetramethylsilane
- TsOH: p-toluenesulfonic acid
- TsO-: p-toluenesulfonyloxy

In the present disclosure, the ¹H NMR spectrum was recorded on a Bruker-500MHz nuclear magnetic resonance instrument, by using, as a solvent, CD₃OD (δ = 3.31 ppm) containing 0.1% TMS (as an internal standard); or using, as a solvent, CDCl₃ (δ = 7.26 ppm) containing 0.1% TMS (as an internal standard); or using, as a solvent, DMSO-*d*₆ (δ = 2.50 ppm) containing 0.03% TMS (as an internal standard). HRMS spectrum was recorded on an AB Triple 4600 mass spectrometer, and HPLC preparation was measured on a SHIMADZU LC-20AP type instrument, and HPLC purity was measured on a SHIMADZU LC-30AP or Waters 1525 type instrument. Unless otherwise specified, all reactions were performed in the air atmosphere. The reactions were followed by TLC or LC-MS, intermediates were isolated and purified by column chromatography using an ISCO or Biotage, and the designed and synthesized target products were separated and purified by the Waters 2767 preparative HPLC.

Solvents and reagents are processed as follows:
The solvents used in the reaction such as DCM, DMF, anhydrous EtOH, and anhydrous MeOH were purchased from Chinese Sinopharm Group; Preparative grade CH₃CN and deionized water were used in HPLC preparation. Unless otherwise specified, other reaction substrates, reagents, medicines were commercially available and used directly or obtained or prepared by methods known to those skilled in the art.

### General synthetic methods

The compounds described herein and/or pharmaceutically acceptable salts thereof can be synthesized using commercially available raw materials by synthetic techniques known in the art. The synthetic schemes described below illustrate the preparation of most compounds. The starting materials or reagents used in each scheme can be commercially available or prepared by methods known to those skilled in the art. The salts, racemates, enantiomers, carbamates, phosphates, sulfates, hydrochlorides and prodrug forms of the compounds of Formula (I) of the present invention can be prepared by those skilled in the art according to routine techniques in the art.

### General synthesis method 1:

### Scheme 1

In Scheme 1, the group X_{b} is as defined in Formula (I) of the present disclosure. In an alternative embodiment, the heterocyclyl group in Scheme 1 can be suitably modified to a heteroaryl group.

In Scheme 1, step 1: to a solution of aldehyde (1 eq) dissolved in tetrahydrofuran was added corresponding amine (1 eq). The mixture was reacted at room temperature until the reaction was complete as monitored. To the reaction mixture was added NaBH₄ (1.5 eq) in batches under ice bath, and then reacted at room temperature until the starting materials were consumed as monitored by LC-MS. A crude aldol-amine condensation product was obtained, which was used directly in the next step.

Step 2: To a mixture of the crude product from step 1 in DCM was slowly added dropwise PBr₃ (1.2 eq) under ice bath. Then the mixture was reacted under ice bath, concentrated under reduced pressure to give bromide product which was used directly in the next step.

Step 3: To a mixture of the above concentrated bromide product in N,N-dimethylformamide was added 1 eq of 3-(4-mercapto-1-oxoisoindolin-2-yl)piperidine-2,6-dione (CAS No.: 2378582-39-7) (or 3-(4-hydroxy-1-oxoisoindolin-2-yl)piperidine-2,6-dione (CAS No.: 1061604-41-8), or 2-(2,6-dioxopiperidin-3-yl)-4-mercaptoisoindoline-1,3-dione (CAS No.: 2378582-97-7), or 2-(2,6-dioxopiperidin-3-yl)-4-hydroxyisoindoline-1,3-dione (CAS No.: 5054-59-1)), and potassium carbonate (1.2 eq). The mixture was reacted at room temperature and filtered. The filtrate was subjected to preparative HPLC for separation, and lyophilized to obtain the corresponding final target compound.

### General synthesis method 2:

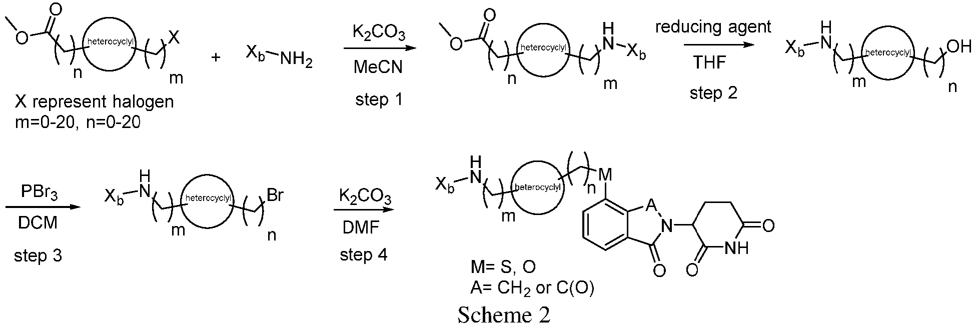

In Scheme 2, the group X_{b} is as defined in Formula (I) of the present disclosure. In an alternative embodiment, the heterocyclyl group in Scheme 2 can be suitably modified to a heteroaryl group.

Step 1: to a mixture of halocarboxylate (1.1 eq) in acetonitrile were added potassium carbonate (1.1 eq) and corresponding amine (1 eq). The mixture was reacted at 35°C to give amine-substituted intermediate.

Step 2: to a mixture of the amine-substituted intermediate from step 1 (1 eq) in THF was slowly added reducing agent (1.1 eq) under ice bath. The mixture was reacted at room temperature. To the mixture was added ethyl acetate, and the reaction was quenched with NaOH to give hydroxy substituted intermediate compound.

Step 3: to a mixture of the concentrate of the hydroxy substituted intermediate from step 2 in methylene chloride was added PBr₃ (1.1 eq) under ice bath. The mixture was reacted at room temperature, concentrated to give crude brominated product which was used directly in the next step.

Step 4: to a mixture of the concentrated brominated product in N, N-dimethylformamide were added 3-(4-mercapto-1-oxoisoindolin-2-yl)piperidine-2,6-dione (or 3-(4-hydroxy-1-oxoisoindolin-2-yl)piperidine-2,6-dione, or 2-(2,6-dioxopiperidin-3-yl)-4-mercaptoisoindoline-1,3-dione, or 2-(2,6-dioxopiperidin-3-yl)-4-hydroxyisoindoline-1,3-dione), and potassium carbonate (1.2 eq). The mixture was reacted at room temperature, and filtered. The filtrate was subjected to preparative HPLC for separation, and lyophilized to obtain the corresponding final target compound.

### General synthesis method 3:

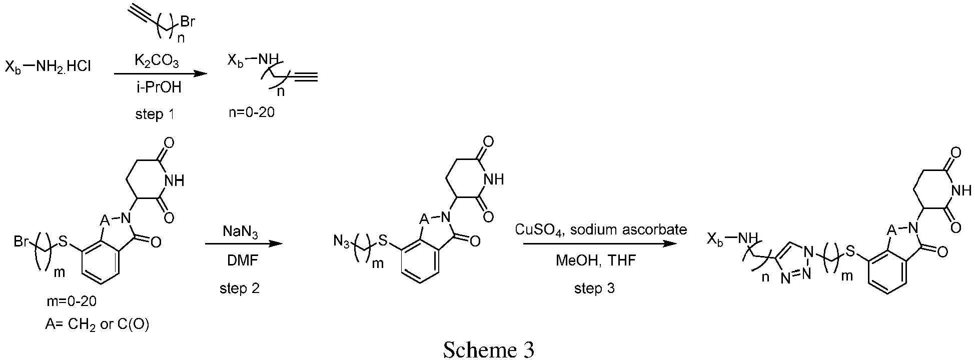

In Scheme 3, the group X_{b} is as defined in Formula (I) of the present disclosure.

Step 1: to a solution of amine hydrochloride (1eq) in isopropanol were sequentially added bromoalkyne (1eq) and potassium carbonate (3eq) at room temperature. The mixture was heated and reacted to give 3-alkynylamine compounds.

Step 2: to a solution of bromoalkyl thio-lenalidomide (1eq) (which can prepared according to Scheme 7 described in WO2019196812) in N,N-dimethylformamide was added sodium azide (1.1 eq) at room temperature. The mixture was heated and reacted to give the azide intermediate compound.

Step 3: to a solution of 3-alkynylamine compounds (1eq) and the azide intermediate (1eq) in methanol/tetrahydrofuran were sequentially added copper sulfate solution and sodium ascorbate solution. The mixture was reacted at room temperature to give triazolyl derivatives.

### General synthesis method 4:

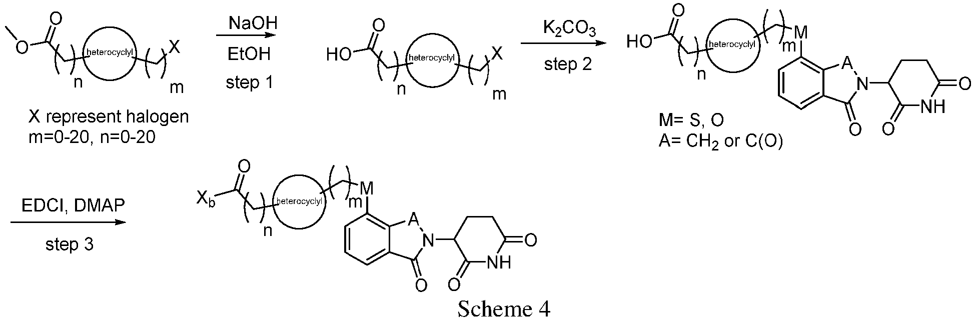

In Scheme 4, the group X_{b} is as defined in Formula (I) of the present disclosure. In an alternative embodiment, the heterocyclyl group in Scheme 4 can be suitably modified to a heteroaryl group.

Step 1: to a mixture of halocarboxylate (1 eq) in ethanol was added dropwise NaOH. The mixture was reacted at room temperature, and then the pH of the mixture was adjusted to acidity with HCl. the mixture was extracted to give the carboxylic acid intermediate.

Step 2: to a mixture of the carboxylic acid intermediate from step 1 and 3-(4-mercapto-1-oxoisoindolin-2-yl)piperidine-2,6-dione (1 eq) (or 3-(4-hydroxy-1-oxoisoindolin-2-yl)piperidine-2,6-dione, or 2-(2,6-dioxopiperidin-3-yl)-4-mercaptoisoindoline-1,3-dione, or 2-(2,6-dioxopiperidin-3-yl)-4-hydroxyisoindoline-1,3-dione) in DMF was added potassium carbonate (1.5 eq). The mixture was reacted at room temperature, and then the pH of the mixture was adjusted to acidity with HCl. The reaction mixture was subject to preparative reversed-phase liquid chromatography for separation and purification to give the carboxylic acid M-lenalidomide.

Step 3: An egg-shaped flask was charged with the carboxylic acid M-lenalidomide from step 2, amine with X_{b} group (1.1 eq) (or alcohol or thiol compound with X_{b} group), followed by addition of EDCI (1.5 eq) and catalytic amount of DMAP and DMF. The mixture was reacted at room temperature overnight. The reaction solution was suction filtered, and the filtrate was subjected to preparative HPLC for separation and purification. The collected fractions were concentrated, and lyophilized to give the desired target compound.

### General synthesis method 5:

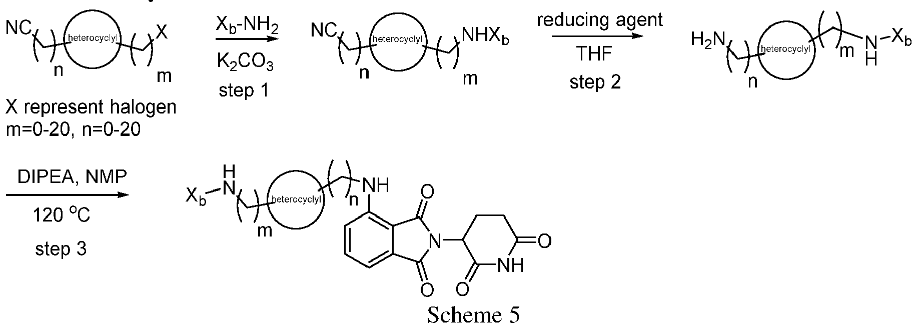

In Scheme 5, the group X_{b} is as defined in Formula (I) of the present disclosure. In an alternative embodiment, the heterocyclyl group in Scheme 5 can be suitably modified to a heteroaryl group.

Step 1: to a mixture of halogenated cyano compound and amine (1 eq) in acetonitrile was added potassium carbonate (1.5 eq). The mixture was reacted at room temperature to give amine-substituted intermediate.

Step 2: to a mixture of amine-substituted intermediate from step 1 in THF was slowly added reducing agent under ice bath. The mixture was reacted under ice bath for 1h, and at room temperature for 2h. To the mixture was added ethyl acetate for dilution, and added dropwise NaOH. The resulting mixture was filtered, and the filtrate was concentrated to give the cyano reduction product which was used directly in next step.

Step 3: To a mixture of 2-(2,6-dioxopiperidin-3-yl)-4-fluoroisoindoline-1,3-dione (CAS No.: 835616-60-9) (1 eq) in NMP were added overdose of product from step 2 and DIPEA (1.5 eq). The mixture was reacted in microwave, and then filtered. The filtrate was subjected to preparative HPLC for separation. The collected fractions were concentrated, and lyophilized to give the desired target compound.

### General synthesis method 6:

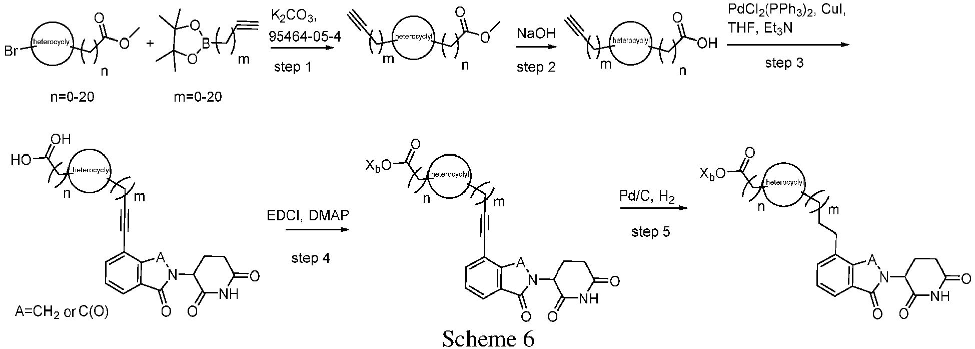

In Scheme 6, the group X_{b} is as defined in Formula (I) of the present disclosure, and 95464-05-4 refers to the compound indicated by CAS No.: 95464-05-4, i.e., 1,1'-Bis(diphenylphosphino)ferrocene-palladium(II)dichloride dichloromethane complex. In an alternative embodiment, the heterocyclyl group in Scheme 6 can be suitably modified to a heteroaryl group.

Step 1: to a mixture of bromocarboxylate compound and alkynyl borate (1 eq) in dioxane were added water, potassium carbonate (2 eq) and 95464-05-4 (10%/mol). The mixture was reacted under reflux to give the alkynyl carboxylate intermediate which was used directly in next step.

Step 2: to a solution of the alkynyl carboxylate intermediate from step 1 in ethanol was added NaOH. The mixture was reacted at 45 °C overnight, and cooled, and the pH of the mixture was adjusted to acidity with dilute hydrochloric acid. The resulting mixture was concentrated to give 5-alkynyl carboxylic acid intermediate which was used directly in next step.

Step 3: to a mixture of the 5-alkynyl carboxylic acid intermediate and 4-bromine lenalidomide (CAS No.: 2093387-36-9) (1 eq) or 4-bromo pomalidomide (CAS No.: 2093536-12-8) (1 eq) in tetrahydrofuran were added triethylamine (1.2 eq), triphenylphosphine palladium chloride (10%/mol) and cuprous iodide. The mixture was reacted under reflux, filter, and the filtrate was subjected to preparative reversed-phase liquid chromatography for separation to give the desired carboxylic acid intermediate.

Step 4: An egg-shaped flask were charged with the carboxylic acid intermediate from step 3 and compound having amino, hydroxy or mercapto (1.1 eq), followed by addition of EDCI (1.5 eq) and catalytic amount of DMAP and DMF. The mixture was reacted at room temperature overnight. The reaction solution was suction filtered, and the filtrate was subjected to preparative HPLC for separation and purification. The collected fractions were concentrated, and lyophilized to give the desired target compound. **Step 5:** to a mixture of the condensation product from step 4 in methanol was added 10% Pd/C. The mixture was stirred overnight in a hydrogen atmosphere, filtered, and the filtrate was subjected to preparative HPLC for separation, and lyophilized to give the corresponding reduced target compound.

Depending upon the target compounds, the above schemes and their reaction substrates, reaction conditions (including reaction dosage, temperature, duration, etc.), work up, etc. can be appropriately modified and adjusted by techniques and methods well known to those skilled in the art to obtain the desired target compounds. The obtained target compounds can be further modified by changing the substituents and the like to obtain subsequent target compounds through methods well known to those skilled in the art.

### Examples

### Example 1: preparation of hydrochloride of 3-(4-(((5-((adamantan-1-ylamino)methyl)furan-2-yl)methyl)thio)-1-oxoisoindolin-2-yl)piperidine-2,6-dione (SIAIS313118)

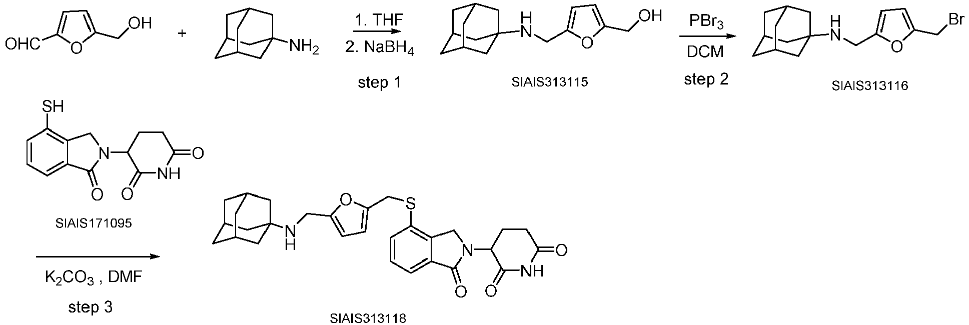

With reference to the method of Scheme 1, the target compound was prepared under appropriate conditions that will be recognized by one skilled in the art.

Specifically, step 1: to a solution of 5-(hydroxymethyl)furan-2-carbaldehyde (0.126 g, 1 mmol) dissolved in tetrahydrofuran (5 mL) was added 1-adamantanamine (1 eq). The mixture was reacted at room temperature for 2h until the reaction was complete as monitored. To the reaction mixture was added NaBH₄ (56.7 mg, 1.5 eq) in batches under ice bath, and then reacted at room temperature for 1h until the starting materials were consumed as monitored by LC-MS. To the reaction mixture was added water (2 mL). The resulting mixture was extracted with ethyl acetate. The ethyl acetate organic phase was washed with saturated brine, concentrated to give crude SIAIS313115 which was used directly in next step. MS m/z: 262 [M+H⁺].

Step 2: To a mixture of the crude concentrate from step 1 in DCM (5 mL) was slowly added dropwise PBr₃ (112 µL, 1.2 eq) under ice bath. Then the mixture was reacted under ice bath for 2h, and then concentrated under reduced pressure to give crude product SIAIS313116 which was used directly in the next step. MS m/z: 324 [M+H⁺].

Step 3: To a mixture of the above concentrated crude product in N,N-dimethylformamide (5 mL) was added 3-(4-mercapto-1-oxoisoindolin-2-yl)piperidine-2,6-dione (SIAIS171095; CAS No.: 2378582-39-7) (1 eq) and potassium carbonate (1.2 eq). The mixture was reacted at room temperature for 2h and filtered. The filtrate was subjected to preparative HPLC (eluent (v/v): acetonitrile/(water+0.05%HCl) = 10%-100%) for separation. The collected fractions were rotary evaporated to remove acetonitrile, and lyophilized to give the hydrochloride of target compound SIAIS313118 (white solid, 0.1 g, yield 19%). ¹H NMR (500 MHz, DMSO) *δ* 10.89 (s, 1H), 9.27 (s, 2H), 7.72 (d, *J* = 7.7 Hz, 1H), 7.62 (d, *J* = 7.4 Hz, 1H), 7.53 (t, *J* = 7.6 Hz, 1H), 6.58 (s, 1H), 6.28 (s, 1H), 5.11 (dd, *J* = 13.1, 5.0 Hz, 1H), 4.35 (s, 2H), 4.33 (d, *J =* 17.3 Hz, 1H), 4.22 (d, *J =* 17.3 Hz, 1H), 4.08 (t, *J* = 5.4 Hz, 2H), 2.96 - 2.85 (m, 1H), 2.61 (d, *J* = 17.4 Hz, 1H), 2.48 - 2.38 (m, 1H), 2.10 (s, 3H), 2.06 - 1.98 (m, 1H), 1.90 (s, 6H), 1.66 (d, *J* = 12.2 Hz, 3H), 1.58 (d, *J* = 12.1 Hz, 3H). HRMS (ESI) C₂₉H₃₄N₃O₄S⁺ [M+H]⁺, calcd for 520.2270; found, 520.2266.

### Example 2: preparation of hydrochloride of 3-(4-(((2-((adamantan-1-ylamino)methyl)thiazol-4-yl)methyl)thio)-1-oxoisoindolin-2-yl)piperidine-2,6-dione (SIAIS313160)

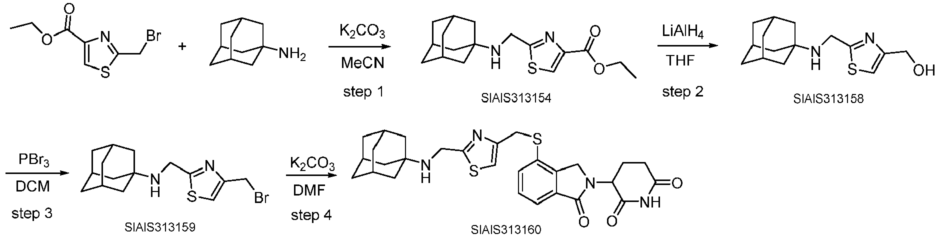

With reference to the method of Scheme 2, the target compound was prepared under appropriate conditions that will be recognized by one skilled in the art.

Specifically, step 1: to a mixture of ethyl 2-(bromomethyl)thiazole-4-carboxylate (0.15 g, 0.6 mmol) in acetonitrile were added potassium carbonate (91 mg, 1.1 eq) and 1-adamantanamine (99.6 mg, 1.1 eq). The mixture was reacted at 35°C for 2h, and then concentrated. The resulting residue was subjected to column chromatography to give compound SIAIS313154 (0.16 g, 81.6%). MS m/z: 321 [M+H⁺].

Step 2: the compound SIAIS313154 (0.16 g, 0.5 mmol) in THF was slowly added LiAlH₄ (21 mg, 1.1 eq) under ice bath. The mixture was then reacted at room temperature for 1h. To the mixture was added ethyl acetate (5 mL), and the reaction was quenched with NaOH (2M). The resulting mixture was filtered, extracted with ethyl acetate, and concentrated to give crude prodct SIAIS313158 which was used directly in next step. MS m/z: 279 [M+H⁺].

Step 3: to a mixture of the concentrate containing the crude prodct SIAIS313158 in methylene chloride was added dropwise PBr₃ (60 µl, 1.1 eq) under ice bath. The mixture was reacted at room temperature for 1h, concentrated to give crude product SIAIS313159 which was used directly in the next step.

Step 4: to a mixture of the concentrated product SIAIS313159 in N, N-dimethylformamide were added compound SIAIS171095 (1 eq) and potassium carbonate (1.2 eq). The mixture was reacted at room temperature for 2h, and filtered. The filtrate was subjected to preparative HPLC (eluent (v/v): acetonitrile/(water+0.05%HCl) = 10%-100%) for separation. The collected fractions were rotary evaporated to remove acetonitrile, and lyophilized to give the hydrochloride of target compound SIAIS313160 (white solid, 38 mg, yield 14%). ¹H NMR (500 MHz, DMSO) *δ* 11.01 (s, 1H), 9.34 (s, 2H), 7.73 (d, *J* = 7.5 Hz, 1H), 7.60 (d, *J* = 8.0 Hz, 2H), 7.53 (t, *J* = 7.5 Hz, 1H), 5.13 (dd, *J* = 13.1, 4.3 Hz, 1H), 4.49 (d, *J* = 16.3 Hz, 4H), 4.32 (d, *J* = 17.3 Hz, 1H), 4.19 (d, *J* = 17.4 Hz, 1H), 2.98 - 2.83 (m, 1H), 2.65 - 2.56 (m, 1H), 2.49 - 2.32 (m, 1H), 2.13 (s, 3H), 2.05 - 1.97 (m, 1H), 1.93 (s, 6H), 1.67 (d, *J* = 12.0 Hz, 3H), 1.59 (d, *J* = 12.0 Hz, 3H). HRMS (ESI) C₂₈H₃₃N₄O₃S₂⁺ [M+H]⁺, calcd for 537.1994; found, 537.1997.

### Example 3: preparation of hydrochloride of 3-(4-(((4-((adamantan-2-ylamino)methyl)thiazol-2-yl)methyl)thio)-1-oxoisoindolin-2-yl)piperidine-2,6-dione (SIAIS313185)

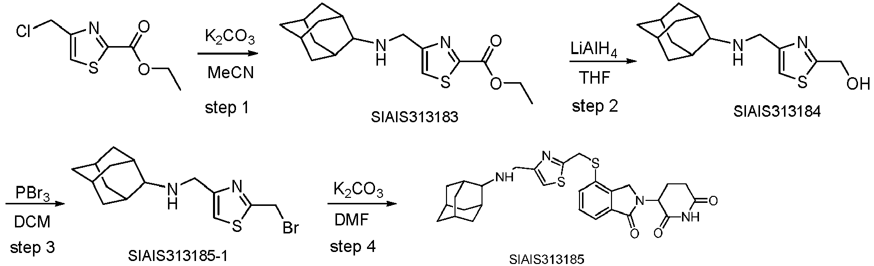

With reference to the method of Scheme 2, the target compound was prepared under appropriate conditions that will be recognized by one skilled in the art.

Specifically, step 1: to a mixture of ethyl 4-(chloromethyl)thiazole-2-carboxylate (0.2 g, 0.8 mmol) in acetonitrile were added potassium carbonate (91 mg, 1.1 eq) and 2-adamantanamine (1.1 eq). The mixture was reacted at 35°C for 2h, then concentrated, and the obtained product was used directly in the next step.

Step 2: to a mixture of compound SIAIS313183 in THF was slowly added LiAlH₄ (33 mg, 1.1 eq) under ice bath. The mixture was reacted at room temperature for 1h. To the mixture was added ethyl acetate (5 mL), and the reaction was quenched with 2 M NaOH. The resulting mixture was filtered, extracted with ethyl acetate, concentrated and subjected to column chromatography to give compound SIAIS313184 (30 mg, 17%). MS m/z: 279 [M+H⁺].

Step 3: to a mixture of the concentrate containing compound SIAIS313184 in methylene chloride was added dropwise PBr₃ (14.4 µL, 1.1 eq) under ice bath. The mixture was reacted at room temperature for 1h, and concentrated to give compound SIAIS313185-1 which was used directly in the next step.

Step 4: to a mixture of the concentrate from step 3 in N, N-dimethylformamide (5ml) were added compound SIAIS171095 (1 eq) and potassium carbonate (1.2 eq). The mixture was reacted at room temperature for 2h, and filtered. The filtrate was subjected to preparative HPLC (eluent (v/v): acetonitrile/(water+0.05%HCl) = 10%-100%) for separation. The collected fractions were rotary evaporated to remove acetonitrile, and lyophilized to give the hydrochloride of target compound SIAIS313185 (white solid, 15 mg, yield 17%). ¹H NMR (500 MHz, DMSO) *δ* 11.00 (s, 1H), 9.12 (s, 2H), 7.85 (s, 1H), 7.72 (dd, *J* = 13.1, 7.8 Hz, 1H), 7.62 (d, *J* = 7.5 Hz, 1H), 7.51 (t, *J* = 7.6 Hz, 1H), 5.13 (dd, *J* = 13.2, 5.1 Hz, 1H), 4.75 (s, 2H), 4.37 (d, *J* = 17.6 Hz, 1H), 4.24 (d, *J* = 16.7 Hz, 3H), 3.14 (d, *J* = 26.7 Hz, 1H), 2.99 - 2.84 (m, 1H), 2.60 (d, *J* = 15.7 Hz, 1H), 2.48 - 2.37 (m, 1H), 2.12 (s, 2H), 2.06 (d, *J* = 13.4 Hz, 2H), 2.02 - 1.95 (m, 1H), 1.79 (d, *J* = 10.9 Hz, 4H), 1.68 (s, 2H), 1.60 - 1.47 (m, 4H). HRMS (ESI) C₂₈H₃₃N₄O₃S₂⁺ [M+H]⁺, calcd for 537.1994; found, 537.1990.

### Example 4: preparation of hydrochloride of 3-(4-(((4-((adamantan-1-ylamino)methyl)thiazol-2-yl)methyl)thio)-1-oxoisoindolin-2-yl)piperidine-2,6-dione (SIAIS287129)

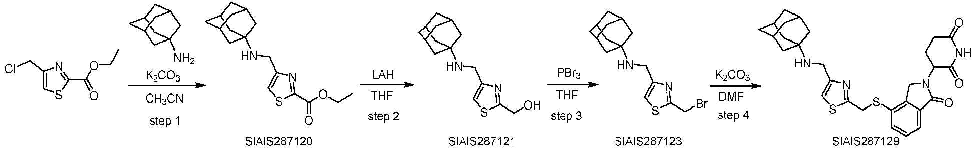

With reference to the method of Scheme 2, the target compound was prepared under appropriate conditions that will be recognized by one skilled in the art.

Specifically, step 1: to a solution of ethyl 4-(chloromethyl)thiazole-2-carboxylate (500 mg, 2.43 mmol) and 1-adamantanamine (456 mg, 2.43 mmol) in acetonitrile (10 ml) was added potassium carbonate (672 mg, 4.86 mmol). The mixture was warmed to 70°C and stirred for 16 hours. The mixture was cooled to room temperature and filtered. The filtrate was concentrated under reduced pressure, and the residue was washed with water (10 mL), extracted with dichloromethane (20 mL×3). The combined organic phases were dried over anhydrous sodium sulfate, filtered, and the filtrate was concentrated under reduced pressure. The resulting residue was subjected to column chromatography (dichloromethane : methanol = 10:1) for separation to give compound SIAIS287120 (0.43 g, yield 48%) as a white solid. ¹H NMR (500 MHz, CDCl₃) *δ* 7.61 (s, 1H), 4.48 (q, *J =* 10.0 Hz, 2H), 4.07 (s, 2H), 2.11 (s, 3H), 1.76 (s, 6H), 1.72-1.63 (m, 6H), 1.43 (t, *J =* 10.0 Hz, 3H). MS m/z: 321.3 [M+H⁺].

Step 2: to a solution of compound SIAIS287120 (100 mg, 0.31 mmol) in THF (4 mL) was slowly added LiAlH₄ (24 mg, 0.62 mmol) at 0 °C. The reaction solution was reacted at 0 °C for 2h. The reaction was quenched with water (0.1 mL). The resulting mixture was stirred for 10 min, dried over anhydrous sodium sulfate. The mixture was filtered, and the filtrate was concentrated under reduced pressure to give crude product SIAIS287121 (pale yellow solid, 80 mg) which was directly used in the next step. MS m/z: 279.3 [M+H⁺].

Step 3: to a solution of compound SIAIS287121 (80 mg, 0.29 mmol) in anhydrous tetrahydrofuran (4 mL) was added phosphorus tribromide (78 mg, 0.29 mmol) at 0 °C. The reaction solution was stirred at 0 °C for 1 hour. The reaction was quenched with water (10 mL), extracted with dichloromethane (20 mL×3). Organic phases were combined, dried over anhydrous sodium sulfate, filtered, and the filtrate was concentrated under reduced pressure to give SIAIS287123 (110 mg) as a white solid. The crude product was used directly in the next step. MS m/z: 341.2, 343.1 [M+H⁺].

Step 4: to a solution of the compound SIAIS287123 (30 mg, 0.09 mmol) and SIAIS171095 (24 mg, 0.09 mmol) in DMF (2ml) was added potassium carbonate (24 mg, 0.18 mmol). The mixture was stirred at room temperature for 1 hour, and filtered. The filtrate was subjected to high performance liquid chromatography (hydrochloric acid eluent system) for separation to give the hydrochloride of SIAIS287129 (white solid, 4 mg, yield 9%). ¹H NMR (500 MHz, MeOD) *δ* 7.74 (d, *J =* 5.0 Hz, 1H), 7.71 (d, *J =* 5.0 Hz, 1H), 7.59 (s, 1H), 7.52 (t, *J =* 5.0 Hz, 1H), 5.16 (dd, *J =* 10.0, 5.0 Hz, 1H), 4.60 (s, 2H), 4.42 (d, *J* = 15.0 Hz, 1H), 4.29 (d, *J* = 15.0 Hz, 1H), 4.22 (s, 2H), 2.94-2.87 (m, 1H), 2.80-2.76 (m, 1H), 2.54-2.45 (m, 1H), 2.21(s, 3H), 2.20-2.13 (m, 1H), 1.94 (s, 6H), 1.80 (d, *J =* 10.0 Hz, 3H), 1.72 (d, *J* = 10.0 Hz, 3H). HRMS: calcd for C₃₂H₃₃N₄O₃S₂⁺ [M+H]⁺ 537.1916, found, 537.1969.

### Example 5: preparation of hydrochloride of 3-(4-((2-(4-((adamantan-1-ylamino)methyl)-1H-1,2,3-triazol-1-yl)ethyl)thio)-1-oxoisoindolin-2-yl)piperidine-2,6-dione (SIAIS287097)

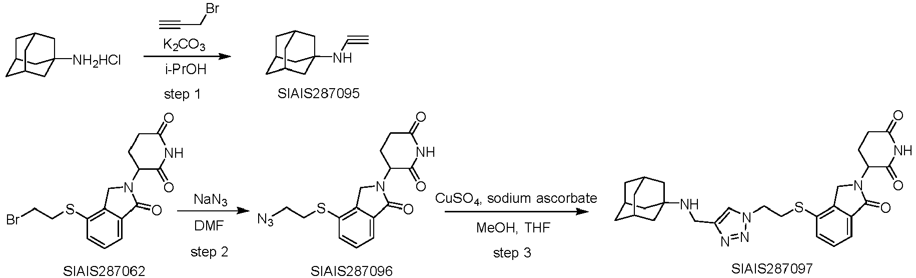

With reference to the method of Scheme 3, the target compound was prepared under appropriate conditions that will be recognized by one skilled in the art.

Specifically, step 1: to a solution of 1-adamantanamine hydrochloride (300 mg, 1.98 mmol) in isopropanol (4 ml) were sequentially added bromopropyne (236 mg, 1.98 mmol) and potassium carbonate (822 mg, 5.95 mmol) at room temperature. The mixture was warmed to 70°C and stirred for 5 hours. The mixture was filtered, and the filtrate was concentrated under reduced pressure, and subjected to column chromatography (PE: EtOAc = 1:1) for separation to give SIAIS287095 (170 mg, yield 45%) as a colorless oil. ¹H NMR (500 MHz, CDCl₃) *δ* 3.44 (d, *J =* 5.0 Hz, 2H), 2.20 (t, *J =* 5.0 Hz, 1H), 2.12-2.05 (m, 3H), 1.72-1.66 (m, 9H), 1.64-1.57 (m, 3H).

Step 2: to a solution of compound SIAIS287062 (100 mg, 0.26 mmol) (which can prepared according to intermediate example 41 described in WO2019196812) in N,N-dimethylformamide (3 mL) was added sodium azide (21 mg, 0.31 mmol) at room temperature. The mixture was warmed to 60°C and stirred for 2 hours. The mixture was diluted with ethyl acetate (30 mL), washed with water (10 mL×3) and saturated brine (10 mL). Organic phase was dried over anhydrous sodium sulfate, and filtered. The filtrate was concentrated under reduced pressure, and subjected to column chromatography (PE : EtOAc = 1:4) to give compound SIAIS287096 as a white solid (30 mg, yield 33%). ¹H NMR (500 MHz, CDCl₃) *δ* 7.97 (s, 1H), 7.79 (d, *J =* 10.0 Hz, 1H), 7.57 (d, *J =* 10.0 Hz, 1H), 7.51 (t, *J* = 10.0 Hz, 1H), 5.24 (dd, *J* = 10.0, 5.0 Hz, 1H), 4.47 (d, *J* = 15.0 Hz, 1H), 4.34 (d, *J =* 15.0 Hz, 1H), 3.49 (t, *J* = 10.0 Hz, 2H), 3.16 (dt, *J* = 10.0, 5.0 Hz, 2H), 2.98-2.91 (m, 1H), 2.88-2.81 (m, 1H), 2.45-2.36 (m, 1H), 2.26-2.22 (m, 1H). MS *m*/*z*: 346.2 [M+H⁺].

Step 3: to a solution of compound SIAIS287095 (10 mg, 0.053 mmol) and SIAIS287096 (18 mg, 0.053 mmol) in methanol (2.5 mL) and tetrahydrofuran (0.5 mL) were sequentially added copper sulfate solution (0.16 mL, 0.16 mmol, 1 M) and sodium ascorbate solution (0.079 mL, 0.079 mmol, 1 M). The mixture was stirred at room temperature for 3 hours. The mixture was filtered, and the filtrate was subjected to high performance liquid chromatography (hydrochloric acid eluent system) for separation to give the hydrochloride of compound SIAIS287097 (white solid, 17 mg, yield 61%). ¹H NMR (500 MHz, MeOD) *δ* 8.06 (s, 1H), 7.71 (dd, *J* = 10.0, 5.0 Hz, 2H), 7.55 (t, *J* = 10.0 Hz, 1H), 5.21 (dd, *J* = 10.0, 5.0 Hz, 1H), 4.76-4.67 (m, 2H), 4.45(q, *J* = 20.0 Hz, 2H), 4.20(q, *J* = 20.0 Hz, 2H), 3.72-3.56 (m, 2H), 2.99-2.91 (m, 1H), 2.85-2.80 (m, 1H), 2.59-2.50 (m, 1H), 2.28-2.21 (m, 4H), 2.00 (s, 6H), 1.85-1.74 (m, 6H). HRMS: calcd for C₂₈H₃₅N₆O₃S⁺ [M+H]⁺ 535.2413, found, 535.2479.

### Example 6: preparation of hydrochloride of 3-(4-(((5-((adamantan-1-ylamino)methyl)benzofuran-2-yl)methyl)thio)-1-oxoisoindolin-2-yl)piperidine-2,6-dione (SIAIS287116)

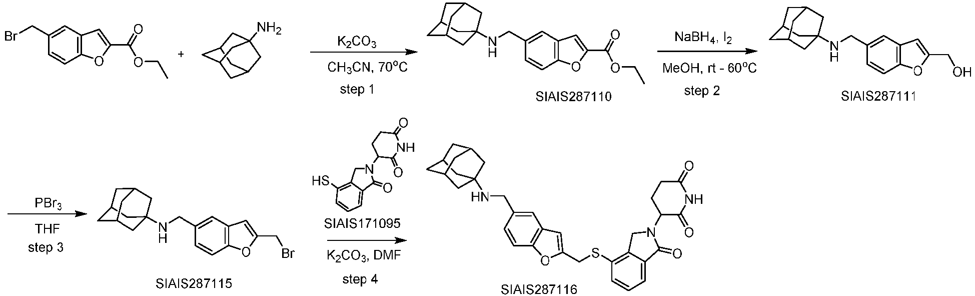

With reference to the method of Scheme 2, the target compound was prepared under appropriate conditions that will be recognized by one skilled in the art.

Specifically, step 1: to a solution of compound ethyl 5-(bromomethyl)benzofuran-2-carboxylate (240 mg, 0.74 mmol) and 1-adamantanamine (138 mg, 0.74 mmol) in acetonitrile (5 mL) was added potassium carbonate (307 mg, 2.23 mmol) at room temperature. The mixture was warmed to 70°C and stirred for 2 hours. The mixture was filtered, and the filtrate was concentrated under reduced pressure. The residue was washed with water (10 mL), extracted with dichloromethane (20 mL×3). Organic phases were combined, dried over anhydrous sodium sulfate, and filtered. The resulting filtrate was concentrated under reduced pressure, and the residue was subjected to a column chromatography (petroleum ether: ethyl acetate = 1:2) for separation to give compound SIAIS287110 (pale yellow solid, 0.24 g, yield 92%). ¹H NMR (500 MHz, CDCl₃) δ 7.61-7.58 (m, 2H), 7.49 (d, *J* = 5.0 Hz, 1H), 7.31 (d, *J* = 10.0 Hz, 1H), 4.43 (q, *J* = 10.0 Hz, 2H), 3.89 (s, 2H), 2.09 (s, 3H), 1.72-1.63 (m, 12H), 1.42 (t, *J* = 10.0 Hz, 3H). MS m/z: 354.5 [M+H]⁺.

Step 2: to a solution of compound SIAIS287110 (100 mg, 0.28 mmol) in methanol (5 mL) was added sodium borohydride (21 mg, 0.56 mmol) and iodine (71 mg, 0.28 mmol) at room temperature. The reaction solution was stirred at room temperature for 4 hours, and then warmed to 60 °C, and stirred for 12 hours. The reaction solution was concentrated under reduced pressure, and the residue was washed with water (10 mL), extracted with dichloromethane (20 mL×3). Organic phases were combined, dried over anhydrous sodium sulfate, and filtered. The filtrate was concentrated under reduced pressure, and the residue was subjected to column chromatography (dichloromethane : methanol = 10:1) for separation to give compound SIAIS287111 (pale yellow solid, 72 mg, yield 82%). MS m/z: 312.3 [M+H]⁺.

Step 3: to a solution of compound SIAIS287111 (40 mg, 0.13 mmol) in anhydrous tetrahydrofuran (2 ml) was added phosphorus tribromide (14 mg, 0.05 mmol) at 0 °C. The reaction solution was stirred at 0 °C for 2 hours. The reaction solution was quenched with water (5 mL), extracted with dichloromethane (20 mL×3). Organic phases were combined, dried over anhydrous sodium sulfate, and filtered. The filtrate was concentrated under reduced pressure to give compound SIAIS287115 (30 mg) as pale yellow solid. The crude product was used directly in the next step. MS m/z: 374.2, 376.1 [M+H]⁺.

Step 4: to a solution of compound SIAIS287115 (30 mg, 0.08 mmol) and SIAIS171095 (22 mg, 0.08 mmol) in DMF (2 ml) was added potassium carbonate (22 mg, 0.16 mmol). The mixture was stirred at room temperature for 2 hours. The mixture was filtered, and the filtrate was subjected to preparative HPLC (hydrochloric acid eluent system) for separation to give the hydrochloride of compound SIAIS287116 (white solid, 14 mg, yield 31%). ¹H NMR (500 MHz, MeOD) *δ* 7.73-7.67 (m, 2H), 7.59 (d, *J* = 10.0 Hz, 1H), 7.55 (d, *J* = 10.0 Hz, 1H), 7.49 (t, *J* = 10.0 Hz, 1H), 7.33-7.28 (m, 1H), 6.56 (s, 1H), 5.09 (dd, *J =* 15.0, 5.0 Hz, 1H), 4.37 (s, 2H), 4.34-4.28 (m, 2H), 4.25 (s, 2H), 2.90-2.83 (m, 1H), 2.75-2.70 (m, 1H), 2.31-2.28 (m, 1H), 2.26 (s, 3H), 2.04(s, 6H), 2.03-1.99 (m, 1H), 1.86-1.74 (m, 6H). HRMS: calcd for C₃₃H₃₆N₃O₄S⁺ [M+H]⁺ 570.2348, found, 570.2418.

### Example 7: preparation of hydrochloride of 3-(4-(((6-((adamantan-1-ylamino)methyl)quinolin-2-yl)methyl)thio)-1-oxoisoindolin-2-yl)piperidine-2,6-dione (SIAIS338048)

With reference to the method of example 6, the hydrochloride of compound SIAIS338048 was prepared under appropriate conditions that will be recognized by one skilled in the art, except that the ethyl 5-(bromomethyl)benzofuran-2-carboxylate in step 1 was replaced by ethyl 5-(bromomethyl)quinoline-2-carboxylate.

The hydrochloride of compound SIAIS338048 was obtained as a white solid (6.2 mg, yield 36%). ¹H NMR (500 MHz, MeOD) *δ* 8.86 (d, *J* = 8.6 Hz, 1H), 8.36 (s, 1H), 8.13 (d, *J* = 9.2 Hz, 2H), 7.91 (d, *J* = 8.5 Hz, 1H), 7.70 (d, *J* = 7.6 Hz, 1H), 7.62 (d, *J* = 7.6 Hz, 1H), 7.43 (t, *J* = 7.5 Hz, 1H), 5.09 (dd, *J* = 13.2, 4.8 Hz, 1H), 4.77 - 4.60 (m, 2H), 4.44 (d, *J* = 18.8 Hz, 3H), 4.28 (d, *J* = 17.5 Hz, 1H), 2.92 - 2.84 (m, 1H), 2.76 (d, *J* = 17.8 Hz, 1H), 2.46 - 2.38 (m, 1H), 2.27 (s, 3H), 2.15 - 2.05 (m, 7H), 1.81 (dd, *J* = 30.4, 12.6 Hz, 6H). MS m/z: 581.5 [M+H⁺].

### Example 8: preparation of adamantan-1-yl 4-(((2-(2,6-dioxopiperidin-3-yl)-1-oxoisoindolin-4-yl)thio)methyl)thiazole-2-carboxylate (SIAIS355072)

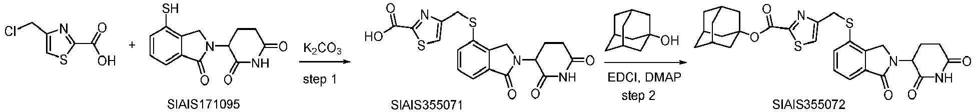

With reference to the method of Scheme 4, the target compound was prepared under appropriate conditions that will be recognized by one skilled in the art.

Specifically, step 1: to a mixture of 4-(chloromethyl)thiazole-2-carboxylic acid (0.35 g, 2 mmol) and compound SIAIS171095 (0.55 g, 1 eq) in DMF (10 mL) was added potassium carbonate (0.41 g, 1.5 eq). The mixture was reacted at room temperature for 2h, and then the pH of the mixture was adjusted to acidity with 1N HCl. The resulting mixture was subjected to preparative reverse-phase liquid chromatography for separation and purification to give the intermediate compound SIAIS355071 (pink solid, 0.19 g, yield 23%). MS m/z: 418 [M+H]⁺.

Step 2: a 10 mL egg-shaped flask was charged with compound SIAIS355071 (38 mg, 0.09 mmol) and 1-adamantanol (15 mg, 1.1 eq), followed by addition of EDCI (26 mg, 1.5 eq) and a catalytic amount of DMAP and DMF. The mixture was reacted at room temperature overnight. The reaction solution was suction filtered, and the filtrate was subjected to preparative HPLC (eluent (v/v): acetonitrile/(water + 0.05% HCl) = 10% - 100%) for separation. The collected fractions were rotary evaporated to remove acetonitrile, and lyophilized to give the target compound SIAIS355072 (white solid, 5 mg, yield 10%). ¹H NMR (500 MHz, DMSO) *δ* 10.98 (s, 1H), 7.86 (s, 1H), 7.75 (d, *J* = 7.7 Hz, 1H), 7.60 (d, *J* = 8.0 Hz, 1H), 7.51 (t, *J* = 7.4 Hz, 1H), 5.12 (d, *J* = 13.2 Hz, 2H), 4.53 (s, 2H), 4.24 (dd, *J* = 50.5, 17.4 Hz, 2H), 2.91 (t, *J* = 12.5 Hz, 1H), 2.66 - 2.55 (m, 1H), 2.46 - 2.34 (m, 1H), 2.07 (s, 2H), 1.99 (d, *J* = 11.2 Hz, 4H), 1.90 - 1.77 (m, 5H), 1.74 (d, *J* = 10.1 Hz, 2H), 1.59 (d, *J* = 12.2 Hz, 2H). HRMS: calcd for C₂₈H₃₀N₃O₅S₂⁺ [M+H]⁺ 552.1626, found, 552.1621.

### Example 9: preparation of N-(adamantan-1-yl)-2-(((2-(2,6-dioxopiperidin-3-yl)-1-oxoisoindolin-4-yl)thio)methyl)benzo[b]thiophene-5-carboxamide (SIAIS338091)

With reference to the method of example 8, the target compound SIAIS338091 was prepared under appropriate conditions that will be recognized by one skilled in the art, except that the 4-(chloromethyl)thiazole-2-carboxylic acid in step 1 was replaced with ethyl 2-(bromomethyl)benzo[b]thiophene-5-carboxylate, and 1-adamantanol in step 2 was replaced with 1-adamantanamine.

The target compound SIAIS338091 was obtained as a white solid (6.2 mg, yield 36%). ¹H NMR (500 MHz, MeOD) *δ* 7.99 (s, 1H), 7.81 (d, *J* = 8.4 Hz, 1H), 7.72 (d, *J* = 7.7 Hz, 1H), 7.69 (d, *J* = 7.4 Hz, 1H), 7.63 (d, *J* = 8.4 Hz, 1H), 7.49 (t, *J* = 7.5 Hz, 1H), 7.07 (s, 1H), 5.05 (dd, *J* = 13.3, 5.1 Hz, 1H), 4.49 (s, 2H), 4.30 (d, *J* = 17.1 Hz, 1H), 4.16 (d, *J* = 17.3 Hz, 1H), 2.86 - 2.78 (m, 1H), 2.72 - 2.66 (m, 1H), 2.25 - 2.19 (m, 1H), 2.18 - 2.14 (m, 6H), 2.12 - 2.08 (m, 3H), 1.90 - 1.84 (m, 1H), 1.78 - 1.74 (m, 6H). MS *m*/*z*: 600.6 [M+H]⁺.

### Example 10: preparation of 4-(((2-(2,6-dioxopiperidin-3-yl)-1-oxoisoindolin-4-yl)thio)methyl)-N-(octahydro-2,5-methanopentalen-6a-yl)thiazole-2-carboxamide (SIAIS355073)

With reference to the method of example 8, the target compound SIAIS355073 was prepared under appropriate conditions that will be recognized by one skilled in the art, except that the 1-adamantanol in step 2 was replaced with noradamantanamine.

The target compound SIAIS355073 was obtained as a white solid (18 mg, yield 37%). ¹H NMR (500 MHz, DMSO) *δ* 10.99 (s, 1H), 8.21 (s, 1H), 7.71 (d, *J =* 10.1 Hz, 2H), 7.62 (d, *J =* 7.3 Hz, 1H), 7.53 (t, *J =* 7.4 Hz, 1H), 5.12 (dd, *J* = 13.0, 4.5 Hz, 1H), 4.45 (s, 2H), 4.27 - 4.15 (m, 2H), 2.92 (t, *J* = 13.2 Hz, 1H), 2.92 (t, *J* = 13.2 Hz, 1H), 2.65 - 2.55 (m, 1H), 2.45 - 2.34 (m, 2H), 2.24 (s, 2H), 2.09 (d, *J* = 9.7 Hz, 2H), 1.93 (d, *J* = 10.5 Hz, 5H), 1.62 - 1.46 (m, 4H). HRMS: calcd for C₂₇H₂₉N₄O₄S₂⁺ [M+H]⁺ 537.1630, found, 537.1631.

### Example 11: preparation of hydrochloride of 4-(((5-((adamantan-1-ylamino)methyl)pyridin-2-yl)methyl)amino)-2-(2,6-dioxopiperidin-3-yl)isoindoline-1,3-dione (SIAIS355067)

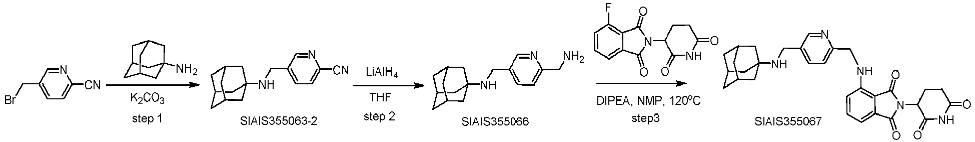

With reference to the method of Scheme 5, the target compound SIAIS355067 was prepared under appropriate conditions that will be recognized by one skilled in the art.

Specifically, step 1: to a mixture of 5-(bromomethyl)picolinonitrile and 1-adamantanamine (0.68 g, 1 eq) in acetonitrile (10mL) was added potassium carbonate (0.93 g, 1.5 eq). The mixture was reacted at room temperature for 2h, extracted with ethyl acetate, and washed with brine to give compound SIAIS355063-2 (brown solid, 0.53 g). MS m/z: 268 [M+H]⁺.

Step 2: to a mixture of compound SIAIS355063-2 (4.5 mmol) in THF (10 ml) was slowly added 2.5 N LiAlH₄ solution under ice bath. The mixture was reacted under ice bath for 1 h, and then at room temperature for 2 h. The mixture was diluted with ethyl acetate under ice bath, followed by addition dropwise 3 N NaOH (2 mL). The mixture was filtered, and the filtrate was concentrated to give compound SIAIS355066, which was directly used in the next step.

Step 3: To a mixture of 2-(2,6-dioxopiperidin-3-yl)-4-fluoroisoindoline-1,3-dione (35 mg, 0.126 mmol) in NMP (2 mL) were added overdose of product from step 2 and DIPEA (32 µL, 1.5 eq). The mixture was reacted in microwave at 120 °C for 2 h, and then filtered. The filtrate was subjected to preparative HPLC (eluent (v/v): acetonitrile/(water +0.05% HCl) = 10% -100%) for separation. The collected fractions were rotary evaporated to remove acetonitrile, and lyophilized to give the hydrochloride of the desired target compound (white solid, 12 mg, yield 18%). ¹H NMR (500 MHz, DMSO) *δ* 11.12 (s, 1H), 9.35 (s, 2H), 8.85 (s, 1H), 8.24 (d, *J =* 7.7 Hz, 1H), 7.61 (d, *J =* 8.0 Hz, 1H), 7.55 (t, *J* = 7.7 Hz, 1H), 7.47 (s, 1H), 7.07 (d, *J* = 6.8 Hz, 1H), 7.02 (d, *J* = 8.5 Hz, 1H), 5.09 (dd, *J* = 12.2, 4.5 Hz, 1H), 4.77 (s, 2H), 4.19 (s, 2H), 2.90 (t, *J* = 12.8 Hz, 1H), 2.65 - 2.55 (m, 2H), 2.15 (s, 3H), 2.10 - 2.03 (m, 1H), 1.99 (s, 6H), 1.69 (d, *J =* 11.6 Hz, 3H), 1.61 (d, *J =* 11.7 Hz, 3H). HRMS: calcd for C₃₀H₃₄N₅O₄⁺ [M+H]⁺ 528.2611, found, 528.2603.

### Biological activity assay

Materials:
Halt proteosome and phosphatase inhibitors (Thermo Fisher)
Cell TITER BLUE detection kits (Promega Corporation)
Cell TITER GLO detection kits (Promega Corporation)
CCK8 (WST) reagent (DOJINDO LABORATORIES, Japan)
RPMI1640 (GIBICO Company)
Fetal bovine serum (FBS) (GIBICO Company)
Penicillin-Streptomycin (GIBICO Company)
SuperSignal West Pico Chemiluminescent Substrate (Thermo Fisher)
SuperSignal West Femto Maximum Sensitivity Substrate (Thermo Fisher)
Cycloheximide (Sigma)

### Antibodies:

Most antibodies were purchased from Cell Signaling Technology, including IKZF1 (#9034S), IKZF3(#15103S). GAPDH antibody was purchased from Abcam Company.

### Cell culture

The tested cell line used were: Multiple myeloma cell line MM.1S, Mino (human mantle cell lymphoma cell), SR (anaplastic large cell lymphoma cell), PBMC (peripheral blood mononuclear cell), which were purchased from ATCC (American type Culture Collection). The medium was RPMI1640 supplemented with 10% FBS (fetal calf serum) and 1% Penicillin-Streptomycin. The cells used were identified as correct cells by STR cells, and were negative for mycoplasma through routine inspections.

### Determination of half inhibitory concentration (IC₅₀) of the compounds of the present disclosure

IC₅₀ values of the compounds of the present disclosure (including examples 1-11 compounds) were measured using Cell Titer Blue, Cell Titer GLO, or WST reagent from Promega Company. Assay details are as follows: Cells were seeded in 100 µL RPMI1640 complete medium containing serum at a density of 15,000 cells/well. After 24h, the inoculated cells were treated with diluted commercial inhibitor and the compounds of the present disclosure to be tested after serial dilution. After the cells were treated with the compounds of the present disclosure to be tested (including examples 1-11 compounds) for 72h, cell viability was determined after adding the cell viability detection kit listed above according to the reagent operating instructions. The negative control was DMSO, and the positive control was a commercial inhibitor, both of which were used to treat the cells through the same method as that of the compounds of the present disclosure. The growth inhibition of the compounds of the present disclosure on cells was plotted by Prism Graphpad software, and the IC₅₀ values of the compounds of the present disclosure were calculated therefrom. Results were shown in Tables 2 and 3.

### Western Blotting assay

Tumor cells were plated in a 24-well plate at a cell seeding density of 3×10⁵ cells/mL, with 1mL culture media per well. After 24h, the cells were treated with different concentrations of the compounds of the present disclosure. After 16 hours, the cells were collected, and washed with PBS. The supernatant was discarded, and the cells were placed on ice, and treated with RIPA protein lysate containing Halt protease and phosphatase inhibitor. The lysate was centrifuged at 10000RPM at 4°C for 10 minutes, and the supernatant was collected. An equal amount of proteins were loaded in 4X SDS sample solution, denatured at 95°C for 5 minutes, and then freezed to -20°C or directly subjected to protein electrophoresis (4-15% gradient gel, Bio-rad). Electrophoresis apparatus and related components were purchased from Bio-rad company, and electrophoresis set at a constant pressure of 120V for 1h. Then transferring membrane was conducted by using PVDF (polyvinylidene fluoride) at 400mA for 1h on ice. Afterwards, the membranes were block for 30 minutes by using the TarKara Blocking Buffer at room temperature. Western blotting was conducted according to the antibody product manual of Cell Signaling Technology Company. Results were shown in Figure 1.

DC₅₀ value (the drug concentration required for degrading proteins by 50%, abbreviated as DC₅₀) reads method: comparing the gray values of the Western blotting bands for the drug treatment with the gray values of the Western blotting band for the DMSO control, and reading the drug concentration range corresponding to the gray value of the Western blotting bands for the drug treatment which is equal to half of the gray value of the Western blotting band for the DMSO control.

DC₅₀ value could also be calculated as follows: using software ImageJ to quantify the gray values of the Western blotting bands for the drug treatment, fitting the relationship curve between drug concentrations and gray values, and from the fitted curve, calculating the drug concentration corresponding to half of the gray value of the Western blotting band for the DMSO control.

### Evaluation of TNF-α Inhibition Effect

PBMC cells were cultured at 37° C in 5% CO₂ atmosphere, and then seeded in 96-well plates at 1×10⁶ cells/well. Compounds (including compounds of Table 1 and Examples 1-11 compounds) were dissolved in DMSO and diluted to corresponding concentrations so that the final concentration of DMSO added to the cell culture did not exceed 0.5%. Cells were pretreated with compounds and vehicle control (DMSO) for 1 h, then stimulated with lipopolysaccharide (LPS; 1 µg/ml), and continually cultured for 18-20 h. Then, the supernatant was collected, and tested for TNF-α level by ELISA kit. IC₅₀ was then calculated by Graphpad Prism 7.0.

### Experimental result

The Compounds of the present invention in our research were based on the immunomodulatory drugs. We studied the cell inhibition and protein degradation activities of the compounds of the present invention (including the compounds in Table 1 and Examples 1-11 compounds) designed on the basis of immunomodulatory drugs including pomalidomide and lenalidomide. It was found that the compounds of the present invention (including the compounds in Table 1 and Examples 1-11 compounds) designed on the basis of heteroatom-substituted lenalidomide derivatives were more active against multiple myeloma cancer cell MM.1S than lenalidomide. The compounds of the present invention (including the compounds in Table 1 and Examples 1-11 compounds) could not only inhibit cancer cell proliferation, but also promote the degradation of IKZF, and thus can be developed as a therapeutic drug for immune-related tumor patients. Detailed experiment results were shown below.

### 1.1 Proliferation inhibition of the compounds of the present invention (including the compounds in Table 1 and Examples 1-11 compounds) based on pomalidomide and lenalidomide derivatives in tumor cells

We tested the activities of the compounds of the present invention (including the compounds in Table 1 and Examples 1-11 compounds) in MM.1S cell, mantle cell lymphoma Mino cells, and anaplastic large cell lymphoma SR cells through a dose-dependent manner. These cells overexpressed IKZF1/3 and was not sensitive to existing immunomodulatory drugs. The cells were treated with the compounds of the present invention at 10 different successively decreasing concentrations (starting at the highest concentration of 10µM; 5-fold serial dilutions) for 72h, and then the cell viability determination was performed in accordance with CCK-8 reagent operating instructions. The experiment was repeated more than three times. Results were shown in Tables 2 and 3.

**Table 2. IC₅₀ values (half inhibitory concentration) of the compounds of the present invention in tumor cells**

| Compounds | MM.1S/ IC₅₀(nM) | Compounds | MM.1S/ IC₅₀(nM) |
|---|---|---|---|
| Lenalidomide | 19.59±3.30 | Pomalidomide | 9.38 ± 1.33 |
| SIAIS313118 | 0.09±0.02 | SIAIS313185 | 0.16±0.02 |
| SIAIS338091 | 0.02±0.00 | SIAIS287116 | 0.003 |
| SIAIS313160 | 0.13±0.03 | SIAIS287129 | 0.181 |
| SIAIS355073 | 0.51±0.25 | | |
| SIAIS355067 | 4.46±0.18 | SIAIS338048 | 0.06±0.04 |

**Table 3. IC₅₀ values (half inhibitory concentration) of the compounds of the present invention in lymphoma cells**

| **Cell line** | **Compounds** | **Reagent** | **IC₅₀ (nM)** |
|---|---|---|---|
| SR | Lenalidomide | WST | NA |
| SR | SIAIS287116 | WST | 0.454 |
| SR | SIAIS287129 | WST | 4.017 |
| Mino | Pomalidomide | WST | 48.02 |
| Mino | SIAIS287116 | WST | 0.387 |
| Mino | SIAIS287129 | WST | 2.59 |

| | | | |
|---|---|---|---|
| note: NA stands for IC₅₀ values not detected | | | |

The compounds of the present invention (including the compounds in Table 1 and Examples 1-11 compounds) developed based on lenalidomide that we designed and developed can significantly inhibit the proliferation of multiple myeloma cells (Table 2). The inhibitory effect of lenalidomide on SR cells was less than 50%. Compared with lenalidomide, the compounds of the present invention (including the compounds in Table 1 and Examples 1-11 compounds) exhibited a stronger inhibitory effect, such as the compound (SIAIS287116) which had IC₅₀ value of about 0.5 nM in SR cells. The IC₅₀ of pomalidomide in Mino cells was 48 nM, while most compounds of the present invention had a half inhibitory dose significantly lower than that of the commercial inhibitor, such as the half inhibition concentration of the compound (SIAIS287116) on cells is around 0.39 nM, which is 123-fold lower than that of pomalidomide.

### 1.2 Study on the IKZF degradation of the compounds of the present invention (half inhibitory concentration, DC₅₀)

We selected and tested part of the compounds of the present invention (including the compounds in Table 1 and Examples 1-11 compounds) in MM.1S cell through western blotting assay in a dose-dependent manner. The studies have shown that these compounds can significantly degrade IKZF1/3 at effective concentrations as low to picomolar levels. As shown in Figure 1, the compound SIAIS287116 can also significantly degrade IZKF3 protein at a concentration of less than 0.01 nM.

### 1.3 TNF-α Inhibition Effect of the compounds of the present invention

The results of the TNF-α activity inhibition experiment were shown in Table 4. The results showed that, compared with lenalidomide, the compounds of the present invention (including the compounds of Table 1 and Examples 1-11 compounds) could significantly inhibit the release of TNF-α stimulated by lipopolysaccharide (LPS).

**Table 4. IC₅₀ (half inhibitory concentration) of the compounds of the present invention on TNF-α in PMBC cells**

| Compound No. | IC₅₀ on TNF-α (nM) |
|---|---|
| Lenalidomide | 10-100 |
| SIAIS313160 | <1 |
| SIAIS287097 | <5 |
| SIAIS287116 | <1 |
| SIAIS338091 | <1 |
| SIAIS355067 | <5 |

The basic principles, main features and advantages of the present invention have been shown and described above. Those skilled in the art should understand that the present invention is not limited by the above-mentioned embodiments, and without departing from the spirit and scope of the present invention, the present invention can also undergo various changes and improvements, and these changes and improvements all fall within the scope of the present invention. The claimed scope of the present invention is defined by the appended claims and their equivalents.

## Claims

1. A compound of Formula (I) 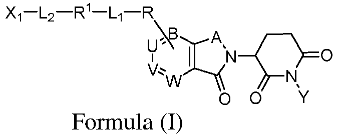 or a salt, an enantiomer, a stereoisomer, a solvate, or a polymorph thereof, wherein
Y represents H or C₁₋₃ alkyl;
A represents CH₂ or C(O);
B, U, V, W are the same or different and each independently represent CH or N, wherein B, U, V and W are not simultaneously N, and when any of B, U, V, and W is attached to R, it is not N;
R represents O, S, N(R²), CH₂, alkenylene or alkynylene, wherein R² represents H or C₁₋₃ alkyl, or R represents a bond;
R¹ represents optionally substituted heterocyclylene or optionally substituted heteroarylene, wherein said heterocyclylene and said heteroarylene each independently optionally substituted with a substituent(s) selected from the group consisting of C₁₋₃ alkyl, C₁₋₃ alkoxy, C₃₋₆ cycloalkyl, halogenated C₁₋₃ alkyl, halogen, hydroxy, cyano, amino, or any combination thereof;
L₁ represents Formula -L₃-L₄-^{∗}, where symbol * indicates the point of attachment of L₄ to R,
L₃ represents O, S, S(O)₂O, OS(O)₂, S(O)₂N(R³), N(R³)S(O)₂, C(O)O, OC(O), N(R³), C(O)N(R³), or N(R³)C(O), wherein R³ represents H or C₁₋₃ alkyl, or
L₃ represents a bond; and
L₄ represents optionally substituted methylene or optionally substituted linear or branched C₂₋₄₀ alkylene, wherein the linear or branched C₂₋₄₀ alkylene is optionally interrupted one or more times by a group selected from the group consisting of: optionally substituted heterocyclylene, optionally substituted heteroarylene, O, S, S(O)₂O, OS(O)₂, S(O)₂N(R³), N(R³)S(O)₂, C(O)O, OC(O), N(R³), C(O)N(R³), N(R³)C(O) or any combination thereof, wherein R³ represents H or C₁₋₃ alkyl, wherein the methylene, hydrogen(s) of one or more CH₂ of backbone of said linear or branched C₂₋₄₀ alkylene, the heterocyclylene and the heteroarylene are each independently optionally substituted by a substituent(s) selected from the group consisting of C₁₋₃ alkyl, C₁₋₃ alkoxy, C₃₋₆ cycloalkyl, halogenated C₁₋₃ alkyl, halogen, hydroxy, cyano, amino or any combination thereof, or
L₄ represents a bond, provided that R, L₃ and L₄ do not simultaneously represent a bond, and when L₄ represents a bond, either R or L₃ represents a bond; and
L₂ represents Formula -L₅-L₆-^{∗∗}, where symbol ** indicates the point of attachment of L₆ to R¹,
L₆ represents O, S, S(O)₂O, OS(O)₂, S(O)₂N(R³), N(R³)S(O)₂, C(O)O, OC(O), N(R³), C(O)N(R³), or N(R³)C(O), wherein R³ represents H or C₁₋₃ alkyl, or
L₆ represents a bond; and
L₅ represents optionally substituted methylene or optionally substituted linear or branched C₂₋₄₀ alkylene, wherein the linear or branched C₂₋₄₀ alkylene is optionally interrupted one or more times by a group selected from the group consisting of: optionally substituted heterocyclylene, optionally substituted heteroarylene, O, S, S(O)₂O, OS(O)₂, S(O)₂N(R³), N(R³)S(O)₂, C(O)O, OC(O), N(R³), C(O)N(R³), N(R³)C(O) or any combination thereof, wherein R³ represents H or C₁₋₃ alkyl, wherein the methylene, hydrogen(s) of one or more CH₂ of backbone of said linear or branched C₂₋₄₀ alkylene, the heterocyclylene and the heteroarylene are each independently optionally substituted by a substituent(s) selected from the group consisting of C₁₋₃ alkyl, C₁₋₃ alkoxy, C₃₋₆ cycloalkyl, halogenated C₁₋₃ alkyl, halogen, hydroxy, cyano, amino or any combination thereof, or
L₅ represents a bond; and
X₁ represents Formula X_{b}-Xₐ-, wherein
Xₐ represents -(CH₂)₀₋₂₀-O-^{#}, -(CH₂)₀₋₂₀-S-^{#}, -(CH₂)₀₋₂₀-C(O)O-^{#}, -(CH₂)₀₋₂₀-OC(O)-^{#}, - (CH₂)₀₋₂₀-N(R³)-^{#}, -(CH₂)₀₋₂₀-C(O)N(R³)-^{#}, -(CH₂)₀₋₂₀-N(R³)C(O)-^{#}, -(CH₂)₀₋₂₀-S(O)₂N(R³)-^{#}, - (CH₂)₀₋₂₀-N(R³)S(O)₂-^{#}, -(CH₂)₀₋₂₀-S(O)₂O-^{#}, -(CH₂)₀₋₂₀-OS(O)₂-^{#}, -(CH₂)₀₋₂₀-S(O)₂-^{#}, -(CH₂)₀₋₂₀-S(O)₂-^{#}, alkynylene, or alkenylene, wherein R³ represents H or C₁₋₃ alkyl (e.g., methyl, ethyl or propyl), and symbol # indicates the point of attachment to L₂, or
Xₐ represents a bond, provided that when L₅ represents a bond, either Xₐ or L₆ represents a bond; and
X_{b} represents optionally substituted adamantanyl, optionally substituted noradamantanyl, optionally substituted meta-menthanyl, optionally substituted p-menthanyl, optionally substituted quinuclidinyl, optionally substituted bicyclo[2.2.1]heptyl or optionally substituted bicyclo[2.2.1]heptenyl, wherein said adamantanyl, said noradamantanyl, said meta-menthanyl, said p-menthanyl, said quinuclidinyl, said bicyclo[2.2.1]heptyl, and said bicyclo[2.2.1]heptenyl are each independently optionally substituted by a substituent(s) selected from the group consisting of C₁₋₃ alkyl, halogenated C₁₋₃ alkyl, C₁₋₃ alkoxy, C₁₋₃ alkyl-NH-, amino-C₁₋₃ alkylene, amino, oxo, halogen, hydroxy, cyano, C₁₋₃ alkyl- NHC(O)-, mercapto, or any combination thereof.

2. The compound of Formula (I) or a salt, an enantiomer, a stereoisomer, a solvate, or a polymorph thereof as claimed in claim 1, wherein the compound of formula (I) is also a compound of Formula (Ia): 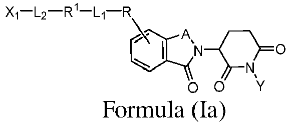 wherein A, R, R¹, L₁, L₂, X₁ and Y are as defined in claim 1.

3. The compound of Formula (I) or a salt, an enantiomer, a stereoisomer, a solvate, or a polymorph thereof as claimed in claim 1, wherein the compound of formula (I) is also a compound of Formula (Ib): 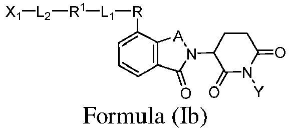 wherein A, R, R¹, L₁, L₂, X₁ and Y are as defined in claim 1.

4. The compound of Formula (I) or a salt, an enantiomer, a stereoisomer, a solvate, or a polymorph thereof as claimed in claim 1, wherein the compound of formula (I) is also a compound of Formula (Ic): 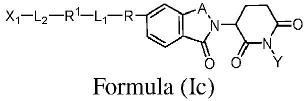 wherein A, R, R¹, L₁, L₂, X₁ and Y are as defined in claim 1.

5. The compound of Formula (I) or a salt, an enantiomer, a stereoisomer, a solvate, or a polymorph thereof as claimed in claim 1, wherein the compound of formula (I) is also a compound of Formula (Id): 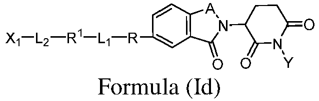 wherein A, R, R¹, L₁, L₂, X₁ and Y are as defined in claim 1.

6. The compound of Formula (I) or a salt, an enantiomer, a stereoisomer, a solvate, or a polymorph thereof as claimed in claim 1, wherein the compound of formula (I) is also a compound of Formula (Ie): 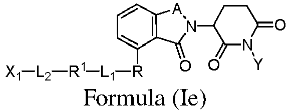 wherein A, R, R¹, L₁, L₂, X₁ and Y are as defined in claim 1.

7. The compound of Formula (I) or a salt, an enantiomer, a stereoisomer, a solvate, or a polymorph thereof as claimed in claim 1, wherein R represents O, S, N(R²), CH₂, alkenylene or alkynylene, where R² represents H or C₁₋₃ alkyl.

8. The compound of Formula (I) or a salt, an enantiomer, a stereoisomer, a solvate, or a polymorph thereof as claimed in claim 7, wherein R¹ represents the following groups optionally further substituted with a substituent(s) selected from the group consisting of C₁₋₃ alkyl, C₁₋₃ alkoxy, C₃₋₆ cycloalkyl, halogenated C₁₋₃ alkyl, halogen, hydroxy, cyano, amino, or any combination thereof:
furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzoisoxazolylene, benzothiazolylene, benzoisothiazolylene, benzotriazolylene, benzo[2,1,3]oxadiazolylene, benzo[2,1,3]thiadiazolylene, benzo[1,2,3]thiadiazolylene, quinolylene, isoquinolylene, naphthyridinylene, cinnolinylene, quinazolinylene, quinoxalinylene, phthalazinylene, pyrazolo[1,5-a]pyridinylene, pyrazolo[1,5-a]pyrimidinylene, imidazo[1,2-a]pyridinylene, 1H-pyrrolo[3,2-b]pyridinylene, 1H-pyrrolo[2,3-b]pyridinylene, 4H-fluoro[3,2-b]pyrrolylene, pyrrolo[2,1-b]thiazolylene, imidazo[2,1-b]thiazolylene, azetidinylene, oxetanylene, pyrrolidinylene, imidazolidylene, pyrazolidylene, triazolylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxanylene, or diazepanylene.

9. The compound of Formula (I) or a salt, an enantiomer, a stereoisomer, a solvate, or a polymorph thereof as claimed in any one of claims 1-8, wherein L₁ represents Formula -L₃-L₄-^{∗},
wherein symbol * indicates the point of attachment of L₄ to R,
L₃ represents a bond; and
L₄ represents optionally substituted methylene or optionally substituted linear or branched C₂₋₄₀ alkylene, wherein the linear or branched C₂₋₄₀ alkylene is optionally interrupted one or more times by a group selected from the group consisting of: optionally substituted heterocyclylene, optionally substituted heteroarylene, O, S, S(O)₂O, OS(O)₂, S(O)₂N(R³), N(R³)S(O)₂, C(O)O, OC(O), N(R³), C(O)N(R³), N(R³)C(O) or any combination thereof, wherein R³ represents H or C₁₋₃ alkyl, wherein the methylene, hydrogen(s) of one or more CH₂ of backbone of said linear or branched C₂₋₄₀ alkylene, the heterocyclylene and the heteroarylene are each independently optionally substituted by a substituent(s) selected from the group consisting of C₁₋₃ alkyl, C₁₋₃ alkoxy, C₃₋₆ cycloalkyl, halogenated C₁₋₃ alkyl, halogen, hydroxy, cyano, amino or any combination thereof.

10. The compound of Formula (I) or a salt, an enantiomer, a stereoisomer, a solvate, or a polymorph thereof as claimed in claim 9, wherein L₁ represents Formula -L₃-L₄-^{∗}, wherein symbol * indicates the point of attachment of L₄ to R, L₃ represents a bond; and L₄ represents linear or branched C₁₋₄₀ alkylene optionally substituted by a substituent(s) selected from the group consisting of C₁₋₃ alkyl, C₁₋₃ alkoxy, C₃₋₆ cycloalkyl, halogenated C₁₋₃ alkyl, halogen, hydroxyl, cyano, amino or any combination thereof.

11. The compound of Formula (I) or a salt, an enantiomer, a stereoisomer, a solvate, or a polymorph thereof as claimed in claim 10, wherein L₄ represents following groups: -CH₂-, -(CH₂)₂-, -(CH₂)₃-, - (CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₁₂-, -(CH₂)₁₃-, - (CH₂)₁₄-, -(CH₂)₁₅-, -(CH₂)₁₆-, -(CH₂)₁₇-, -(CH₂)₁₈-, -(CH₂)₁₉-, or -(CH₂)₂₀-; wherein hydrogen(s) of one or more CH₂ of the groups is optionally further replaced with a substituent(s) selected from the group consisting of C₁₋₃ alkyl, C₁₋₃ alkoxy, C₃₋₆ cycloalkyl, halogenated C₁₋₃ alkyl, halogen, hydroxy, cyano, amino, or any combination thereof.

12. The compound of Formula (I) or a salt, an enantiomer, a stereoisomer, a solvate, or a polymorph thereof as claimed in any one of claims 1-8, wherein L₂ represents Formula -L₅-L₆-^{∗∗}, wherein symbol ** indicates the point of attachment of L₆ to R¹,
L₆ represents a bond; and
L₅ represents optionally substituted methylene or optionally substituted linear or branched C₂₋₄₀ alkylene, wherein the linear or branched C₂₋₄₀ alkylene is optionally interrupted one or more times by a group selected from the group consisting of: optionally substituted heterocyclylene, optionally substituted heteroarylene, O, S, S(O)₂O, OS(O)₂, S(O)₂N(R³), N(R³)S(O)₂, C(O)O, OC(O), N(R³), C(O)N(R³), N(R³)C(O) or any combination thereof, wherein R³ represents H or C₁₋₃ alkyl, wherein the methylene, hydrogen(s) of one or more CH₂ of backbone of said linear or branched C₂₋₄₀ alkylene, the heterocyclylene and the heteroarylene are each independently optionally substituted by a substituent(s) selected from the group consisting of C₁₋₃ alkyl, C₁₋₃ alkoxy, C₃₋₆ cycloalkyl, halogenated C₁₋₃ alkyl, halogen, hydroxy, cyano, amino, or any combination thereof.

13. The compound of Formula (I) or a salt, an enantiomer, a stereoisomer, a solvate, or a polymorph thereof as claimed in claim 9 or 12, wherein
the heteroarylene is the following groups optionally further substituted with a substituent(s) selected from the group consisting of C₁₋₃ alkyl, C₁₋₃ alkoxy, C₃₋₆ cycloalkyl, halogenated C₁₋₃ alkyl, halogen, hydroxy, cyano, amino, or any combination thereof:
furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzoisoxazolylene, benzothiazolylene, benzoisothiazolylene, benzotriazolylene, benzo[2,1,3]oxadiazolylene, benzo[2,1,3]thiadiazolylene, benzo[1,2,3]thiadiazolylene, quinolylene, isoquinolylene, naphthyridinylene, cinnolinylene, quinazolinylene, quinoxalinylene, phthalazinylene, pyrazolo[1,5-a]pyridinylene, pyrazolo[1,5-a]pyrimidinylene, imidazo[1,2-a]pyridinylene, 1H-pyrrolo[3,2-b]pyridinylene, 1H-pyrrolo[2,3-b]pyridinylene, 4H-fluoro[3,2-b]pyrrolylene, pyrrolo[2,1-b]thiazolylene or imidazo[2,1-b]thiazolylene; or
the heterocyclylene is the following groups optionally further substituted by a substituent(s) selected from the group consisting of C₁₋₃ alkyl, C₁₋₃ alkoxy, C₃₋₆ cycloalkyl, halogenated C₁₋₃ alkyl, halogen, hydroxy, cyano, amino, or any combination thereof:
azetidinylene, oxetanylene, pyrrolidinylene, imidazolidylene, pyrazolidylene, triazolylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxanylene, or diazepanylene.

14. The compound of Formula (I) or a salt, an enantiomer, a stereoisomer, a solvate, or a polymorph thereof as claimed in any one of claims 1-8, wherein L₂ represents Formula -L₅-L₆-^{∗∗}, wherein symbol ** indicates the point of attachment of L₆ to R¹, and both L₅ and L₆ represent a bond.

15. The compound of Formula (I) or a salt, an enantiomer, a stereoisomer, a solvate, or a polymorph thereof as claimed in claim 12, wherein L₂ represents Formula -L₅-L₆-^{∗∗}, wherein symbol ** indicates the point of attachment of L₆ to R¹,
L₆ represents a bond; and
L₅ represents linear or branched C₁₋₄₀ alkylene optionally substituted by a substituent(s) selected from the group consisting of C₁₋₃ alkyl, C₁₋₃ alkoxy, C₃₋₆ cycloalkyl, halogenated C₁₋₃ alkyl, halogen, hydroxy, cyano, amino or any combination thereof.

16. The compound of Formula (I) or a salt, an enantiomer, a stereoisomer, a solvate, or a polymorph thereof as claimed in claim 15, wherein L₅ represents the following groups: -CH₂-, -(CH₂)₂-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₁₂-, -(CH₂)₁₃-, - (CH₂)₁₄-, -(CH₂)₁₅-, -(CH₂)₁₆-, -(CH₂)₁₇-, -(CH₂)₁₈-, -(CH₂)₁₉-, or -(CH₂)₂₀-; wherein hydrogen(s) of one or more CH₂ of the group is optionally further replaced by a substituent(s) selected from the group consisting of C₁₋₃ alkyl, C₁₋₃ alkoxy, C₃₋₆ cycloalkyl, halogenated C₁₋₃ alkyl, halogen, hydroxy, cyano, amino, or any combination thereof.

17. The compound of Formula (I) or a salt, an enantiomer, a stereoisomer, a solvate, or a polymorph thereof as claimed in claim 12, wherein L₅ represents a group having the following general formula: - (CH₂)ₙ₁-N(R³)-(CH₂)ₙ₂-^{∗∗∗}, -(CH₂)ₙ₁-C(O)O-(CH₂)ₙ₂-^{∗∗∗}, -(CH₂)ₙ₁-OC(O)-(CH₂)ₙ₂-^{∗∗∗}, -(CH₂)ₙ₁-C(O)N(R³)-(CH₂)ₙ₂-^{∗∗∗}, -(CH₂)ₙ₁-N(R³)C(O)-(CH₂)ₙ₂-^{∗∗∗}, -(CH₂)ₙ₁-S(O)₂O-(CH₂)ₙ₂-^{∗∗∗}, -(CH₂)ₙ₁-OS(O)₂-(CH₂)ₙ₂-^{∗∗∗}, -(CH₂)ₙ₁-S(O)₂N(R³)-(CH₂)ₙ₂-^{∗∗∗}, or -(CH₂)ₙ₁-N(R³)S(O)₂-(CH₂)ₙ₂-^{∗∗∗},
wherein symbol *** indicates the point of attachment of L₅ to L₆, R³ represents H or C₁₋₃ alkyl, and hydrogen(s) of one or more CH₂ of said group is optionally further replaced by a substituent(s) selected from the group consisting of C₁₋₃ alkyl, C₁₋₃ alkoxy, C₃₋₆ cycloalkyl, halogenated C₁₋₃ alkyl, halogen, hydroxy, cyano, amino, or any combination thereof, and
n1 and n2 each independently represent an integer of 1, 2, 3, 4, 5, 6, 7, 8, 9, 10, 11, 12, 13, 14, or 15.

18. The compound of Formula (I) or a salt, an enantiomer, a stereoisomer, a solvate, or a polymorph thereof as claimed in claim 17, wherein L₅ represents the following groups: -CH(CH₃)C(O)OCH₂-^{∗∗∗}, -CH₂CH(CH₃)C(O)OCH₂-^{∗∗∗}, -(CH₂)₂CH(CH₃)C(O)OCH₂-^{∗∗∗}, -(CH₂)₃CH(CH₃)C(O)OCH₂-^{∗∗∗}, - (CH₂)₄CH(CH₃)C(O)OCH₂-^{∗∗∗}, -(CH₂)₅CH(CH₃)C(O)OCH₂-^{∗∗∗}, -(CH₂)₆CH(CH₃)C(O)OCH₂-^{∗∗∗}, - (CH₂)₇CH(CH₃)C(O)OCH₂-^{∗∗∗}, -CH(CH₃)OC(O)CH₂-^{∗∗∗}, -CH₂CH(CH₃)OC(O)CH₂-^{∗∗∗}, - (CH₂)₂CH(CH₃)OC(O)CH₂-^{∗∗∗}, -(CH₂)₃CH(CH₃)OC(O)CH₂-^{∗∗∗}, -(CH₂)₄CH(CH₃)OC(O)CH₂-^{∗∗∗}, - (CH₂)₅CH(CH₃)OC(O)CH₂-^{∗∗∗}, -(CH₂)₆CH(CH₃)OC(O)CH₂-^{∗∗∗}, -(CH₂)₇CH(CH₃)OC(O)CH₂-^{∗∗∗}, - (CH₂)₈CH(CH₃)OC(O)CH₂-^{∗∗∗}, -CH(CH₃)C(O)NHCH₂-^{∗∗∗}, -CH₂CH(CH₃)C(O)NHCH₂-^{∗∗∗}, - (CH₂)₂CH(CH₃)C(O)NHCH₂-^{∗∗∗}, -(CH₂)₃CH(CH₃)C(O)NHCH₂-^{∗∗∗}, -(CH₂)₄CH(CH₃)C(O)NHCH₂-^{∗∗∗}, -(CH₂)₅CH(CH₃)C(O)NHCH₂-^{∗∗∗}, -(CH₂)₆CH(CH₃)C(O)NHCH₂-^{∗∗∗}, - (CH₂)₇CH(CH₃)C(O)NHCH₂-^{∗∗∗}, -(CH₂)₈CH(CH₃)C(O)NHCH₂-^{∗∗∗}, -(CH₂)₉CH(CH₃)C(O)NHCH₂-^{∗∗∗} or -(CH₂)₁₀CH(CH₃)C(O)NHCH₂-^{∗∗∗}, wherein hydrogen(s) of one or more CH₂ of said group is optionally further replaced by a substituent(s) selected from the group consisting of C₁₋₃ alkyl, C₁₋₃ alkoxy, C₃₋₆ cycloalkyl, halogenated C₁₋₃ alkyl, halogen, hydroxy, cyano, amino, or any combination thereof; and symbol *** indicates the point of attachment of L₅ to L₆.

19. The compound of Formula (I) or a salt, an enantiomer, a stereoisomer, a solvate, or a polymorph thereof as claimed in claim 1, wherein the L₂-R¹-L₁ moiety in Formula (I) represents the following groups:

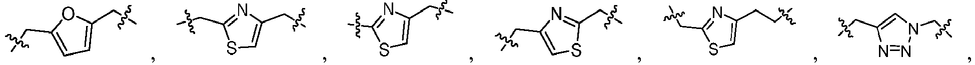

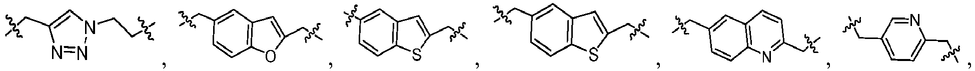

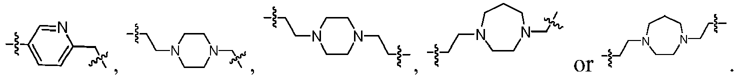

20. The compound of Formula (I) or a salt, an enantiomer, a stereoisomer, a solvate, or a polymorph thereof as claimed in claim 12, wherein
X₁ represents Formula X_{b}-Xₐ-, wherein
Xₐ represents -(CH₂)₀₋₁₅-O-^{#}, -(CH₂)₀₋₁₅-S-^{#}, -(CH₂)₀₋₁₅-C(O)O-^{#}, -(CH₂)₀₋₁₅-OC(O)-^{#}, - (CH₂)₀₋₁₅-N(R³)-^{#}, -(CH₂)₀₋₁₅-C(O)N(R³)-^{#}, -(CH₂)₀₋₁₅-N(R³)C(O)-^{#}, -(CH₂)₀₋₁₅-S(O)₂N(R3)-^{#}, - (CH₂)₀₋₁₅-N(R³)S(O)₂-^{#}, -(CH₂)₀₋₁₅-S(O)₂O-^{#}, -(CH₂)₀₋₁₅-OS(O)₂-^{#}, -(CH₂)₀₋₁₅-S(O)₂-^{#}, -(CH₂)₀₋₁₅-S(O)₂-^{#}, alkynylene or alkenylene, wherein R³ represents H or C₁₋₃ alkyl, and symbol # represents the point of attachment to L₂, or
Xₐ represents a bond, provided that Xₐ, L₅ and L₆ do not simultaneously represent a bond, and when L₅ represents a bond, either Xₐ or L₆ represents a bond; and
X_{b} represents optionally substituted adamantanyl, optionally substituted noradamantanyl, optionally substituted meta-menthanyl, optionally substituted p-menthanyl, optionally substituted quinuclidinyl, optionally substituted bicyclo[2.2.1]heptyl or optionally substituted bicyclo[2.2.1]heptenyl, wherein said adamantanyl, said noradamantanyl, said meta-menthanyl, said p-menthanyl, said quinuclidinyl, said bicyclo[2.2.1]heptyl, and said bicyclo[2.2.1]heptenyl are each independently optionally substituted by a substituent(s) selected from the group consisting of C₁₋₃ alkyl, halogenated C₁₋₃ alkyl, C₁₋₃ alkoxy, C₁₋₃ alkyl-NH-, amino-C₁₋₃ alkylene, amino, oxo, halogen, hydroxy, cyano, C₁₋₃ alkyl-NHC(O)-, mercapto, or any combination thereof.

21. The compound of Formula (I) or a salt, an enantiomer, a stereoisomer, a solvate, or a polymorph thereof as claimed in any one of claims 14-16 and 19-20, wherein
X₁ represents Formula X_{b}-Xₐ-, wherein
Xₐ represents -(CH₂)₀₋₁₅-O-^{#}, -(CH₂)₀₋₁₅-S-^{#}, -(CH₂)₀₋₁₅-C(O)O-^{#}, -(CH₂)₀₋₁₅-OC(O)-^{#}, - (CH₂)₀₋₁₅-N(R³)-^{#}, -(CH₂)₀₋₁₅-C(O)N(R³)-^{#}, -(CH₂)₀₋₁₅-N(R³)C(O)-^{#}, -(CH₂)₀₋₁₅-S(O)₂N(R³)-^{#}, - (CH₂)₀₋₁₅-N(R³)S(O)₂-^{#}, -(CH₂)₀₋₁₅-S(O)₂O-^{#}, -(CH₂)₀₋₁₅-OS(O)₂-^{#}, -(CH₂)₀₋₁₅-S(O)₂-^{#}, -(CH₂)₀₋₁₅-S(O)₂-^{#}, alkynylene or alkenylene, wherein R³ represents H or C₁₋₃ alkyl, and symbol # represents the point of attachment to L₂, and
X_{b} represents optionally substituted adamantanyl, optionally substituted noradamantanyl, optionally substituted meta-menthanyl, optionally substituted p-menthanyl, optionally substituted quinuclidinyl, optionally substituted bicyclo[2.2.1]heptyl or optionally substituted bicyclo[2.2.1]heptenyl, wherein said adamantanyl, said noradamantanyl, said meta-menthanyl, said p-menthanyl, said quinuclidinyl, said bicyclo[2.2.1]heptyl, and said bicyclo[2.2.1]heptenyl are each independently optionally substituted by a substituent(s) selected from the group consisting of C₁₋₃ alkyl, halogenated C₁₋₃ alkyl, C₁₋₃ alkoxy, C₁₋₃ alkyl-NH-, amino-C₁₋₃ alkylene, amino, oxo, halogen, hydroxy, cyano, C₁₋₃ alkyl-NHC(O)-, mercapto, or any combination thereof.

22. The compound of Formula (I) or a salt, an enantiomer, a stereoisomer, a solvate, or a polymorph thereof as claimed in claim 21, wherein Xₐ represents the following groups: O, -CH₂O-^{#}, -(CH₂)₂O-^{#}, - (CH₂)₃O-^{#}, -(CH₂)₄O-^{#}, -(CH₂)₅O-^{#}, -(CH₂)₆O-^{#}, -(CH₂)₇O-^{#}, -(CH₂)₈O-^{#}, -(CH₂)₉O-^{#}, -(CH₂)₁₀O-^{#}, S, - CH₂S-^{#}, -(CH₂)₂S-^{#}, -(CH₂)₃S-^{#}, -(CH₂)₄S-^{#}, -(CH₂)₅S-^{#}, -(CH₂)₆S-^{#}, -C(O)O-^{#}, -CH₂C(O)O-^{#}, - (CH₂)₃C(O)O-^{#}, -(CH₂)₄C(O)O-^{#}, -(CH₂)₅C(O)O-^{#}, -(CH₂)₆C(O)O-^{#}, -(CH₂)₇C(O)O-^{#}, -(CH₂)₈C(O)O-^{#}, -(CH₂)₉C(O)O-^{#}, -(CH₂)₁₀C(O)O-^{#}, -(CH₂)₂CH(CH₃)C(O)O-^{#}, -(CH₂)₃CH(CH₃)C(O)O-^{#}, - (CH₂)₄CH(CH₃)C(O)O-^{#}, -(CH₂)₅CH(CH₃)C(O)O-^{#}, -(CH₂)₆CH(CH₃)C(O)O-^{#}, - (CH₂)₇CH(CH₃)C(O)O-^{#}, -OC(O)-^{#}, -CH₂OC(O)-^{#}, -(CH₂)₂OC(O)-^{#}, -(CH₂)₃OC(O)-^{#}, -(CH₂)₄OC(O)-^{#}, -(CH₂)₅OC(O)-^{#}, -(CH₂)₆OC(O)-^{#}, -(CH₂)₇OC(O)-^{#}, -(CH₂)₈OC(O)-^{#}, -(CH₂)₉OC(O)-^{#}, - (CH₂)₁₀OC(O)-^{#}, -CH₂CH(CH₃)OC(O)-^{#}, -(CH₂)₂CH(CH₃)OC(O)-^{#}, -(CH₂)₃CH(CH₃)OC(O)-^{#}, - (CH₂)₄CH(CH₃)OC(O)-^{#}, -(CH₂)₅CH(CH₃)OC(O)-^{#}, -(CH₂)₆CH(CH₃)OC(O)-^{#}, - (CH₂)₇CH(CH₃)OC(O)-^{#}, -(CH₂)₈CH(CH₃)OC(O)-^{#}, NH, -CH₂NH-^{#}, -(CH₂)₂NH-^{#}, -(CH₂)₃NH-^{#}, - (CH₂)₄NH-^{#}, -(CH₂)₅NH-⁴, -(CH₂)₆NH-^{#}, -(CH₂)₇NH-^{#}, -(CH₂)₈NH-^{#}, -(CH₂)₉NH-^{#}, -(CH₂)₁₀NH-^{#}, N(CH₃), -CH₂N(CH₃)-^{#}, -(CH₂)₂N(CH₃)-^{#}, -(CH₂)₃N(CH₃)-^{#}, -(CH₂)₄N(CH₃)-^{#}, -(CH₂)₅N(CH₃)-^{#}, - (CH₂)₆N(CH₃)-^{#}, -(CH₂)₇N(CH₃)-^{#}, -(CH₂)₈N(CH₃)-^{#}, -(CH₂)₉N(CH₃)-^{#}, -(CH₂)₁₀N(CH₃)-^{#}, -C(O)NH-^{#}, -CH₂C(O)NH-^{#}, -(CH₂)₂C(O)NH-^{#}, -(CH₂)₃C(O)NH-^{#}, -(CH₂)₄C(O)NH-^{#}, -(CH₂)₅C(O)NH-^{#}, - (CH₂)₆C(O)NH-^{#}, -(CH₂)₇C(O)NH-^{#}, -(CH₂)₈C(O)NH-^{#}, -(CH₂)₉C(O)NH-^{#}, -(CH₂)₁₀C(O)NH-^{#}, - CH₂CH(CH₃)C(O)NH-^{#}, -(CH₂)₂CH(CH₃)C(O)NH-^{#}, -(CH₂)₃CH(CH₃)C(O)NH-^{#}, - (CH₂)₄CH(CH₃)C(O)NH-^{#}, -(CH₂)₅CH(CH₃)C(O)NH-^{#}, -(CH₂)₆CH(CH₃)C(O)NH-^{#}, - (CH₂)₇CH(CH₃)C(O)NH-^{#}, -(CH₂)₈CH(CH₃)C(O)NH-^{#}, -(CH₂)₉CH(CH₃)C(O)NH-^{#}, - (CH₂)₁₀CH(CH₃)C(O)NH-^{#}, -NHC(O)-^{#}, -CH₂NHC(O)-^{#}, -(CH₂)₂NHC(O)-^{#}, -(CH₂)₃NHC(O)-^{#}, - (CH₂)₄NHC(O)-^{#}, -(CH₂)₅NHC(O)-^{#}, -(CH₂)₆NHC(O)-^{#}, -(CH₂)₇NHC(O)-^{#}, -(CH₂)₈NHC(O)-^{#}, - (CH₂)₉NHC(O)-^{#}, -(CH₂)₁₀NHC(O)-^{#}, -CH₂S(O)₂NH-^{#}, -(CH₂)₂S(O)₂NH-^{#}, -(CH₂)₃S(O)₂NH-^{#}, - (CH₂)₄S(O)₂NH-^{#}, -(CH₂)₅S(O)₂NH-^{#}, -(CH₂)₆S(O)₂NH-^{#}, -(CH₂)₇S(O)₂NH-^{#}, -(CH₂)₈S(O)₂NH-^{#}, - (CH₂)₉S(O)₂NH-^{#}, -(CH₂)₁₀S(O)₂NH-^{#}, -CH₂NHS(O)₂-^{#}, -(CH₂)₂NHS(O)₂-^{#}, -(CH₂)₃NHS(O)₂-^{#}, - (CH₂)₄NHS(O)₂-^{#}, -(CH₂)₅NHS(O)₂-^{#}, -(CH₂)₆NHS(O)₂-^{#}, -(CH₂)₇NHS(O)₂-^{#}, -(CH₂)₈NHS(O)₂-^{#}, - (CH₂)₉NHS(O)₂-^{#}, -(CH₂)₁₀NHS(O)₂-^{#}, -CH₂S(O)₂O-^{#}, -(CH₂)₂S(O)₂O-^{#}, -(CH₂)₃S(O)₂O-^{#}, - (CH₂)₄S(O)₂O-^{#}, -(CH₂)₅S(O)₂O-^{#}, -(CH₂)₆S(O)₂O-^{#}, -(CH₂)₇S(O)₂O-^{#}, -(CH₂)₈S(O)₂O-^{#}, - (CH₂)₉S(O)₂O-^{#}, -(CH₂)₁₀S(O)₂O-^{#}, -CH₂OS(O)₂-^{#}, -(CH₂)₂OS(O)₂-^{#}, -(CH₂)₃OS(O)₂-^{#}, - (CH₂)₄OS(O)₂-^{#}, -(CH₂)₅OS(O)₂-^{#}, -(CH₂)₆OS(O)₂-^{#}, -(CH₂)₇OS(O)₂-^{#}, -(CH₂)₈OS(O)₂-^{#}, - (CH₂)₉OS(O)₂-^{#}, -(CH₂)₁₀OS(O)₂-^{#}, ethynylene or vinylene; wherein symbol # indicates the point of attachment to L₂, and hydrogen(s) of one or more CH₂ of said group is optionally further replaced by a substituent(s) selected from the group consisting of C₁₋₃ alkyl, C₁₋₃ alkoxy, C₃₋₆ cycloalkyl, halogenated C₁₋₃ alkyl, halogen, hydroxy, cyano, amino, or any combination thereof.

23. The compound of Formula (I) or a salt, an enantiomer, a stereoisomer, a solvate, or a polymorph thereof as claimed in any one of claims 1-22, wherein X_{b} represents:
1-adamantanyl, 2-adamantanyl, 3-adamantanyl, 4-adamantanyl, 5-adamantanyl, 6-adamantanyl, 7-adamantanyl, 8-adamantanyl, 9-adamantanyl, 10-adamantanyl, haloadamantanyl, oxoadamantanyl, hydroxyadamantanyl, methyladamantanyl, dimethyladamantanyl, 1-noradamantanyl, 2-noradamantanyl, 3-noradamantanyl, 4-noradamantanyl, 5-noradamantanyl, 6-noradamantanyl, 7-noradamantanyl, 8-noradamantanyl, 9-noradamantanyl, p-menthanyl, meta-menthanyl, quinuclidinyl, bicyclo[2.2.1]heptan-2-yl, bicyclo[2.2.1]heptan-3-yl, bicyclo[2.2.1]heptan-4-yl, bicyclo[2.2.1]heptan-5-yl, bicyclo[2.2.1]heptan-6-yl, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl, 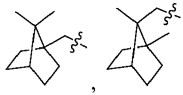 , 7,7-dimethyl-2-oxobicyclo[2.2.1]heptan-1-yl, 7,7-dimethyl-2-oxobicyclo[2.2.1]heptan-3-yl, 7,7-dimethyl-2-oxobicyclo[2.2.1]heptan-4-yl, 7,7-dimethyl-2-oxobicyclo[2.2.1]heptan-5-yl, 7,7-dimethyl-2-oxobicyclo[2.2.1]heptan-6-yl, bicyclo[2.2.1]hept-5-en-2-yl, bicyclo[2.2.1]hept-5-en-3-yl, or bicyclo[2.2.1]hept-5-en-7-yl.

24. The compound of Formula (I) or a salt, an enantiomer, a stereoisomer, a solvate, or a polymorph thereof as claimed in any one of claims 1-22, wherein X_{b} represents:

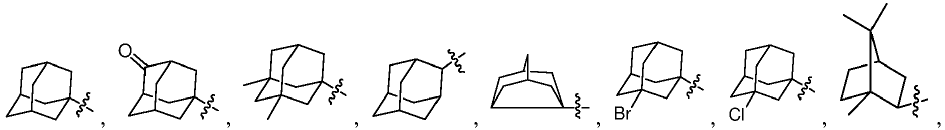

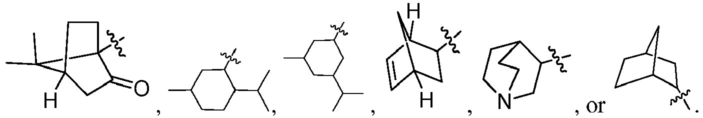

25. The compound of Formula (I) or a salt, an enantiomer, a stereoisomer, a solvate, or a polymorph thereof as claimed in claim 1, which is selected from:
3-(4-(((5-((adamantan-1-ylamino)methyl)furan-2-yl)methyl)thio)-1-oxoisoindolin-2-yl)piperidine-2,6-dione;
3-(1-oxo-4-(((5-(((1,7,7-trimethylbicyclo[2.2.1]heptan-2-yl)amino)methyl)furan-2-yl)methyl)thio)isoindolin-2-yl)piperidine-2,6-dione;
3-(4-(((5-((adamantan-1-yloxy)methyl)furan-2-yl)methyl)thio)-1-oxoisoindolin-2-yl)piperidine-2,6-dione;
3-(4-(((5-((adamantan-2-yloxy)methyl)furan-2-yl)methyl)thio)-1-oxoisoindolin-2-yl)piperidine-2,6-dione;
(5-(((2-(2,6-dioxopiperidin-3-yl)-1-oxoisoindolin-4-yl)thio)methyl)furan-2-yl)methyl adamantane-1-carboxylate;
(5-(((2-(2,6-dioxopiperidin-3-yl)-1-oxoisoindolin-4-yl)thio)methyl)furan-2-yl)methyl 2-(adamantan-1-yl)acetate;
(5-(((2-(2,6-dioxopiperidin-3-yl)-1-oxoisoindolin-4-yl)thio)methyl)furan-2-yl)methyl 4-(adamantan-1-yl)-2-methylbutanoate;
3-(4-(((5-((2-(adamantan-1-yl)ethoxy)methyl)furan-2-yl)methyl)thio)-1-oxoisoindolin-2-yl)piperidine-2,6-dione;
3-(4-(((5-((adamantan-1 -ylmethoxy)methyl)furan-2-yl)methyl)thio)-1 -oxoisoindolin-2-yl)piperidine-2,6-dione;
3-(1-oxo-4-(((5-(((1,7,7-trimethylbicyclo[2.2.1]heptan-2-yl)oxy)methyl)furan-2-yl)methyl)thio)isoindolin-2-yl)piperidine-2,6-dione;
3-(4-(((5-(((2-isopropyl-5-methylcyclohexyl)oxy)methyl)furan-2-yl)methyl)thio)-1-oxoisoindolin-2-yl)piperidine-2,6-dione;
3-(4-(((5-(((hexahydro-2,5-methanopentalen-3a(1H)-yl)amino)methyl)furan-2-yl)methyl)thio)-1-oxoisoindolin-2-yl)piperidine-2,6-dione;
3-(4-(((2-((adamantan-1 -ylamino)methyl)thiazol-4-yl)methyl)thio)-1 -oxoisoindolin-2-yl)piperidine-2,6-dione;
3-(4-(((2-((adamantan-1-yloxy)methyl)thiazol-4-yl)methyl)thio)-1-oxoisoindolin-2-yl)piperidine-2,6-dione;
(4-(((2-(2,6-dioxopiperidin-3-yl)-1-oxoisoindolin-4-yl)thio)methyl)thiazol-2-yl)methyl adamantane-1-carboxylate;
N-((4-(((2-(2,6-dioxopiperidin-3-yl)-1-oxoisoindolin-4-yl)thio)methyl)thiazol-2-yl)methyl)adamantane-1-carboxamide;
4-(adamantan-1-yl)-N-((4-(((2-(2,6-dioxopiperidin-3-yl)-1-oxoisoindolin-4-yl)thio)methyl)thiazol-2-yl)methyl)-2-methylbutanamide;
(4-(((2-(2,6-dioxopiperidin-3-yl)-1-oxoisoindolin-4-yl)thio)methyl)thiazol-2-yl)methyl 2-(adamantan-1-yl)acetate;
3-(4-(((2-((adamantan-1 -ylmethoxy)methyl)thiazol-4-yl)methyl)thio)-1 -oxoisoindolin-2-yl)piperidine-2,6-dione;
3-(1-oxo-4-(((2-(((1,7,7-trimethylbicyclo[2.2.1]heptan-2-yl)oxy)methyl)thiazol-4-yl)methyl)thio)isoindolin-2-yl)piperidine-2,6-dione;
(4-(((2-(2,6-dioxopiperidin-3-yl)-1-oxoisoindolin-4-yl)thio)methyl)thiazol-2-yl)methyl 3-bromoadamantane-1-carboxylate;
adamantan-1-yl 4-(((2-(2,6-dioxopiperidin-3-yl)-1-oxoisoindolin-4-yl)thio)methyl)thiazole-2-carboxylate;
adamantan-1-yl 2-(4-(((2-(2,6-dioxopiperidin-3-yl)-1-oxoisoindolin-4-yl)thio)methyl)thiazol-2-yl)acetate;
N-(adamantan-1-yl)-2-(4-(((2-(2,6-dioxopiperidin-3-yl)-1-oxoisoindolin-4-yl)thio)methyl)thiazol-2-yl)acetamide;
4-(((2-(2,6-dioxopiperidin-3-yl)-1-oxoisoindolin-4-yl)thio)methyl)-N-(octahydro-2,5-methanopentalen-6a-yl)thiazole-2-carboxamide;
(4-(((2-(2,6-dioxopiperidin-3-yl)-1-oxoisoindolin-4-yl)thio)methyl)thiazol-2-yl)methyl 3-chloroadamantane-1-carboxylate;
1-(7,7-dimethyl-2-oxobicyclo[2.2.1]heptan-1-yl)-N-((2-(((2-(2,6-dioxopiperidin-3-yl)-1-oxoisoindolin-4-yl)thio)methyl)thiazol-4-yl)methyl)methanesulfonamide;
(2-(((2-(2,6-dioxopiperidin-3-yl)-1-oxoisoindolin-4-yl)thio)methyl)thiazol-4-yl)methyl 4-oxoadamantane-1-carboxylate;
3-(4-(((4-((adamantan-2-ylamino)methyl)thiazol-2-yl)methyl)thio)-1-oxoisoindolin-2-yl)piperidine-2,6-dione;
3-(4-(((4-(((2-isopropyl-5-methylcyclohexyl)oxy)methyl)thiazol-2-yl)methyl)thio)-1-oxoisoindolin-2-yl)piperidine-2,6-dione;
3-(4-(((4-((adamantan-2-yloxy)methyl)thiazol-2-yl)methyl)thio)-1-oxoisoindolin-2-yl)piperidine-2,6-dione;
(2-(((2-(2,6-dioxopiperidin-3-yl)-1-oxoisoindolin-4-yl)thio)methyl)thiazol-4-yl)methyl bicyclo[2.2.1]hept-5-ene-2-carboxylate;
3-(4-(((4-(((3,5-dimethyladamantan-1-yl)amino)methyl)thiazol-2-yl)methyl)thio)-1-oxoisoindolin-2-yl)piperidine-2,6-dione;
3-(4-(((4-((adamantan-1 -ylmethoxy)methyl)thiazol-2-yl)methyl)thio)-1 -oxoisoindolin-2-yl)piperidine-2,6-dione;
3-(4-(((4-(((adamantan-1 -ylmethyl)amino)methyl)thiazol-2-yl)methyl)thio)-1 -oxoisoindolin-2-yl)piperidine-2,6-dione;
3-(4-(((4-((adamantan-1 -ylamino)methyl)thiazol-2-yl)methyl)thio)-1 -oxoisoindolin-2-yl)piperidine-2,6-dione;
N-(adamantan-1-yl)-2-(((2-(2,6-dioxopiperidin-3-yl)-1-oxoisoindolin-4-yl)thio)methyl)thiazole-4-carboxamide;
N-(adamantan-1-yl)-2-(2-(((2-(2,6-dioxopiperidin-3-yl)-1-oxoisoindolin-4-yl)thio)methyl)thiazol-4-yl)acetamide;
adamantan-1-yl 2-(((2-(2,6-dioxopiperidin-3-yl)-1-oxoisoindolin-4-yl)thio)methyl)thiazole-4-carboxylate;
3-(4-((2-(4-((adamantan-1-ylamino)methyl)-1H-1,2,3-triazol-1-yl)ethyl)thio)-1-oxoisoindolin-2-yl)piperidine-2,6-dione;
3-(4-(((5-(((adamantan-1-yl)amino)methyl)pyridin-2-yl)methyl)thio)-1-oxoisoindolin-2-yl)piperidine-2,6-dione;
N-(adamantan-1-yl)-6-(((2-(2,6-dioxopiperidin-3-yl)-1-oxoisoindolin-4-yl)thio)methyl)nicotinamide;
2-isopropyl-5-methylcyclohexyl 6-(((2-(2,6-dioxopiperidin-3-yl)-1-oxoisoindolin-4-yl)thio)methyl)nicotinate;
6-(((2-(2,6-dioxopiperidin-3-yl)-1-oxoisoindolin-4-yl)thio)methyl)-N-(quinuclidin-3-yl)nicotinamide;
N-(bicyclo[2.2.1]heptan-2-yl)-6-(((2-(2,6-dioxopiperidin-3-yl)-1-oxoisoindolin-4-yl)thio)methyl)nicotinamide;
3-(4-(((5-(((2-isopropyl-5-methylcyclohexyl)oxy)methyl)pyridin-2-yl)methyl)thio)-1-oxoisoindolin-2-yl)piperidine-2,6-dione;
3-(4-(((5-(((adamantan-1 -yl)amino)methyl)pyrimidin-2-yl)methyl)thio)-1 -oxoisoindolin-2-yl)piperidine-2,6-dione;
3-(4-(((5-((adamantan-1-ylamino)methyl)benzofuran-2-yl)methyl)thio)-1-oxoisoindolin-2-yl)piperidine-2,6-dione;
3-(4-(((6-((adamantan-1 -ylamino)methyl)quinolin-2-yl)methyl)thio)-1 -oxoisoindolin-2-yl)piperidine-2,6-dione;
N-(adamantan-1-yl)-2-(((2-(2,6-dioxopiperidin-3-yl)-1-oxoisoindolin-4-yl)thio)methyl)benzo[b]thiophene-5-carboxamide;
N-((adamantan-1-yl)methyl)-2-(((2-(2,6-dioxopiperidin-3-yl)-1-oxoisoindolin-4-yl)thio)methyl)benzo[b]thiophene-5-carboxamide;
(adamantan-1-yl)methyl 2-(((2-(2,6-dioxopiperidin-3-yl)-1-oxoisoindolin-4-yl)thio)methyl)benzo[b]thiophene-5-carboxylate;
2-(adamantan-1-yl)ethyl 2-(((2-(2,6-dioxopiperidin-3-yl)-1-oxoisoindolin-4-yl)thio)methyl)benzo[b]thiophene-5-carboxylate;
(2-(((2-(2,6-dioxopiperidin-3-yl)-1-oxoisoindolin-4-yl)thio)methyl)benzo[b]thiophen-5-yl)methyl 2-(adamantan-1-yl)acetate;
3-(5-(((4-(((adamantan-2-yl)amino)methyl)thiazol-2-yl)methyl)thio)-1-oxoisoindolin-2-yl)piperidine-2,6-dione;
3-(6-(((4-(((adamantan-1 -yl)amino)methyl)thiazol-2-yl)methyl)thio)-1 -oxoisoindolin-2-yl)piperidine-2,6-dione;
3-(4-((5-((adamantan-1 -ylamino)methyl)furan-2-yl)methoxy)-1 -oxoisoindolin-2-yl)piperidine-2,6-dione;
3-(4-((5-((2-(adamantan-1-yl)ethoxy)methyl)furan-2-yl)methoxy)-1-oxoisoindolin-2-yl)piperidine-2,6-dione;
1-(7,7-dimethyl-2-oxobicyclo[2.2.1]heptan-1-yl)-N-((2-(((2-(2,6-dioxopiperidin-3-yl)-1-oxoisoindolin-4-yl)oxy)methyl)thiazol-4-yl)methyl)methanesulfonamide;
(2-(((2-(2,6-dioxopiperidin-3-yl)-1-oxoisoindolin-4-yl)oxy)methyl)thiazol-4-yl)methyl 4-oxoadamantane-1-carboxylate;
3-(4-((4-((adamantan-2-ylamino)methyl)thiazol-2-yl)methoxy)-1-oxoisoindolin-2-yl)piperidine-2,6-dione;
3-(4-((5-((adamantan-1-ylamino)methyl)benzofuran-2-yl)methoxy)-1-oxoisoindolin-2-yl)piperidine-2,6-dione;
3-(4-((2-((adamantan-1-ylamino)methyl)thiazol-4-yl)methoxy)-1-oxoisoindolin-2-yl)piperidine-2,6-dione;
(4-(((2-(2,6-dioxopiperidin-3-yl)-1-oxoisoindolin-4-yl)oxy)methyl)thiazol-2-yl)methyl adamantane-1-carboxylate;
adamantan-1-yl 4-(((2-(2,6-dioxopiperidin-3-yl)-1-oxoisoindolin-4-yl)oxy)methyl)thiazole-2-carboxylate;
4-(((2-(2,6-dioxopiperidin-3-yl)-1-oxoisoindolin-4-yl)oxy)methyl)-N-(octahydro-2,5-methanopentalen-6a-yl)thiazole-2-carboxamide;
4-(adamantan-1-yl)-N-((4-(((2-(2,6-dioxopiperidin-3-yl)-1-oxoisoindolin-4-yl)oxy)methyl)thiazol-2-yl)methyl)-2-methylbutanamide;
(4-(((2-(2,6-dioxopiperidin-3-yl)-1-oxoisoindolin-4-yl)oxy)methyl)thiazol-2-yl)methyl 2-(adamantan-1-yl)acetate;
3-(4-(((5-((2-(adamantan-1-yl)ethoxy)methyl)furan-2-yl)methyl)amino)-1-oxoisoindolin-2-yl)piperidine-2,6-dione;
(2-(((2-(2,6-dioxopiperidin-3-yl)-1-oxoisoindolin-4-yl)amino)methyl)thiazol-4-yl)methyl 7,7-dimethyl-2-oxobicyclo[2.2.1]heptan-1-yl)methanesulfonate;
(2-(((2-(2,6-dioxopiperidin-3-yl)-1-oxoisoindolin-4-yl)amino)methyl)thiazol-4-yl)methyl 4-oxoadamantane-1-carboxylate;
3-(4-(((4-((adamantan-2-yloxy)methyl)thiazol-2-yl)methyl)amino)-1-oxoisoindolin-2-yl)piperidine-2,6-dione;
3-(4-(((5-((adamantan-1 -yloxy)methyl)benzofuran-2-yl)methyl)amino)-1 -oxoisoindolin-2-yl)piperidine-2,6-dione;
3-(4-(((2-((adamantan-1-yloxy)methyl)thiazol-4-yl)methyl)amino)-1-oxoisoindolin-2-yl)piperidine-2,6-dione;
(4-(((2-(2,6-dioxopiperidin-3-yl)-1-oxoisoindolin-4-yl)amino)methyl)thiazol-2-yl)methyl adamantane-1-carboxylate;
adamantan-1-yl 4-(((2-(2,6-dioxopiperidin-3-yl)-1-oxoisoindolin-4-yl)amino)methyl)thiazole-2-carboxylate;
4-(((2-(2,6-dioxopiperidin-3-yl)-1-oxoisoindolin-4-yl)amino)methyl)-N-(octahydro-2,5-methanopentalen-6a-yl)thiazole-2-carboxamide;
4-(((5-((adamantan-1-ylamino)methyl)pyridin-2-yl)methyl)amino)-2-(2,6-dioxopiperidin-3-yl)isoindoline-1,3-dione;
4-(((5-((2-(adamantan-1-yl)ethoxy)methyl)furan-2-yl)methyl)amino)-2-(2,6-dioxopiperidin-3-yl)isoindoline-1,3-dione;
(2-(((2-(2,6-dioxopiperidin-3-yl)-1,3-dioxoisoindolin-4-yl)amino)methyl)thiazol-4-yl)methyl (7,7-dimethyl-2-oxobicyclo[2.2.1]heptan-1-yl)methanesulfonate;
(2-(((2-(2,6-dioxopiperidin-3-yl)-1,3-dioxoisoindolin-4-yl)amino)methyl)thiazol-4-yl)methyl 4-oxoadamantane-1-carboxylate;
4-(((4-((adamantan-2-yloxy)methyl)thiazol-2-yl)methyl)amino)-2-(2,6-dioxopiperidin-3-yl)isoindoline-1,3-dione;
4-(((5-((adamantan-1-yloxy)methyl)benzofuran-2-yl)methyl)amino)-2-(2,6-dioxopiperidin-3-yl)isoindoline-1,3-dione;
4-(((2-((adamantan-1-yloxy)methyl)thiazol-4-yl)methyl)amino)-2-(2,6-dioxopiperidin-3-yl)isoindoline-1,3-dione;
(4-(((2-(2,6-dioxopiperidin-3-yl)-1,3-dioxoisoindolin-4-yl)amino)methyl)thiazol-2-yl)methyl adamantane-1-carboxylate;
adamantan-1-yl 4-(((2-(2,6-dioxopiperidin-3-yl)-1,3-dioxoisoindolin-4-yl)amino)methyl)thiazole-2-carboxylate;
4-(((2-(((adamantan-1-yl)amino)methyl)thiazol-4-yl)methyl)amino)-2-(2,6-dioxopiperidin-3-yl)isoindoline-1,3-dione;
4-(((2-(2,6-dioxopiperidin-3-yl)-1,3-dioxoisoindolin-4-yl)amino)methyl)-N-(octahydro-2,5-methanopentalen-6a-yl)thiazole-2-carboxamide;
4-(((5-((adamantan-1-ylamino)methyl)furan-2-yl)methyl)thio)-2-(2,6-dioxopiperidin-3-yl)isoindoline-1,3-dione;
2-(2,6-dioxopiperidin-3-yl)-4-(((5-(((1,7,7-trimethylbicyclo[2.2.1]heptan-2-yl)oxy)methyl)furan-2-yl)methyl)thio)isoindoline-1,3-dione;
2-(2,6-dioxopiperidin-3-yl)-4-(((5-(((hexahydro-2,5-methanopentalen-3a(1H)-yl)amino)methyl)furan-2-yl)methyl)thio)isoindoline-1,3-dione;
4-(((5-((adamantan-1-ylamino)methyl)pyridin-2-yl)methyl)thio)-2-(2,6-dioxopiperidin-3-yl)isoindoline-1,3-dione;
adamantan-1-yl 4-(((2-(2,6-dioxopiperidin-3-yl)-1,3-dioxoisoindolin-4-yl)thio)methyl)thiazole-2-carboxylate;
4-(((2-((adamantan-1-ylamino)methyl)thiazol-4-yl)methyl)thio)-2-(2,6-dioxopiperidin-3-yl)isoindoline-1,3-dione;
4-(((2-(((adamantan-1-yl)oxy)methyl)thiazol-4-yl)methyl)thio)-2-(2,6-dioxopiperidin-3-yl)isoindoline-1,3-dione;
(4-(((2-(2,6-dioxopiperidin-3-yl)-1,3-dioxoisoindolin-4-yl)thio)methyl)thiazol-2-yl)methyl adamantane-1-carboxylate;
(4-(((2-(2,6-dioxopiperidin-3-yl)-1,3-dioxoisoindolin-4-yl)thio)methyl)thiazol-2-yl)methyl 2-(adamantan-1-yl)acetate;
(4-(((2-(2,6-dioxopiperidin-3-yl)-1,3-dioxoisoindolin-4-yl)thio)methyl)thiazol-2-yl)methyl 4-(adamantan-1-yl)-2-methylbutanoate;
4-(adamantan-1-yl)-N-((4-(((2-(2,6-dioxopiperidin-3-yl)-1,3-dioxoisoindolin-4-yl)thio)methyl)thiazol-2-yl)methyl)-2-methylbutanamide;
4-(((2-(2,6-dioxopiperidin-3-yl)-1,3-dioxoisoindolin-4-yl)thio)methyl)-N-(-octahydro-2,5-methanopentalen-6a-yl)thiazole-2-carboxamide;
4-(((4-((adamantan-1-ylamino)methyl)thiazol-2-yl)methyl)thio)-2-(2,6-dioxopiperidin-3-yl)isoindoline-1,3-dione;
4-(((4-((adamantan-2-ylamino)methyl)thiazol-2-yl)methyl)thio)-2-(2,6-dioxopiperidin-3-yl)isoindoline-1,3-dione;
4-(((4-(((3,5-dimethyladamantan-1-yl)amino)methyl)thiazol-2-yl)methyl)thio)-2-(2,6-dioxopiperidin-3-yl)isoindoline-1,3-dione;
4-(((4-(((adamantan-1-yl)methoxy)methyl)thiazol-2-yl)methyl)thio)-2-(2,6-dioxopiperidin-3-yl)isoindoline-1,3-dione;
4-(((4-((((adamantan-1-yl)methyl)amino)methyl)thiazol-2-yl)methyl)thio)-2-(2,6-dioxopiperidin-3-yl)isoindoline-1,3-dione;
N-(adamantan-1-yl)-2-(((2-(2,6-dioxopiperidin-3-yl)-1,3-dioxoisoindolin-4-yl)thio)methyl)benzo[b]thiophene-5-carboxamide;
N-((adamantan-1-yl)methyl)-2-(((2-(2,6-dioxopiperidin-3-yl)-1,3-dioxoisoindolin-4-yl)thio)methyl)benzo[b]thiophene-5-carboxamide;
(adamantan-1-yl)methyl 2-(((2-(2,6-dioxopiperidin-3-yl)-1,3-dioxoisoindolin-4-yl)thio)methyl)benzo[b]thiophene-5-carboxylate;
2-(adamantan-1-yl)ethyl 2-(((2-(2,6-dioxopiperidin-3-yl)-1,3-dioxoisoindolin-4-yl)thio)methyl)benzo[b]thiophene-5-carboxylate;
4-((5-((adamantan-1-ylamino)methyl)furan-2-yl)methoxy)-2-(2,6-dioxopiperidin-3-yl)isoindoline-1,3-dione;
4-((5-((adamantan-1-ylamino)methyl)pyridin-2-yl)methoxy)-2-(2,6-dioxopiperidin-3-yl)isoindoline-1,3-dione;
adamantan-1-yl 4-(((2-(2,6-dioxopiperidin-3-yl)-1,3-dioxoisoindolin-4-yl)oxy)methyl)thiazole-2-carboxylate;
4-(((2-(2,6-dioxopiperidin-3-yl)-1,3-dioxoisoindolin-4-yl)oxy)methyl)-N-(octahydro-2,5-methanopentalen-6a-yl)thiazole-2-carboxamide;
4-((4-((adamantan-1-ylamino)methyl)thiazol-2-yl)methoxy)-2-(2,6-dioxopiperidin-3-yl)isoindoline-1,3-dione;
4-((4-((adamantan-2-ylamino)methyl)thiazol-2-yl)methoxy)-2-(2,6-dioxopiperidin-3-yl)isoindoline-1,3-dione;
4-((2-((adamantan-1-ylamino)methyl)thiazol-4-yl)methoxy)-2-(2,6-dioxopiperidin-3-yl)isoindoline-1,3-dione;
N-(adamantan-1-yl)-6-(2-(2-(2,6-dioxopiperidin-3-yl)-1,3-dioxoisoindolin-4-yl)ethyl)nicotinamide;
4-(2-(2-((adamantan-1-ylamino)methyl)thiazol-4-yl)ethyl)-2-(2,6-dioxopiperidin-3-yl)isoindoline-1,3-dione;
3-(4-(2-(2-((adamantan-1-ylamino)methyl)thiazol-4-yl)ethyl)-1-oxoisoindolin-2-yl)piperidine-2,6-dione;
4-(4-(2-((adamantan-1-ylamino)methyl)thiazol-4-yl)but-1-yn-1-yl)-2-(2,6-dioxopiperidin-3-yl)isoindoline-1,3-dione;
3-(4-(3-(4-(2-(adamantan-1-ylamino)ethyl)piperazin-1-yl)prop-1-yn-1-yl)-1-oxoisoindolin-2-yl)piperidine-2,6-dione;
3-(4-(3-(4-(2-(adamantan-1 -ylamino)ethyl)piperazin-1 -yl)propyl)-1 -oxoisoindolin-2-yl)piperidine-2,6-dione;
3-(4-(2-(4-(2-(adamantan-1 -ylamino)ethyl)piperazin-1 -yl)ethyl)-1 -oxoisoindolin-2-yl)piperidine-2,6-dione;
3-(4-((2-(4-(2-(adamantan-1 -ylamino)ethyl)piperazin-1 -yl)ethyl)thio)-1 -oxoisoindolin-2-yl)piperidine-2,6-dione;
3-(4-(2-(4-(2-(adamantan-1-ylamino)ethyl)piperazin-1-yl)ethoxy)-1-oxoisoindolin-2-yl)piperidine-2,6-dione;
3-(4-((2-(4-(2-(adamantan-1-ylamino)ethyl)piperazin-1-yl)ethyl)amino)-1-oxoisoindolin-2-yl)piperidine-2,6-dione;
4-(2-(4-(2-(adamantan-1-ylamino)ethyl)piperazin-1-yl)ethyl)-2-(2,6-dioxopiperidin-3-yl)isoindoline-1,3-dione;
4-((2-(4-(2-(adamantan-1-ylamino)ethyl)piperazin-1-yl)ethyl)thio)-2-(2,6-dioxopiperidin-3-yl)isoindoline-1,3-dione;
4-(2-(4-(2-(adamantan-1-ylamino)ethyl)piperazin-1-yl)ethoxy)-2-(2,6-dioxopiperidin-3-yl)isoindoline-1,3-dione;
4-((2-(4-(2-(adamantan-1-ylamino)ethyl)piperazin-1-yl)ethyl)amino)-2-(2,6-dioxopiperidin-3-yl)isoindoline-1,3-dione;
3-(4-(3-(4-(2-(adamantan-1-ylamino)ethyl)-1,4-diazepan-1-yl)prop-1-yn-1-yl)-1-oxoisoindolin-2-yl)piperidine-2,6-dione;
3-(4-(3-(4-(2-(adamantan-1 -ylamino)ethyl)-1,4-diazepan-1 -yl)propyl)-1 -oxoisoindolin-2-yl)piperidine-2,6-dione;
3-(4-(2-(4-(2-(adamantan-1-ylamino)ethyl)-1,4-diazepan-1-yl)ethyl)-1-oxoisoindolin-2-yl)piperidine-2,6-dione;
3-(4-((2-(4-(2-(adamantan-1 -ylamino)ethyl)-1,4-diazepan-1 -yl)ethyl)thio)-1 -oxoisoindolin-2-yl)piperidine-2,6-dione; or
3-(4-(2-(4-(2-(adamantan-1-ylamino)ethyl)-1,4-diazepan-1-yl)ethoxy)-1-oxoisoindolin-2-yl)piperidine-2,6-dione.

26. The compound of Formula (I) or a salt, an enantiomer, a stereoisomer, a solvate, or a polymorph thereof as claimed in any one of claims 1-25, which is hydrochloride of the compound of Formula (I).

27. A pharmaceutical composition comprising the compound of Formula (I) as claimed in any one of claims 1-26 or a pharmaceutically acceptable salt thereof, and at least one pharmaceutically acceptable carrier.

28. The pharmaceutical composition as claimed in claim 27, further comprising at least one therapeutic agent.

29. Use of the compound of Formula (I) or a pharmaceutically acceptable salt thereof as claimed in any one of claims 1 to 26 or the pharmaceutical composition as claimed in claim 27 or 28 for the manufacture of a medicament for the prevention and/or treatment of a disease or disorder associated with TNF-α.

30. The use as claimed in claim 29, wherein the disease or disorder associated with TNF-α is selected from the group consisting of: tumors, infectious diseases, inflammatory diseases, autoimmune diseases, anemia, hemorrhagic shock, transplant rejection, multiple organ dysfunction syndrome (MODS), sarcoidosis, adult respiratory distress syndrome, congestive heart failure, myocardial infarction, acute liver failure, or diabetes.

31. The use as claimed in claim 29, wherein the disease or disorder associated with TNF-α is selected from the group consisting of: tumors, including multiple myeloma, myelodysplastic syndrome (MDS), previously treated myelodysplastic syndrome, plasma cell myeloma, transplantation-related cancer, myelofibrosis, plasma cell myeloma, smoldering myeloma, smoldering multiple myeloma; neutropenia; leukemia, including acute myeloid leukemia, chronic myelogenous leukemia, B-cell chronic lymphocytic leukemia, acute myeloid leukemia (AML); lymphoma, including lymphoma CD20 positive, mantle cell lymphoma, primary lymphoma, B-cell lymphoma, recurrent B-cell non-Hodgkin's lymphoma, recurrent diffuse large B-cell lymphoma, recurrent primary mediastinal (thymus) large B-cell, relapsed transformed non-Hodgkin's lymphoma, refractory B-cell non-Hodgkin's lymphoma, refractory diffuse large B-cell lymphoma, refractory primary mediastinal (thymus) large B-cell, refractory transformed non-Hodgkin's lymphoma; thyroid cancer; melanoma; lung cancer; Unverricht Syndrome; sepsis syndrome; Rheumatoid arthritis; autoimmune encephalomyelitis; ankylosing spondylitis; psoriasis; systemic lupus erythematosus; recurrent oral ulcer; multiple sclerosis; inflammatory bowel disease, including Crohn's disease and ulcerative colitis; Kawasaki disease; bacterial meningitis; cerebral malaria; AIDS; COVID-19 novel coronavirus infection; septic shock; tuberculosis; pneumonia, osteoarthritis, synovitis, systemic inflammatory response syndrome, airway inflammation, bronchitis, chronic obstructive pulmonary disease, asthma; anemia; hemorrhagic shock; organ (including kidney, heart, lung) or tissue transplant rejection; diabetes; sarcoidosis; adult respiratory distress syndrome; congestive heart failure; myocardial infarction; multiple organ failure due to cachexia and septic shock; and acute liver failure.

32. The compound of Formula (I) or a pharmaceutically acceptable salt thereof as claimed in any one of claims 1 to 26, for use in the prevention and/or treatment of a disease or disorder associated with TNF-α.

33. The compound of Formula (I) or a pharmaceutically acceptable salt thereof as claimed in claim 32, wherein the disease or disorder associated with TNF-α is selected from the group consisting of: tumors, infectious diseases, inflammatory diseases, autoimmune diseases, anemia, hemorrhagic shock, transplant rejection, multiple organ dysfunction syndrome (MODS), sarcoidosis, adult respiratory distress syndrome, congestive heart failure, myocardial infarction, acute liver failure, or diabetes.

34. The compound of Formula (I) or a pharmaceutically acceptable salt thereof as claimed in claim 32, wherein the disease or disorder associated with TNF-α is selected from the group consisting of: tumors, including multiple myeloma, myelodysplastic syndrome (MDS), previously treated myelodysplastic syndrome, plasma cell myeloma, transplantation-related cancer, myelofibrosis, plasma cell myeloma, smoldering myeloma, smoldering multiple myeloma; neutropenia; leukemia, including acute myeloid leukemia, chronic myelogenous leukemia, B-cell chronic lymphocytic leukemia, acute myeloid leukemia (AML); lymphoma, including lymphoma CD20 positive, mantle cell lymphoma, primary lymphoma, B-cell lymphoma, recurrent B-cell non-Hodgkin's lymphoma, recurrent diffuse large B-cell lymphoma, recurrent primary mediastinal (thymus) large B-cell, relapsed transformed non-Hodgkin's lymphoma, refractory B-cell non-Hodgkin's lymphoma, refractory diffuse large B-cell lymphoma, refractory primary mediastinal (thymus) large B-cell, refractory transformed non-Hodgkin's lymphoma; thyroid cancer; melanoma; lung cancer; Unverricht Syndrome; sepsis syndrome; Rheumatoid arthritis; autoimmune encephalomyelitis; ankylosing spondylitis; psoriasis; systemic lupus erythematosus; recurrent oral ulcer; multiple sclerosis; inflammatory bowel disease, including Crohn's disease and ulcerative colitis; Kawasaki disease; bacterial meningitis; cerebral malaria; AIDS; COVID-19 novel coronavirus infection; septic shock; tuberculosis; pneumonia, osteoarthritis, synovitis, systemic inflammatory response syndrome, airway inflammation, bronchitis, chronic obstructive pulmonary disease, asthma; anemia; hemorrhagic shock; organ (including kidney, heart, lung) or tissue transplant rejection; diabetes; sarcoidosis; adult respiratory distress syndrome; congestive heart failure; myocardial infarction; multiple organ failure due to cachexia and septic shock; and acute liver failure.

35. A method for treating or preventing a disease or disorder associated with TNF-α, comprising administering to a subject a therapeutically effective amount of the compound of Formula (I) or a pharmaceutically acceptable salt thereof as claimed in any one of claims 1-26, or the pharmaceutical composition as claimed in claim 27 or 28.

36. The method as claimed in claim 35, wherein the disease or disorder associated with TNF-α is selected from the group consisting of: tumors, infectious diseases, inflammatory diseases, autoimmune diseases, anemia, hemorrhagic shock, transplant rejection, multiple organ dysfunction syndrome (MODS), sarcoidosis, adult respiratory distress syndrome, congestive heart failure, myocardial infarction, acute liver failure, or diabetes.

37. The method as claimed in claim 35, wherein the disease or disorder associated with TNF-α is selected from the group consisting of: tumors, including multiple myeloma, myelodysplastic syndrome (MDS), previously treated myelodysplastic syndrome, plasma cell myeloma, transplantation-related cancer, myelofibrosis, plasma cell myeloma, smoldering myeloma, smoldering multiple myeloma; neutropenia; leukemia, including acute myeloid leukemia, chronic myelogenous leukemia, B-cell chronic lymphocytic leukemia, acute myeloid leukemia (AML); lymphoma, including lymphoma CD20 positive, mantle cell lymphoma, primary lymphoma, B-cell lymphoma, recurrent B-cell non-Hodgkin's lymphoma, recurrent diffuse large B-cell lymphoma, recurrent primary mediastinal (thymus) large B-cell, relapsed transformed non-Hodgkin's lymphoma, refractory B-cell non-Hodgkin's lymphoma, refractory diffuse large B-cell lymphoma, refractory primary mediastinal (thymus) large B-cell, refractory transformed non-Hodgkin's lymphoma; thyroid cancer; melanoma; lung cancer; Unverricht Syndrome; sepsis syndrome; Rheumatoid arthritis; autoimmune encephalomyelitis; ankylosing spondylitis; psoriasis; systemic lupus erythematosus; recurrent oral ulcer; multiple sclerosis; inflammatory bowel disease, including Crohn's disease and ulcerative colitis; Kawasaki disease; bacterial meningitis; cerebral malaria; AIDS; COVID-19 novel coronavirus infection; septic shock; tuberculosis; pneumonia, osteoarthritis, synovitis, systemic inflammatory response syndrome, airway inflammation, bronchitis, chronic obstructive pulmonary disease, asthma; anemia; hemorrhagic shock; organ (including kidney, heart, lung) or tissue transplant rejection; diabetes; sarcoidosis; adult respiratory distress syndrome; congestive heart failure; myocardial infarction; multiple organ failure due to cachexia and septic shock; and acute liver failure.
